# EUROPEAN PATENT SPECIFICATION

(11) **EP 2 155 231 B1**
(45) Date of publication and mention of the grant of the patent: **08.08.2012**
(21) Application number: 08742868.6
(22) Date of filing: 10.04.2008
(51) Int. Cl.: A61K 38/16, A61K 31/00

(54) **IMAGING AND THERAPY OF VIRUS-ASSOCIATED TUMORS**
BILDDARSTELLUNG UND BEHANDLUNG VON VIRUS-ASSOZIIERTEN TUMOREN
IMAGERIE ET THÉRAPIE DES TUMEURS ASSOCIÉES À UN VIRUS

(30) Priority: 10.04.2007 US 922755 P; 25.05.2007 US 931921 P
(43) Date of publication of application: 24.02.2010
(73) Proprietor: The Johns Hopkins University, Baltimore, MD 21218 (US)
(72) Inventor: POMPER, Martin G., Baltimore, MD 21212 (US); AMBINDER, Richard F., Lutherville, MD 21093 (US); LIU, Jun Kyu, Clarksville, MD 21029 (US); CHONG, Curtis, Honolulu, HI 96815 (US)
(74) Representative: Hiebl, Inge Elisabeth
(86) International application number: PCT/US2008/004811
(87) International publication number: WO 2008/124197

(56) References cited:
- WO-A2-2006/002142
- WO-A2-2006/039644
- US-A- 5 837 854
- US-A1- 2004 156 854
- US-A1- 2005 123 904
- US-A1- 2007 065 360
- US-B2- 6 677 302
- US-B2- 6 682 715
- KALUZA VESNA ET AL: "Bortezomib is a novel inducer of latent Epstein Barr Virus (EBV) in EBV plus lymphoma cell lines" BLOOD, AMERICAN SOCIETY OF HEMATOLOGY, US, vol. 108, no. 11, Part 1, 1 November 2006 (2006-11-01), page 711A, XP009131313 ISSN: 0006-4971

## Description

### CROSS-REFERENCE TO RELATED APPLICATIONS

This application claims the benefit of U.S. Provisional Application Serial No.: 60/922,755, filed April 10, 2007 and , U.S. Provisional Application Serial No.: 60/931,921, filed May 25, 2007.

### STATEMENT OF RIGHTS TO INVENTIONS MADE UNDER FEDERALLY SPONSORED RESEARCH

The work was supported by in part, by NIH grants US24 CA92871 and P50 CA96888. The Government has some rights to the invention.

### BACKGROUND OF THE INVENTION

Although many people associate Epstein-Barr virus (EBV) with infectious mononucleosis, EBV is a tumor virus that has been implicated in a variety of human cancer, including Burkitt's lymphoma, lymphomas in AIDS patients and one half of all Hodgkin's disease cases. Tumor viruses, including human papilloma virus, hepatitis B virus and EBV, are responsible for approximately 15 percent of all cancers in humans. Existing methods for diagnosing, monitoring, and treating virus associated neoplasias are inadequate, and improved methods are urgently required.

### Summary of the Invention

As described below, the present invention features compositions and methods for diagnosing, monitoring, and treating a neoplasia associated with a naturally occurring infection such as a viral infection or a bacterial infection.

In an aspect, the invention features a pharmaceutical composition labeled for the treatment of a neoplasia naturally infected with a virus. The composition includes an effective amount of a viral lytic induction agent, a proteasome inhibitor, preferably at a sufficient amount to induce viral gene expression or viral replication in a subject to which the composition is administered. Moreover, it is preferred that the amount required to induce viral gene expression or viral replication is not cytotoxic. Lytic induction agents include, but are not limited to, Bortezomib, the pharmaceutical composition also further includes a radiolabeled analog of 2'-fluoro-2'-deoxy-β-D-5-iodouracil-arabinofuranoside (FIAU). The composition can be used for the treatment of neoplasia associated with infection by, for example, Epstein Bar Virus (EBV) or Kaposi's sarcoma herpes virus, such as lymphoma, gastric carcinoma, Kaposi's sarcoma, or nasopharyngeal carcinoma.

In another aspect, the invention features a pharmaceutical composition for the diagnosis of a neoplasia associated with a naturally occurring infection, such as an infection with a virus or a bacteria. The composition includes an effective amount of a radiolabeled analog of 2'-fluoro-2'-deoxy-β-D-5-iodouracil-arabinofuranoside (FIAU). The radiolabeled composition may be labeled for visualization by SPECT or PET, for example using iodine-¹²³I, ¹²⁴I or ¹²⁵I. The radiolabeled composition can include direction for using the composition with a viral lytic induction agent.

In an aspect, the invention features a pharmaceutical composition for the treatment of a neoplasia associated with an infection, such as a infection with a virus or a bacteria. The composition includes an effective amount of a radiolabeled analog of 2'-deoxy-5-iodo-I-beta-D-arabinofuranosyluracil (FIAU). The label can be an alpha, beta, or gamma particle emitter, for example ⁹⁰Y, ¹⁸⁶Re, ¹⁸⁸Re, ⁶⁴Cu, ⁶⁷Cu, ²¹²Pb, ²¹²Bi, ¹²³I, ²¹¹At, ²¹³Bi or ¹³¹I.The radionuclide prefeably emits at least about 0.5 to 2 Gy and is administered to the subject at a concentration of about about 0.001 to about 0.1 mg/kg, or about 0.01 to about 0.1 mg/kg. The composition can be used for the treatment of neoplasia associated with infection by, for example, Epstein Bar Virus (EBV) or Kaposi's sarcoma herpes virus, such as lymphoma, gastric carcinoma, Kaposi's sarcoma, or nasopharyngeal carcinoma. The radiolabeled composition can include direction for using the composition with a viral lytic induction agent.

In various embodiments of the invention, radionuclide labels for therapeutic compositions of the invention can be an alpha, beta, or gamma particle emitter, for example ⁹⁰Y, ¹⁸⁶Re, ¹⁸⁸Re, ⁶⁴Cu, ⁶⁷Cu, ²¹²Pb, ²¹²Bi, ¹²³I, ²¹¹At, ²¹³Bi or ¹³¹I.

In various embodiments of the invention, radionuclide labels for imaging uses in SPECT or PET are, for example iodine-¹²³I, ¹²⁴I or ¹²⁵I.

In various embodiments of the invention, neoplasia associated with infection by, for example, Epstein Bar Virus (EBV) or Kaposi's sarcoma herpes virus, such as lymphoma, gastric carcinoma, Kaposi's sarcoma, or nasopharyngeal carcinoma

In an aspect, the invention features kits for the diagnosis or monitoring of an infection associated neoplasia in a subject. The kit includes an effective amount of a viral lytic induction agent and a radiolabeled analog of 2'-deoxy-5-iodo-I-beta-D-arabinofuranosyluracil (FIAU), and directions for using the kit for diagnosis of the neoplasia. The kit can be used for diagnosis or monitoring of viral or bacterial infection such as neoplasia associated with infection by, for example, Epstein Bar Virus (EBV) or Kaposi's sarcoma herpes virus, such as lymphoma, gastric carcinoma, Kaposi's sarcoma, or nasopharyngeal carcinoma. The infection associated neoplasia can be imaged using SPECT or PET with iodine-¹²³I, ¹²⁴I or ¹²⁵I as the radionuclide. The lytic induction agent can be a proteasome inhibitor, a microtubule disrupting agent, a glucocorticoid or steroid hormone, a nucleoside analog, and anti-inflammatory agent, for example, Bortezomib, Tranilast, Leflunomide, Mebendazol, Cytarabine and Indoprofen.

In an aspect, the invention features a kit for the treatment of a virus associated neoplasia in a subject. The kit includes an effective amount of a viral lytic induction agent and a radiolabeled analog of 2'-deoxy-5-iodo-I-beta-D-arabinofuranosyluracil (FIAU), and directions for using the kit for diagnosis of the neoplasia. The lytic induction agent can be a proteasome inhibitor, a microtubule disrupting agent, a glucocorticoid or steroid hormone, a nucleoside analog, and anti-inflammatory agent, for example, Bortezomib, Tranilast, Leflunomide, Mebendazol, Cytarabine and Indoprofen. The neoplasia associated with infection is, for example, Epstein Bar Virus (EBV) or Kaposi's sarcoma herpes virus, such as lymphoma, gastric carcinoma, Kaposi's sarcoma, or nasopharyngeal carcinoma

In an aspect, the invention features a method of killing a bacteria comprising contacting the bacteria with a radiolabeled analog of 2'-deoxy-5-iodo-I-beta-D-arabinofuranosyluracil (FIAU). The radiolabel can be an alpha, beta, or gamma emitter, for example ⁹⁰Y, ¹⁸⁶Re, ¹⁸⁸Re, ⁶⁴Cu, ⁶⁷Cu, ²¹²Pb, ²¹²Bi, ¹²³I, ²¹¹At, ²¹³Bi and ¹³¹I. Bacteria include both Gram positive and Gram negative bacteria.

In another aspect, the invention features a method of treating a bacterial infection in a subject. the method comprising administering to the subject an effective amount of a radiolabeled analog of2'-deoxy-5-iodo-I-beta-D-arabinofuranosyluracil (FIAU). The radiolabel can be an alpha, beta, or gamma emitter, for example ⁹⁰Y, ¹⁸⁶Re, ¹⁸⁸Re, ⁶⁴Cu, ⁶⁷ Cu, ²¹²Pb, ²¹²Bi, ¹²³I, ²¹¹At, ²¹³Bi and ¹³¹I. Bacteria include both Gram positive and Gram negative bacteria.

### Definitions

By "naturally infected" is meant an infection that occurs without the aid of human intervention. Examples of human intervention include gene therapy, cellular transformation, or cellular transfection.

By "proteasomal inhibitor" is meant a compound that reduces a proteasomal activity, such as the degradation of a ubiquinated protein. Exemplary proteasome inhibitors include, but are not limited to, bortezomib, Lactacystin, and MG132.

By "microtubule disrupting agent" is meant an agent that interferes with the biological function, stability, or growth of a microtubule. Exemplary agents include (colchicine,demecolcine, vinblastine, vincristine, podophyllotoxin,and nocodazole

By "glucocorticoid" is meant a synthetic or naturally occurring corticosteroid drug or hormone that is a functional or structural analog of an endogenous glucocorticoid produced by the adrenal gland. Exemplary glucocorticoids include prednisolone, methylprednisolone, hydrocortisone, betamethasone and dexamethasone.

By "steroid hormone" is meant a synthetic or naturally occurring drug or hormone having a tetracyclic cyclopentaphenanthrene skeleton.

By "nucleoside analog" is meant a synthetic or naturally occurring compound having a sugar and a purine or pyrimidine base. Exemplary nucleoside analogs include 2'-fluoro-2'-deoxy-β-D-5-iodouracil-arabinofuranoside, as well as various deoxyadenosine analogues (e.g., Didanosine, Vidarabine) deoxycytidine analogues (e.g., Cytarabine, Emtricitabine, Lamivudine, Zalcitabine), deoxyguanosine analogues (e.g., Abacavir), deoxythymidine analogues (e.g., Stavudine, Zidovudine, Azidothymidine (AZT)), and deoxyuridine analogues (e.g., Idoxuridine, Trifluridine). Leflunomide, Indoprofen, Mebendazole

By "an anti-inflammatory" is meant an agent that inhibits inflammation or a symptom thereof. Exemplary anti-inflammatory agents include, but are not limited to, *N*-(3,4-dimethoxycinnamoyl)anthranilic acid (tranilast), N-(4'-trifluoromethylphenyl)-5-methylisoxazole-4-carboxamide (leflunomide), and non-steroidal anti-inflammatory drugs (NSAIDs).

By "ameliorate" is meant decrease, suppress, attenuate, diminish, arrest, or stabilize the development or progression of a disease.

By "analog" is meant a molecule that is not identical, but has analogous functional or structural features.

By "alteration" is meant a change (increase or decrease) in the expression levels or activity of a gene or polypeptide as detected by standard art known methods such as those described herein. As used herein, an alteration includes a 10% change in expression levels, preferably a 25% change, more preferably a 40% change, and most preferably a 50% or greater change in expression levels.

The term "neoplasia" includes malignancies characterized by excess cell proliferation or growth, or reduced cell death. In specific embodiments, the term "cancer" includes but is not limited to carcinomas, sarcomas, leukemias, and lymphomas. The term "cancer" also includes primary malignant tumors, e.g., those whose cells have not migrated to sites in the subject's body other than the site of the original tumor, and secondary malignant tumors, e.g., those arising from metastasis, the migration of tumor cells to secondary sites that are different from the site of the original tumor.

The language "therapeutically effective amount" or a "therapeutically effective dose" of a compound is the amount necessary to or sufficient to provide a detectable improvement in of at least one symptom associated or caused by the state, disorder or disease being treated. The therapeutically effective amount can be administered as a single dose or in multiple doses over time. Two or more compounds can be used together to provide a "therapeutically effective amount" to provide a detectable improvement wherein the same amount of either compound alone would be insufficient to provide a therapeutically effective amount.

The term "imaging compound" is intended to include compounds that are capable of being visualized or that are useful for visualizing a cell, tissue, or organ. For example, by planar gamma imaging, single photon emission computed tomography (SPECT) or positron emission tomography (PET). The compounds may be radiolabeled or fluorescent. In specific embodiments, the compounds are nucleosides or nucleoside analogs that bind to a kinase, e.g., a thymidine kinase.

The phrase "viral lytic induction agent" is understood as an agent that induces a virus to change its pattern of gene expression and begin to express RNA and proteins associated with the production of viral particles. Indications that viral lysis has been induced include, but are not limited to, an increase in the expression of a viral polypeptide or polynucleotide (e.g., a polypeptide associated with the lytic stage) or an increase in viral replication. Methods for assaying the level of polypeptide expression include, but are not limited to, immunoassays (e.g., ELISA, Western blot, or radioimmuno assays) to measure the level of the polypeptide. Methods for assaying polynucleotide expression are known in the art and/or are described herein. Such methods include microarray analysis, Northern blot analysis, and RT-PCR, using any appropriate fragment prepared from the nucleic acid molecule as a hybridization probe. The level of gene expression in the presence of the candidate compound is compared to the level measured in a control culture medium lacking the candidate molecule.

The phrase "pharmaceutically acceptable carrier" is art recognized and includes a pharmaceutically acceptable material, composition or vehicle, suitable for administering compounds used in the methods described herein to subjects, *e.g*., mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

As used herein, the term "imaging" refers to the use of technology to visualize a detectable compound after administration to a cell, tissue, or organ. In one embodiment, imaging is carried out by measuring the energy emitted by the compound after localization of the compound following administration. Imaging technologies, such as positron emission tomography (PET), SPECT-CT, and the like are applied.

As used herein, "positron emission tomography imaging" or "PET" incorporates all positron emission tomography imaging systems or equivalents and all devices capable of positron emission tomography imaging. The methods of the invention can be practiced using any such device, or variation of a PET device or equivalent, or in conjunction with any known PET methodology. See, e.g., U.S. Pat. Nos. 6,151,377; 6,072,177; 5,900,636; 5,608,221; 5,532,489; 5,272,343;5,103,098. Animal imaging modalities are included, e.g., micro-PETs (Corcorde Microsystems, Inc.).

### Brief Description of the Drawings

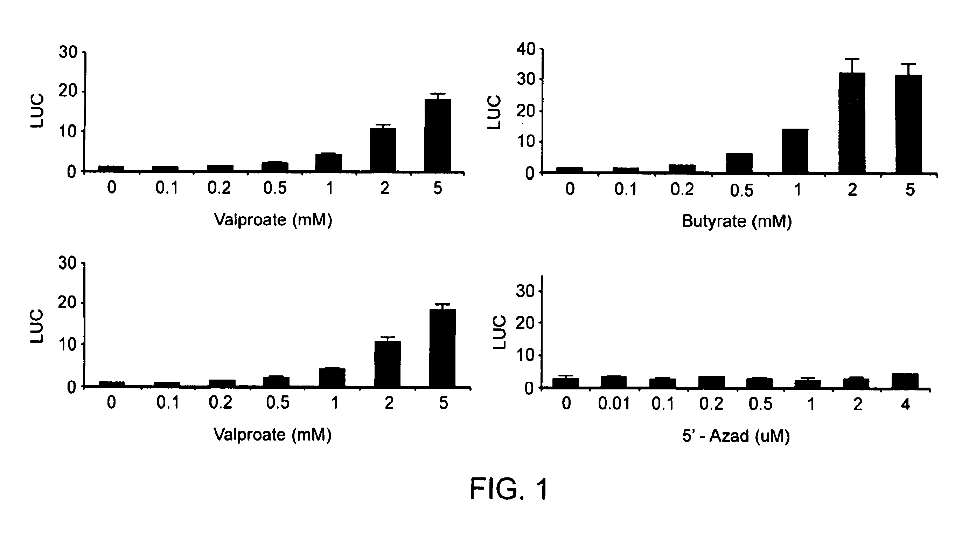

Figure 1 provides four graphs quantitating luciferase activity in AGS cells. Luciferase was expressed under the control of the Zta promoter (-5787+13). The Zta promoter was activated by TPA, butyrate, and valproate in AGS cells. It was not activated by 5'azadeoxycytidine, a DNA methyltransferase inhibitor.

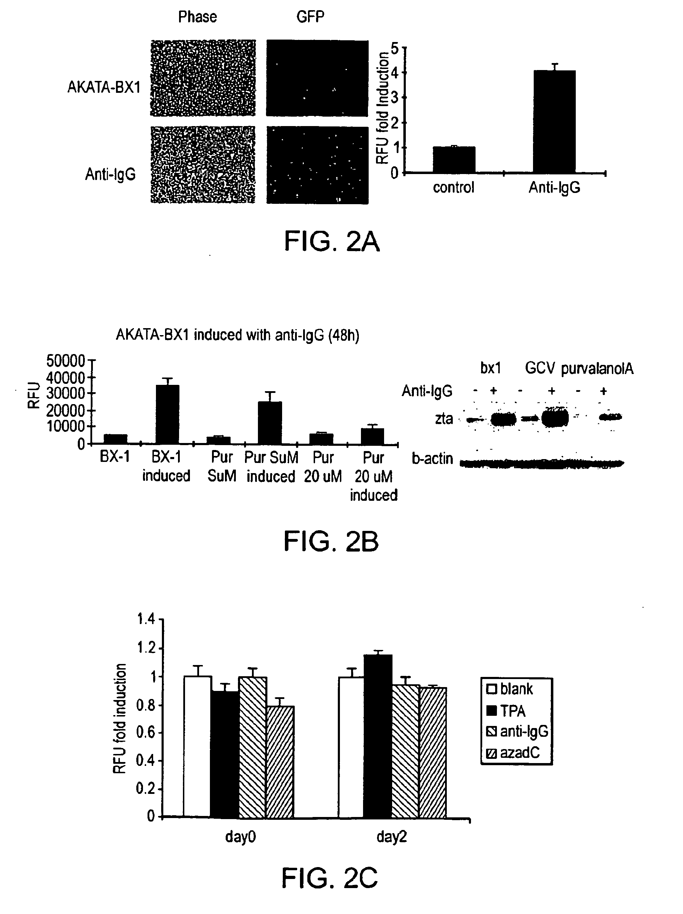

Figures 2A-2C shows GFP signal in AKATA-BX1 cells following recombinant viral EBV induction. Figure 2A includes four micrographs showing that GFP signal in recombinant EBV virus is induced by treatment with anti-IgG. Phase contrast and GFP expression were examined using an inverted fluorescence microscope. GFP signal in AKATA-BX1 was much brighter when induced with anti-IgG. GFP signal enhancement with EBV lytic induction was detected by a microplate reader. Figure 2B is a graph showing that the signal induction was suppressed by drugs that inhibit lytic infection. Purvalanol A inhibited the EBV immediate early gene ZTA expression. Figure 2C is a graph showing that the GFP signal in Hela cells with the CMV promoter driven by GFP was not activated with TPA, antiIgG, or 5 '-azadeoxycytidine.

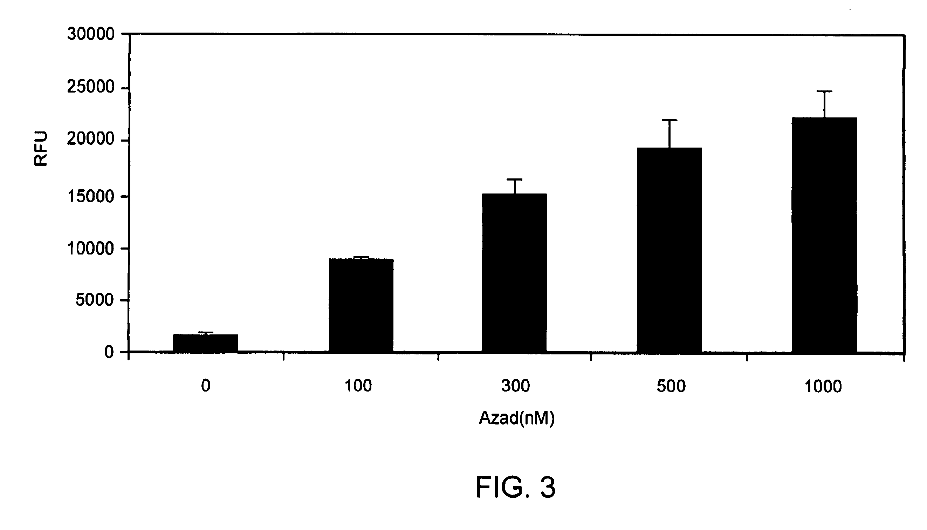

Figure 3 is a graph showing that 5 azadeoxycytidine induced lytic infection of GFP signal in AKATA-BX1 cells.

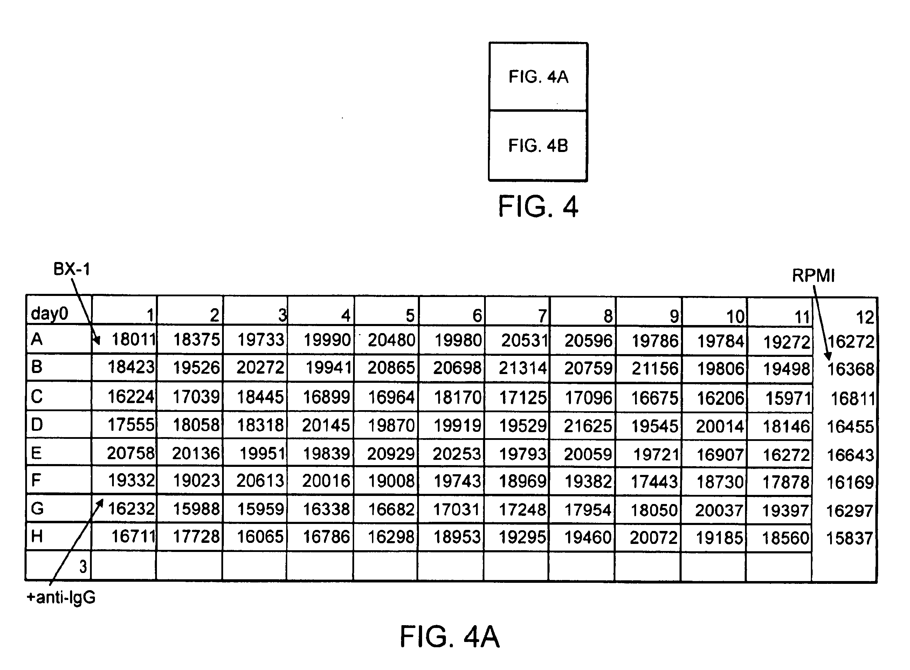

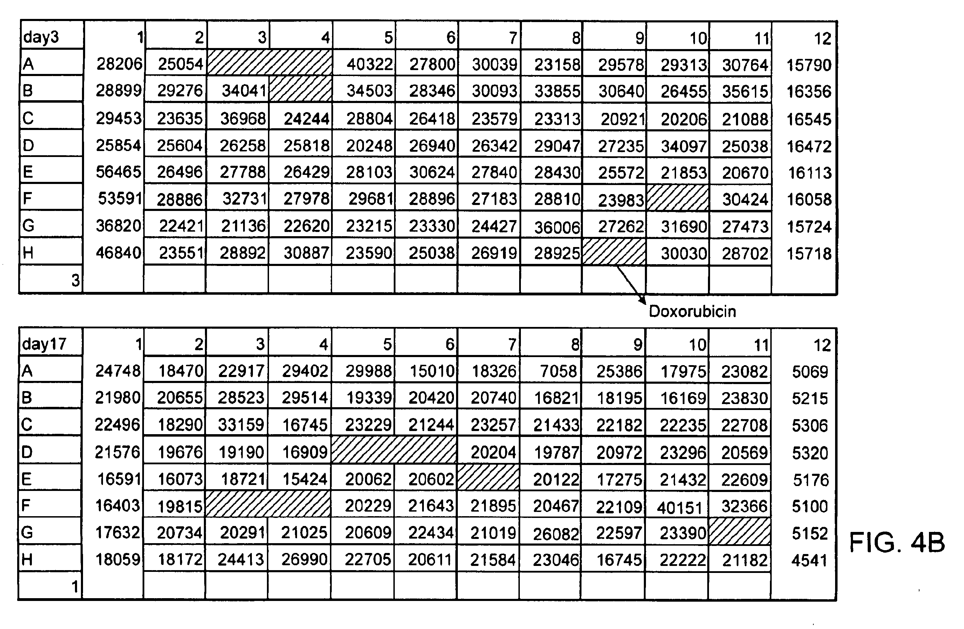

Figure 4 shows a typical readout of a GFP whole virus assay. In the middle 10 columns are 80 drugs from the library. On the right panel is the plain medium without cell or drugs to reduce the background. On the left panel, the first 4 wells are cells without any treatment, as a negative control, and the lower 4 wells are cells treated with lytic induction drugs, as a positive control. The GFP reading was done on day 0 to eliminate drugs with auto fluorescence. On day 3 the positive control wells already show a remarkable increase in GFP signal, any readings comparable to this are considered as hits. Longer incubation showed some wells have readings below average. Suppression hits were picked after day 10.

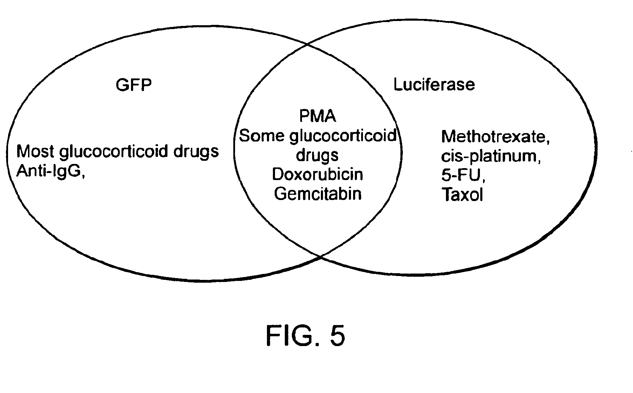

Figure 5 is a Ven diagram showing that drugs known to induce lytic infection in EBV were verified in the two assays.

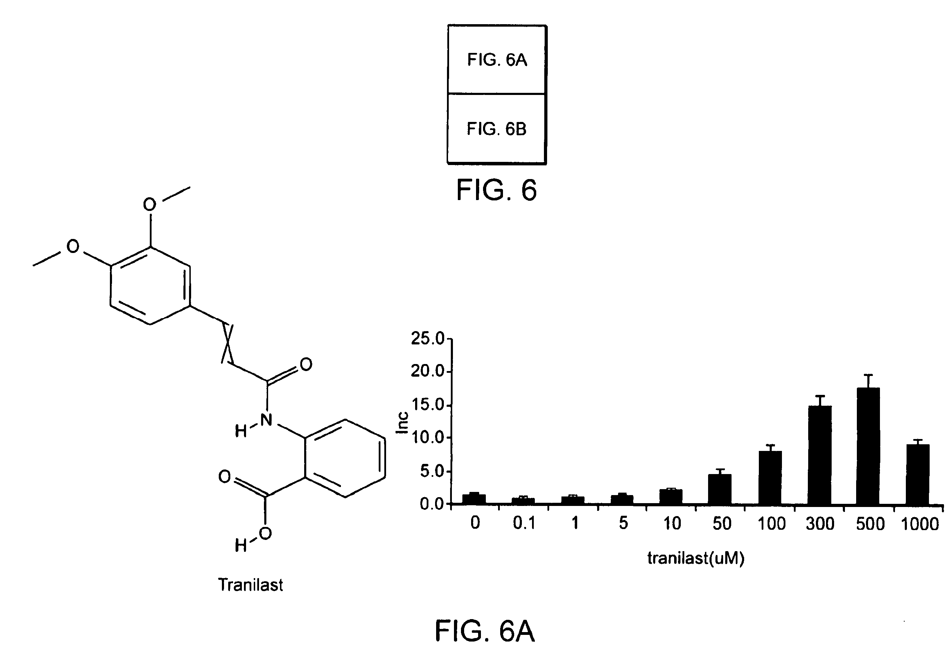

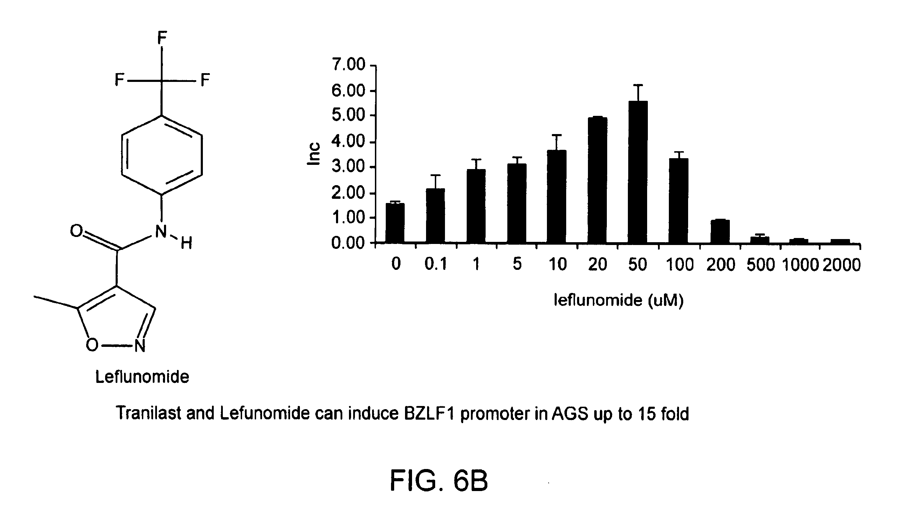

Figure 6 shows that the compounds Tranilast and Lefunomide can induce BZLF1 promoter in AGS up to 15 fold.

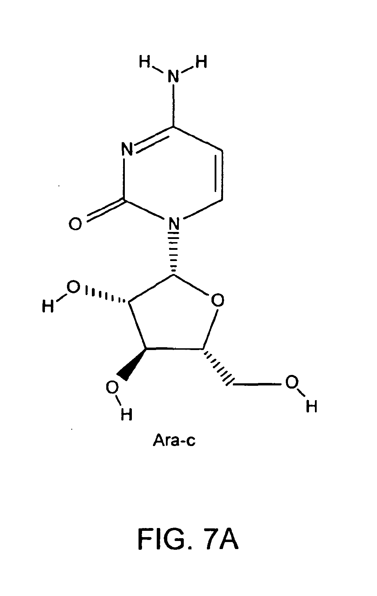

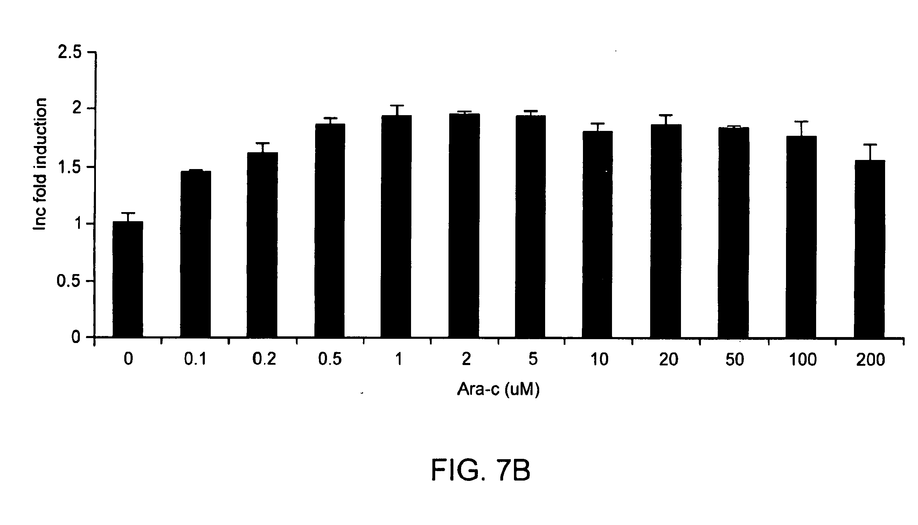

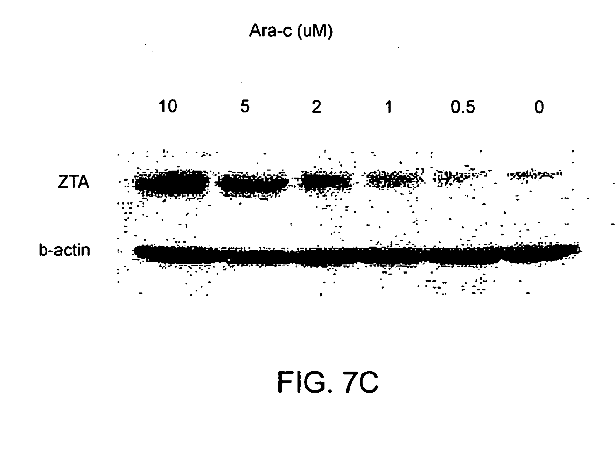

Figures 7A-7C show Cytarabine (Ara-c) results. Ara-c induced lytic EBV infection in B cells and epithelial cells *in vitro.* Figure 7A shows the structure formula of Ara-c. Figure 7B is a graph showing that Ara-c induced BZLF1 promoter in a luciferase assay. Figure 7C is a Western blot showing that Ara-c induced ZTA expression in LCL cells.

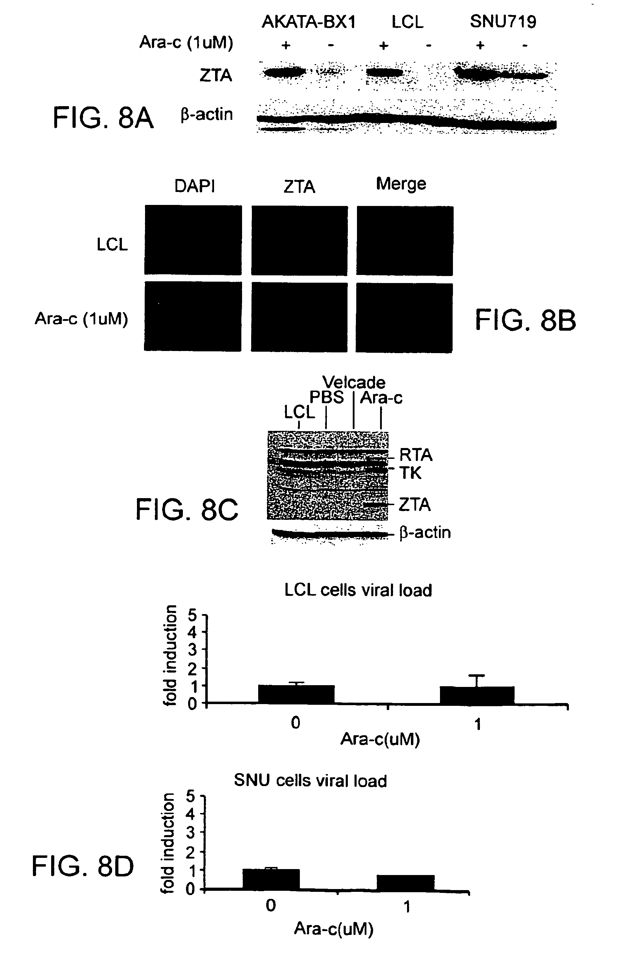

Figures 8A-8D show results with Ara-C. Ara-c induced lytic EBV infection in B cells and epithelial cells in vitro. Figure 8A is a Western blot showing results in EBV positive cell lines AKATA, LCL and SNU719 that were treated with 1 µM Ara-c for 48 hours, and subsequently assayed for expression of the EBVIE gene by assaying the BZLF1 protein by western blot. The immunoblot showed that Ara-c induced BZLF1 protein expression in the EBV positive cell lines. Figure 8B provides six micrographs showing EBV-positive lymphoblastoid cells, which were treated 1 µM Ara-c for 48 hours. Immunofluorescence assays were performed to detect BZLF 1 protein. Cells were stained for BZLF1 (red) and nuclei were stained with DAPI(blue). 40% of LCL cells were induced into lytic infection by I µM Ara-c. Figure 8C is a Western blot showing Ara-c induced EBV early lytic gene TK expression. Figure 8D provides two graphs showing that Ara-c did not activate viral replication of EBV. LCL and SNU719 cells were treated with 1 µM Ara-c for 48 hours. Real-time PCR indicated that EBV viral DNA copy number was not amplified after Ara-c treatment.

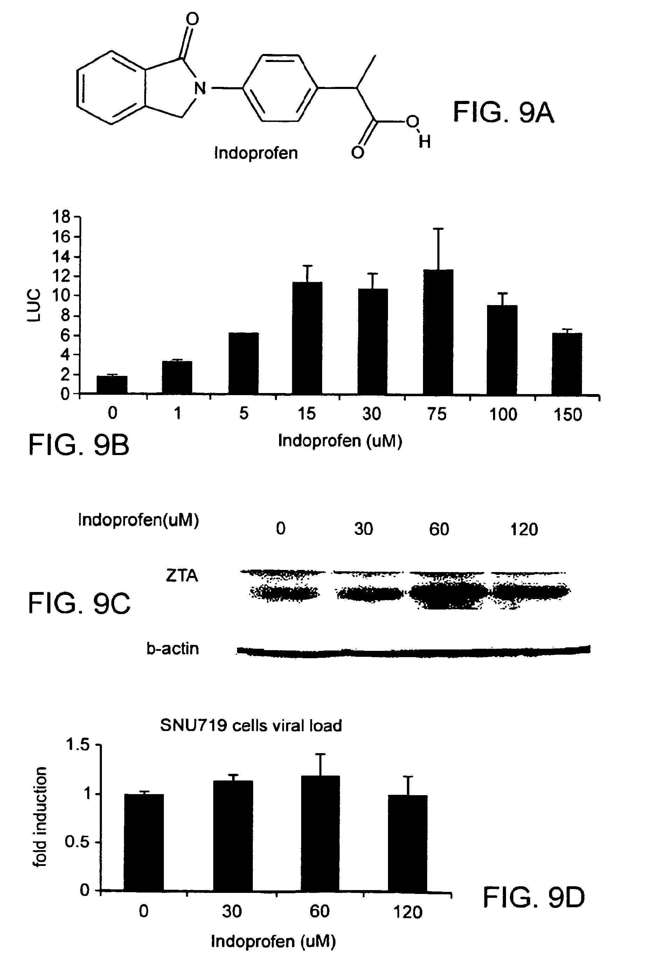

Figures 9A-9D show indoprofen results. Figure 9A shows the structure formula of Indoprofen. Figure 9B is a graph quantitating results of a luciferase assay in AGS showing that Indoprofen can induce BZLF 1 promoter at 10µM. Figure 9C is a Western blot showing that Indoprofen induced ZTA expression in SNU719 cells. Figure 9D is a graph showing that Indoprofen did not induce EBV viral replication in SNU719 cells.

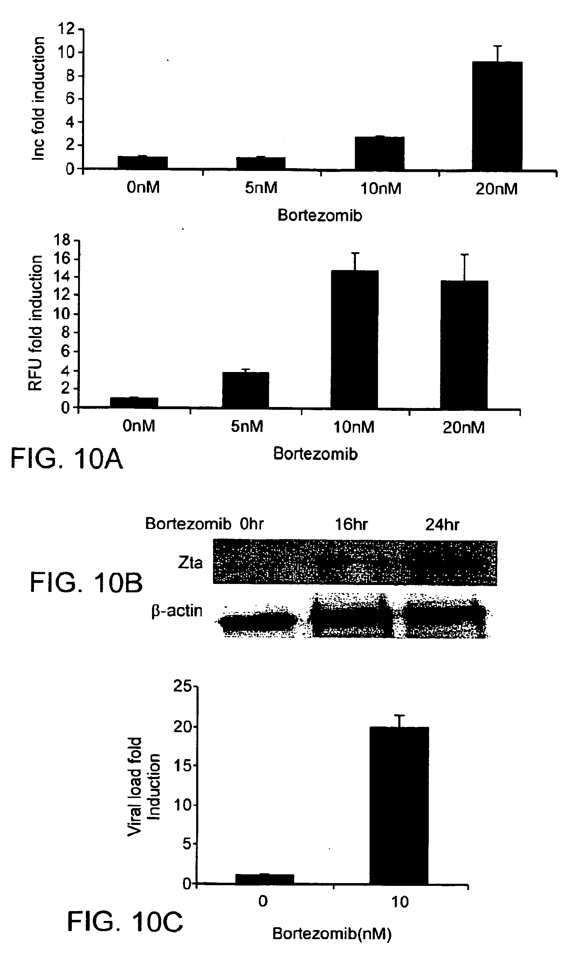

Figures 10A-10C show Bortezomib results. Figure 10A shows two graphs. Figure 10A (left panel) shows quantitation of results in a luciferase assay in AGS cells, showing that Bortezomib induced the BZLF1 promoter at 10 nM. Figure 10B (right panel) shows that at 10 nM, Bortezomib induced GFP signal in the GFP assay in AKATA-BX1 cells. Figure 10B shows that Bortezomib-induced ZTA expression in Rael cells. Figure 10C shows that Bortezomib-induced EBV viral replication in Rael cells. Rael cells were treated with 20nM Bortezomib for 8 hours, and DNA was collected after 48 hours. Real-time PCR indicated that EBV viral DNA copy number was increased 20-fold.

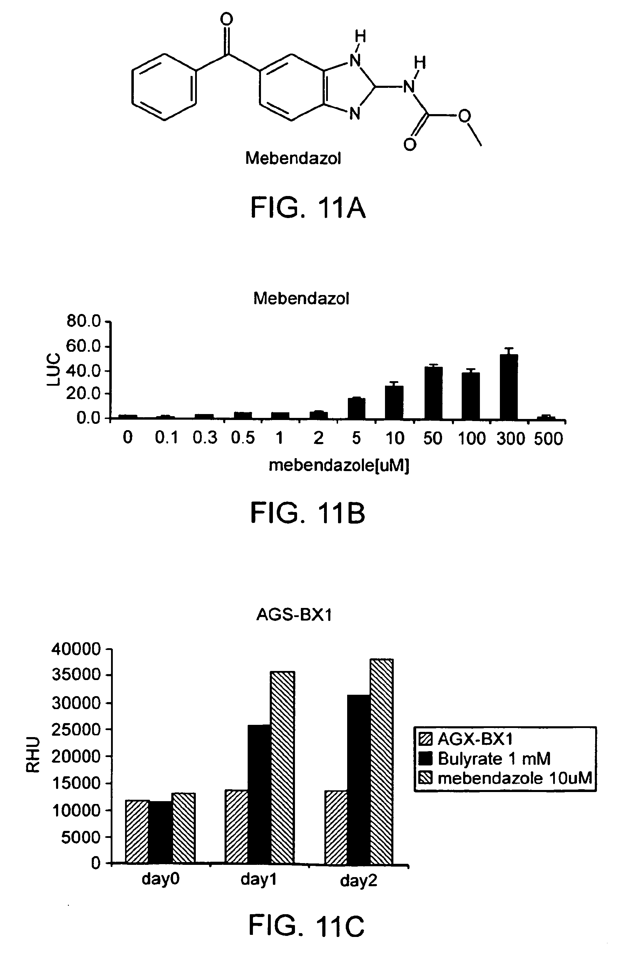

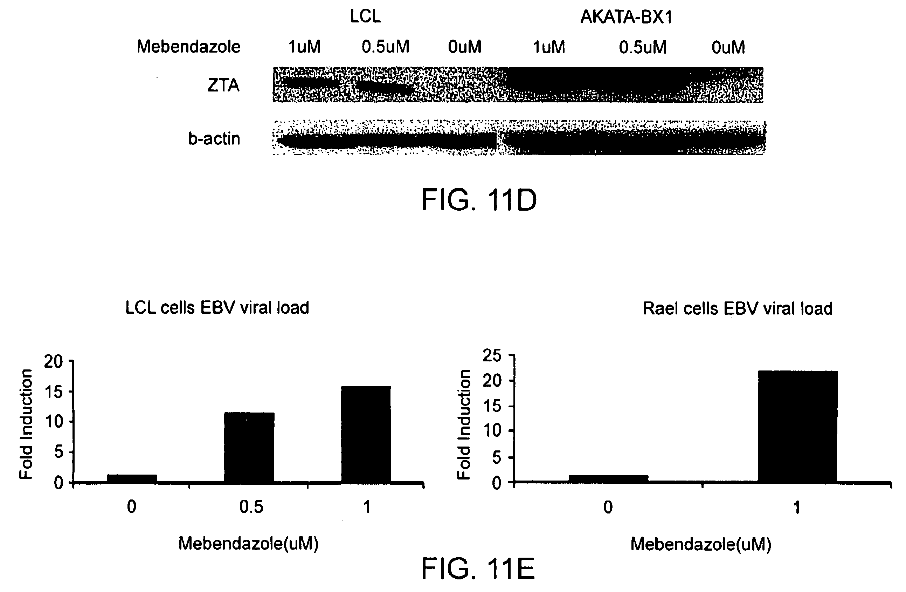

Figure 11A-11E show Mebendazol results. Figure 11A shows the structure formula of Mebendazol. Figure 11B is a graph showing the quantitation of results in a Luciferase assay in AGS cells. These results show that Mebendazole induced the BZLF1 promoter at 1µM. Figure 11C is a graph showing that at 1µM, Mebendazole induced GFP signal in the GFP assay in the AGS-BX1 cells. Figure 11D is a Western blot showing that Mebendazole induced ZTA expression in LCL cells and AKATA cells. Mebendazole also induced EBV viral replication in LCL cells and Rael cells. Figure 11D shows the quantitation of real time PCR results. Cells were treated with 1µM Mebendazole for 48 hours, and DNA was collected. Real-time PCR indicated that EBV viral DNA copy number was increased to 20fold.

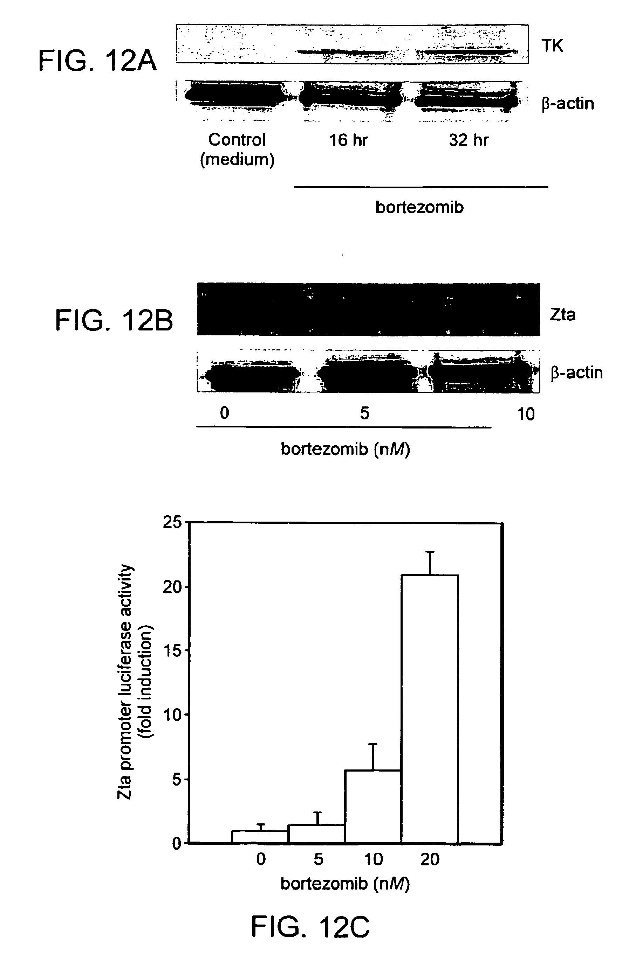

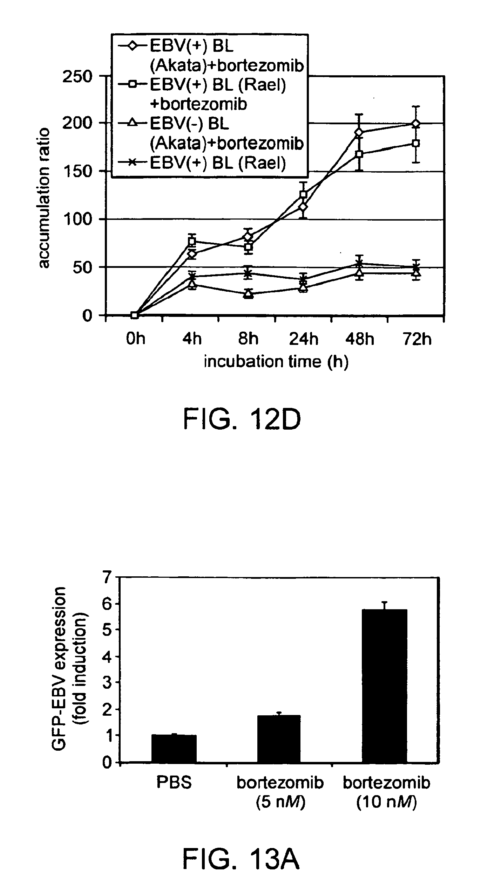

Figures 12A-12D show that bortezomib induces EBV-TK and Zta expression. Figure 12A and 12B are immunoblots showing EBV-TK (A) and Zta (B) expression following treatment of an EBV (+) Burkitt's cell line (Rael) with bortezomib. Total cellular protein was isolated and 10 pg of protein per lane was separated by 12%SDS-PAGE. Figure 12C is a graph showing that Zta luciferase activity increases in a bortezomib dose-dependent manner. AGS-HC13 cells expressing the Zta promoter were treated with bortezomib, and luciferase activity was measured (Figure 12C). Accumulation of [¹⁴C] FIAU increased in EBV(+) Burkitt's [EBV(+) Akata, EBV(+) Real) but not EBV(-) Burkitt's [EBV (-) Akata] following treatment with bortezomib (Figure 12D). "BL" denotes Burkitt's lymphoma.

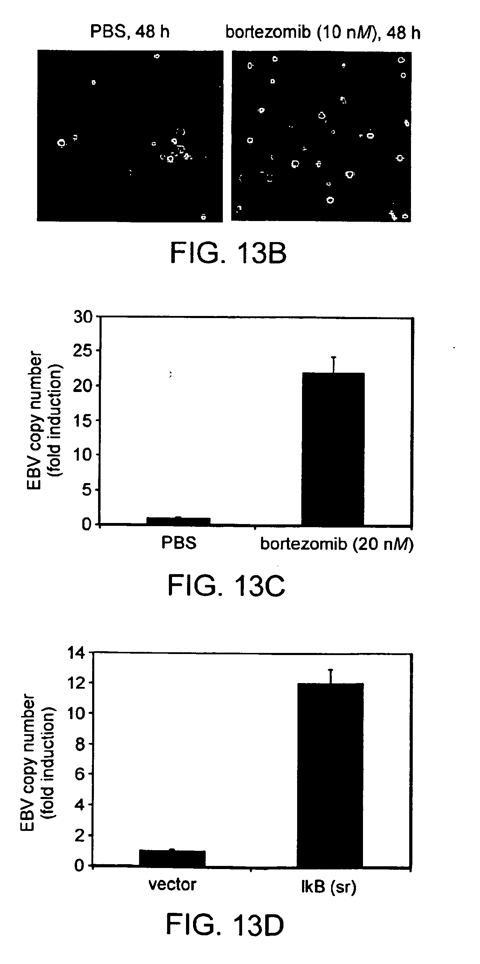

Figures 13A-13D show that Bortezomib up-regulated GFP expression (13A and 13B) and EBV viral load (13C). Figure 13A is a graph showing the effect of treating BX-1 cells expressing GFP with bortezomib at 48 hours. Figure 13B includes two micrographs showing GFP expression in Rael cells at 48 hours. Figure 13C is a graph showing quantitation of viral load as measured by real-time PCR. Figure 13D is a graph showing quantitation of viral load in EBV(+) Rael cells transfected with expression vector carrying I KB super repressor 1KB (sr) and control vector as measured by real-time PCR.

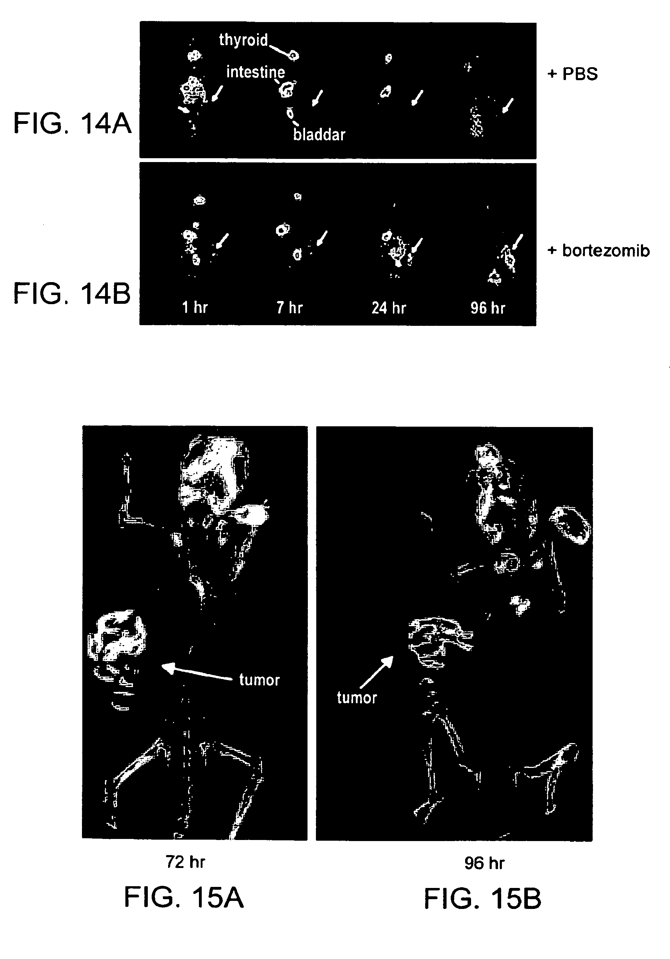

Figures 14A and 14B are images showing a time course of uptake of [¹²⁵I] FIAU by Burkitt's lymphoma xenografts [EBV(+) Akata] following treatment with bortezomib as assessed by planar gamma scintigraphy *in vivo.* Large arrows indicate tumors. The dark area (Figure 14A, small arrow) represents lead shielding of bladder to improve the dynamic range of the images. Each animal has one tumor placed in the hind limb. In Figure 14A, no tumor uptake was evident in animals pretreated with PBS only (control). In Figure 14B, tumors were visualized at later time points in the pretreated animals (2 µg/g bortezomib). Images are from representative animals that were sacrificed for ex vivo biodistribution, with data depicted quantitatively in Table 1.
Figures 15A and 15B show the time course of uptake of [¹²⁵I] FIAU by another EBV(+) Burkitt's xenograft (Akata) by single-photon emission computed tomography/computed tomography, SPECT/CT (SPECT-CT) *in vivo.* Arrows indicate tumors (-1cm in diameter). Each animal has one tumor placed in the flank. Figure 15A shows [¹²⁵I] FIAU tumor uptake at 72 hours after radiopharmaceutical injection. Figure 15B shows [¹²⁵I] FIAU uptake in tumors at 96 hours. Twenty-four hours before radiopharmaceutical administration, animals were treated with bortezomib (2 µg/g, ix.). Tumor tissue is shown in blue with bones (from CT) shown in red.

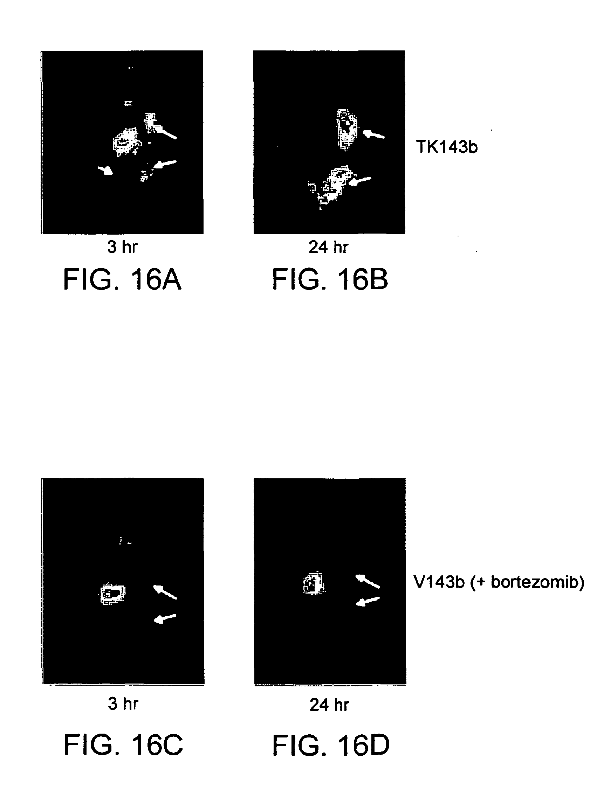

Figures 16A-16D are images showing uptake of [¹²⁵I] FIAU in osteosarcomas by SPECT/CT (SPECT-CT) *in vivo*. Figures 16A and 16B show osteosarcoma 143b that were engineered to constitutively express the EBV-TK (TK143b). Figures 16C and 16D show osteosarcoma 143b tumors that were sham engineered with an empty vector (V143b). Two tumors were present in each animal as indicated with large arrows. The dark area (Figure 16A, small arrow) represents lead shielding of the bladder to improve the dynamic range of the images. Animal shown in Figures 16C and 16D were pretreated with bortezomib (2 µg/g) to determine whether the agent led to up-regulation of a cellular kinase that might account for FIAU phosphorylation.

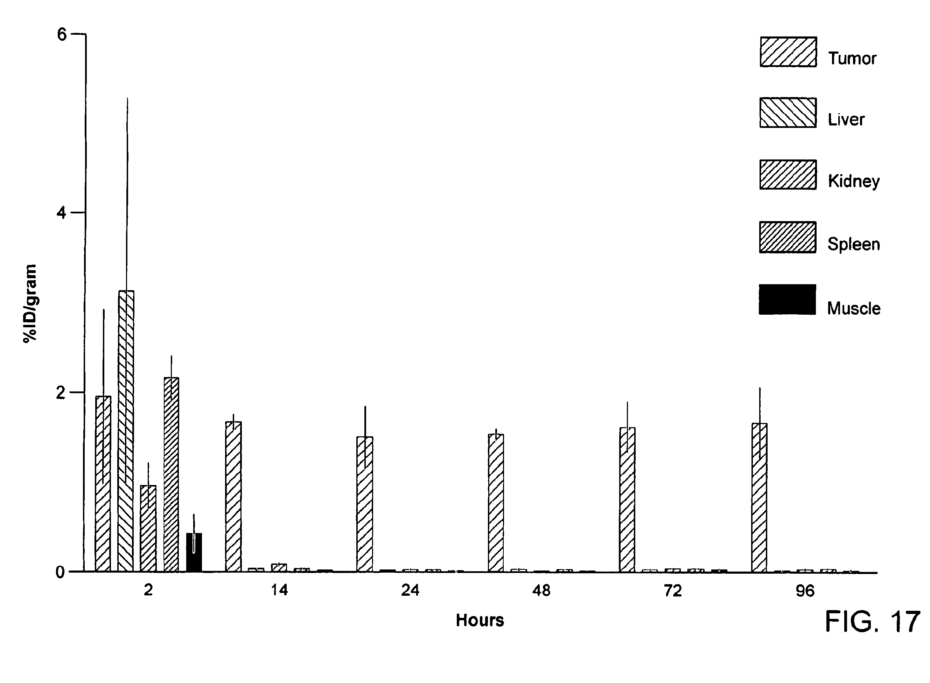

Figure 17 is a graph showing [¹²⁵I]FIAU tissue distribution in a murine xenograft model. [¹²⁵µCi] FIAU was administered intravenously to SCID mice engrafted with EBV-TK(+) tumors. Animals (3-4 at each time point) were sacrificed and tissue distribution measured. The percent of the injected dose (ID) per gram tissue is shown.

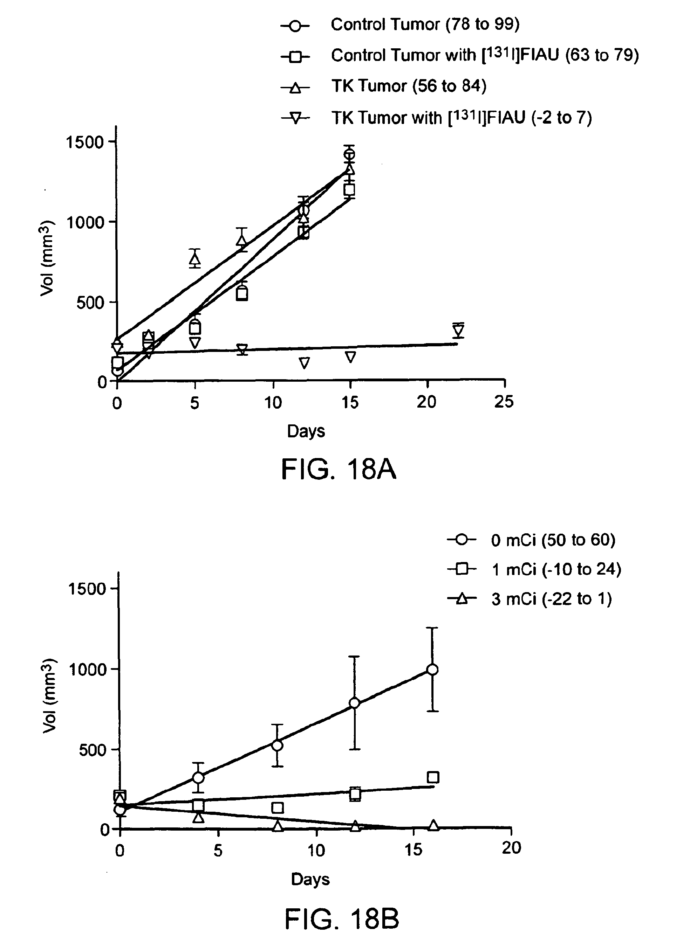

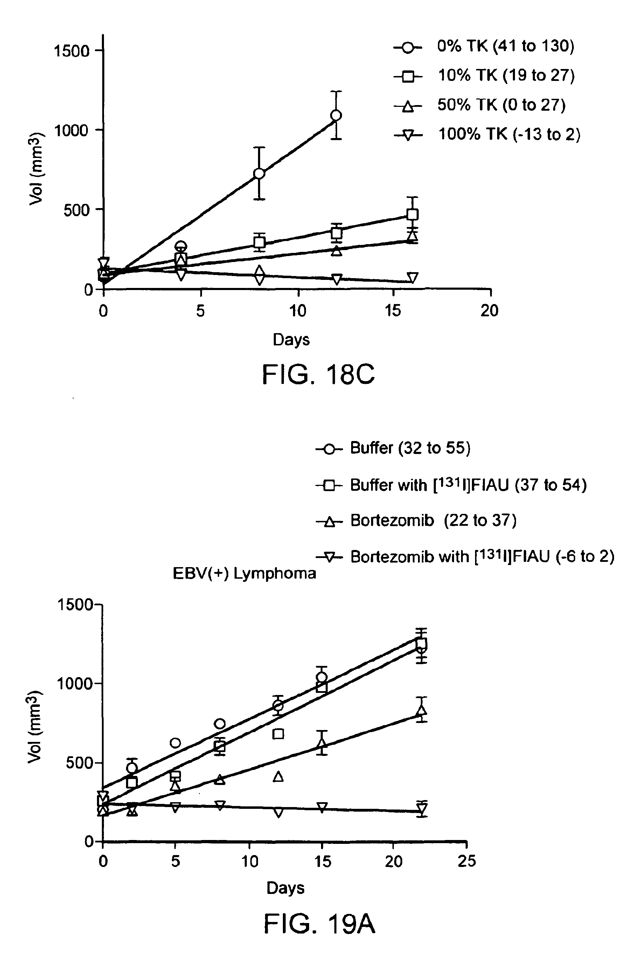

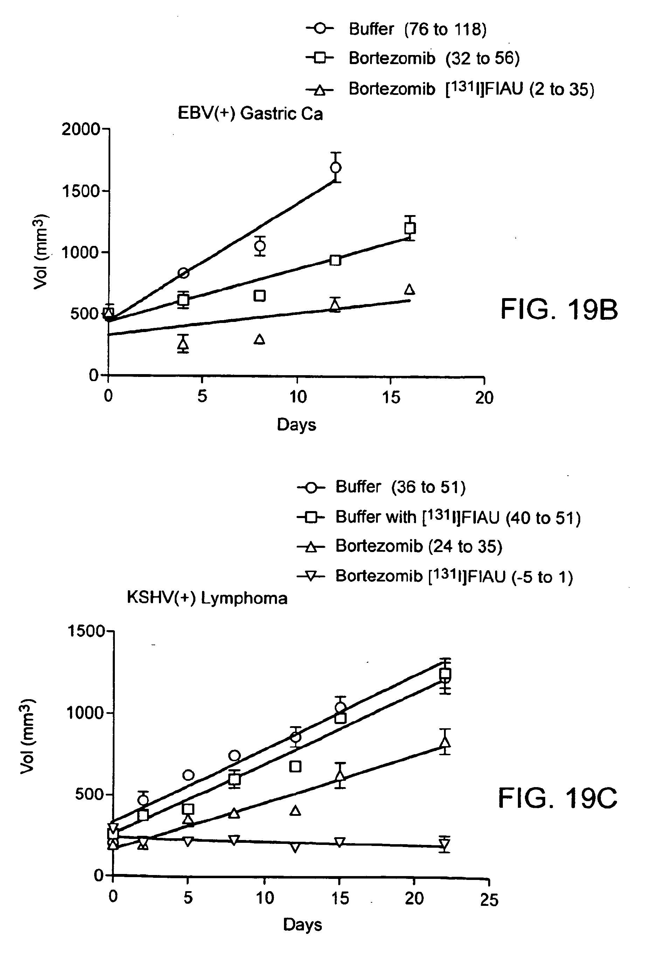

Figures 18A-18C are graphs showing tumor growth curves. Figure 19A shows tumor growth mice with control tumors (human osteosarcoma 143B cells with empty vector) or TK expressing tumors (human osteosarcoma 143B cells expressing EBV TK) were treated IV with 1.6 mCi [¹³¹I]FIAU or buffered saline. Figure 19B show tumor growth dose response. Mice with EBV-TK expressing tumors were treated with buffered saline, 1 mCi [¹³¹I]FIAU or 3 mCi [¹³¹I]FIAU. Figure 18C shows tumor growth in tumors resulting from engraftment of admixtures of EBV-TK expressing and control tumor cells that were treated with 1.7 mCi [¹³¹I]FIAU. Each time point corresponds to 3 animals. The mean, SEM, and least squares linear regression are plotted. The confidence intervals (CI, 95%) of the slopes of best fit linear regression of tumor growth curves are shown in parentheses in the legend.

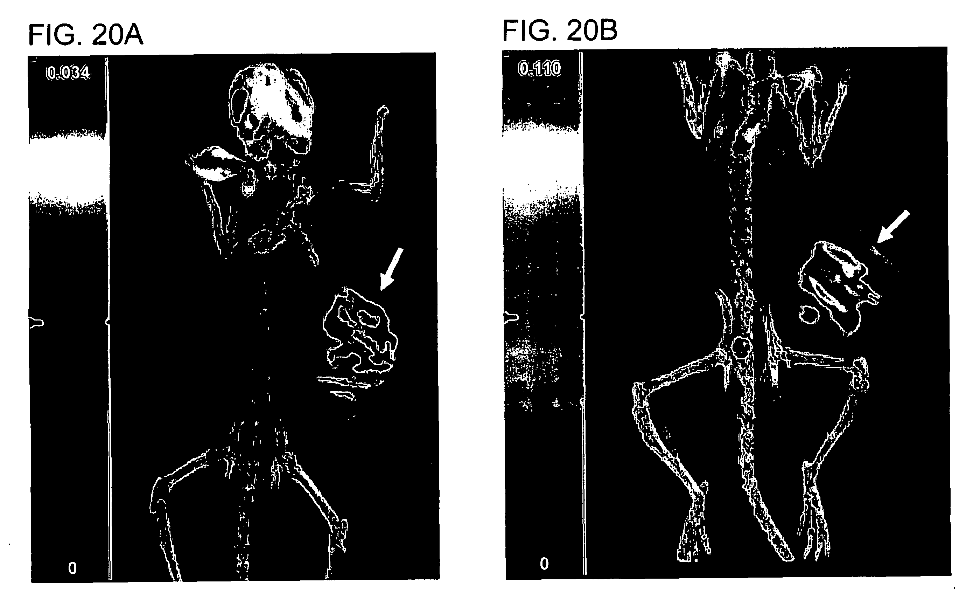

Figures 19A-20C show tumor growth curves in murine xenografts. Figure 19A shows EBV(+) Burkitt's lymphoma (Rael)I Figure 20B shows EBV(+) gastric adenocarcinoma (KT); Figure 19C shows KSHV(+) primary effusion lymphoma (BCBL1) were injected intravenously with buffered saline, buffered saline followed 24 hours later by [¹³I]FIAU, bortezomib, or bortezomib followed 24 hours later by [¹³¹I]FIAU. Each time point corresponds to 3 animals. The mean, SEM, and least squares linear regression are plotted. The confidence intervals (CI, 95%) of the slopes of best fit linear regression of tumor growth curves are shown in parentheses in the legend.
Figure 20A and 20B show [¹²⁵1]FIAU SPECT-CT imaging of tumors following bortezomib treatment. Figure 20A shows an EBV(+) gastric carcinoma (KT) tumor at 72 hours p.i. Figure 20B shows a KSHV(+) lymphoma (BCBL1) at 48 hours p.i.. Yellow arrows indicate tumor location. Color bars indicate the range of [¹²⁵I]FIAU uptake as % ID/g (0.68% in (a) and 1.53% in (b)).

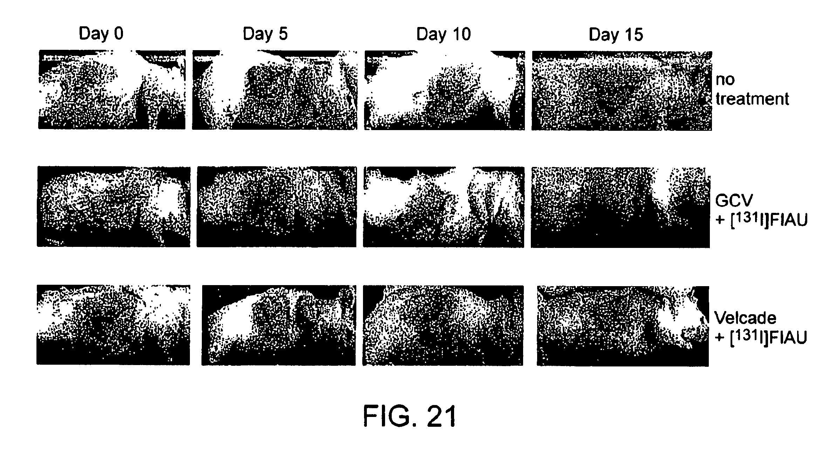

Figure 21 shows enzymatic molecular radiotherapy for KSHV tumors (BCBL 1).

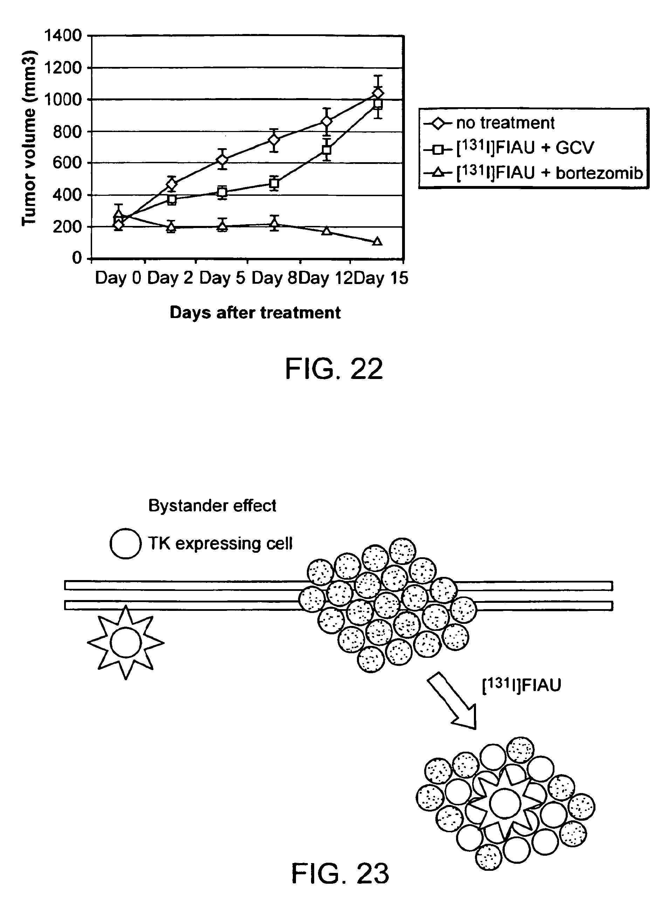

Figure 22 is a graph showing a reduction in KSHV tumor size following therapy with [¹³¹I]FIAU and bortezomib.
Figure 23 is a schematic diagram showing the "bystander effect."

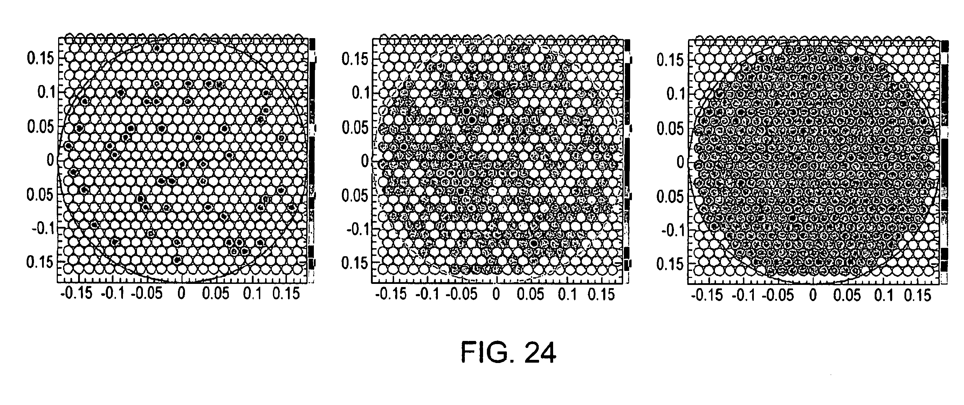

Figure 24 is a depiction of the geometric model used for the Monte Carlo cell-level dosimetry calculation. Cell-level activity distribution for a cross section through the middle of the cell cluster is shown for 10, 50 and 100% of the cells taking up activity. To examine the effect of activity distribution as opposed to total uptake, the total activity in the tumor is kept constant. Accordingly, as shown by the color scale, cells in the 10% scenario have more activity per cell than those in the 100% model (red intensity vs grey); the activity is uniformly distributed within each cell.

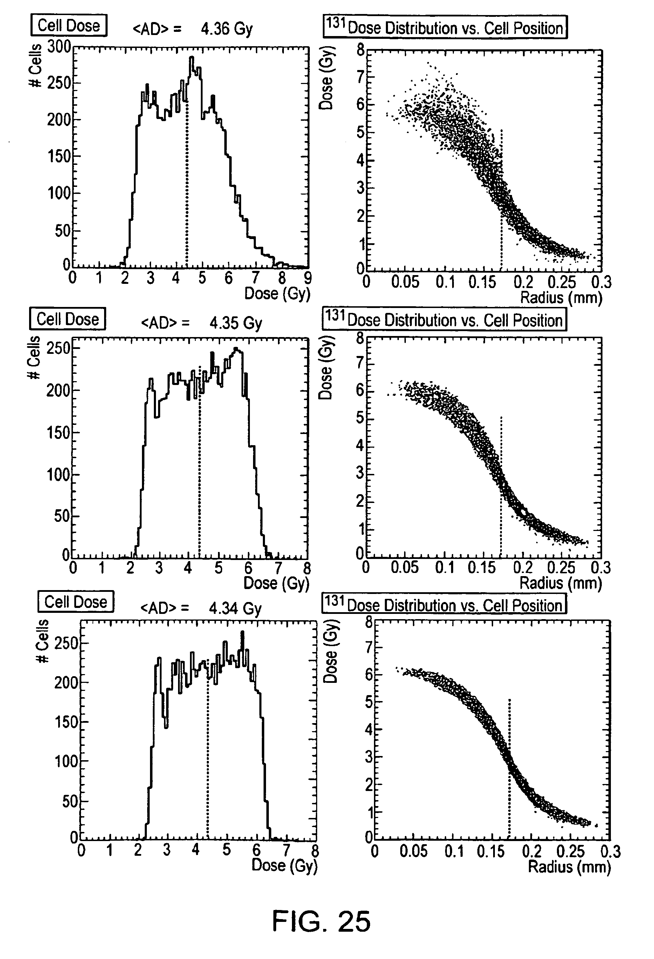

Figure 25 shows dose volume histograms (first column) and spatial absorbed dose scattergram (2nd column) resulting from 2.5 million ¹³¹I decays for 10 (top row), 50 (middle row) and 100% (bottom row) of cells transfected. The dose-volume histograms (1st column) show the number of cells (y-axis) that have received a particular absorbed dose (x-axis); the dotted vertical line corresponds to the mean of the distribution which is also listed above the figure. Every dot in the scattergrams (column 2) corresponds to the absorbed dose received by an individual cell at that position; the dotted line corresponds to the position of the spherical tumor surface.

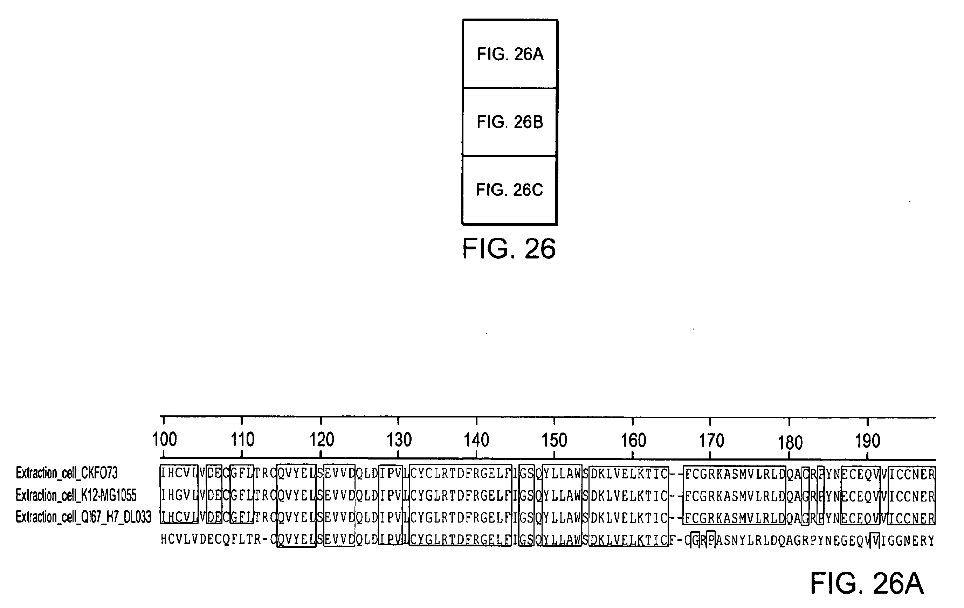

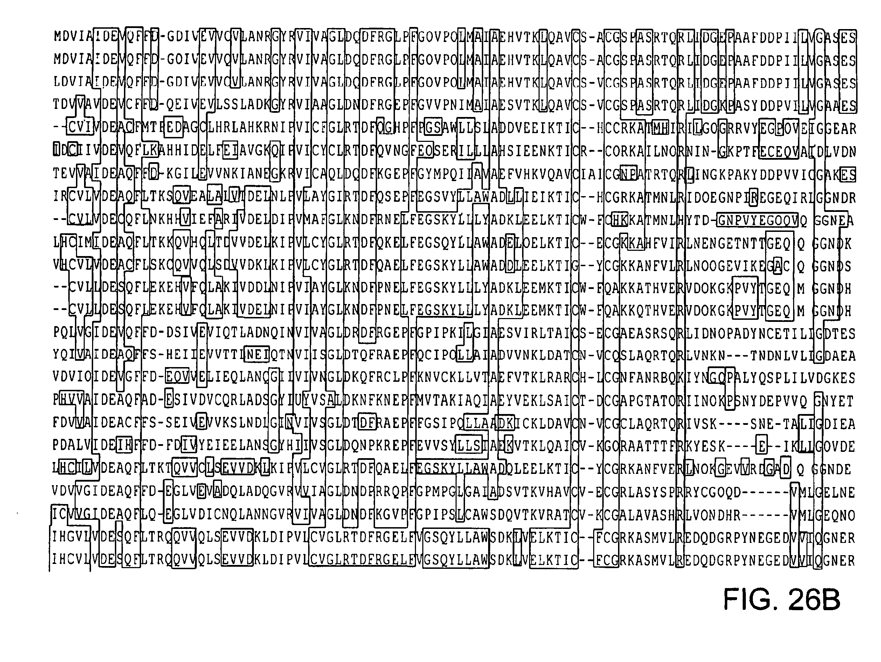

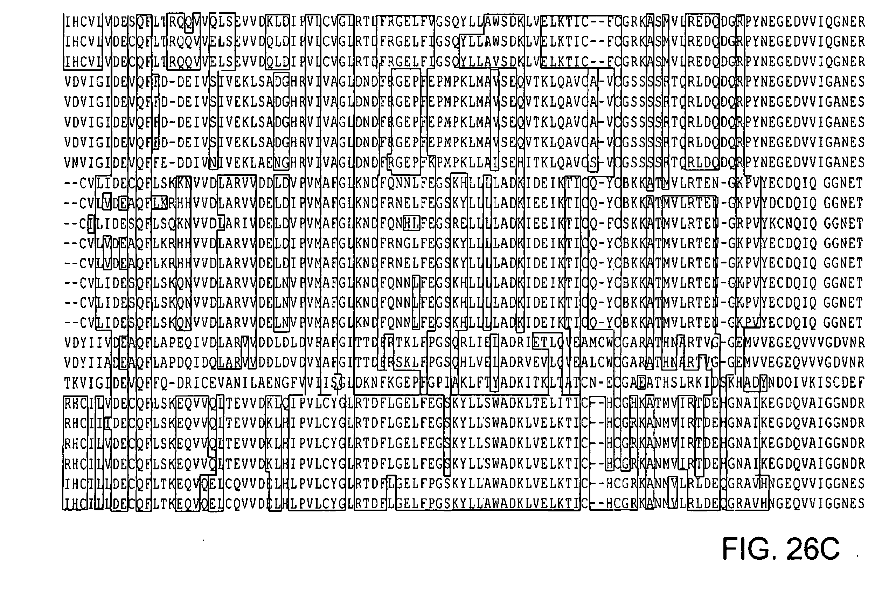

Figure 26 provides the sequences of bacteria and their corresponding thymidine kinases, which are useful in the methods of the invention.

### DETAILED DESCRIPTION OF THE INVENTION

The invention features compositions that are useful for tumor imaging, for the treatment or prevention of a neoplasia associated with an infection (e.g., a virus or bacterial infection), for selecting an effective therapy for a subject that has a neoplasia, and for monitoring therapeutic efficacy. In other embodiments, the invention is useful for the treatment of a bacterial infection (e.g., the treatment of a bacterial containing a bacterial thymidine kinase). In particular embodiments, the invention provides agents to modulate viral gene expression in order to target radiotherapy to tumor tissue. The invention is based, at least in part, on a number of discoveries, which are reported in more detail below. These discoveries include the identification of lytic phase inducing compounds and their use to image tumors, the identification of subjects suffering from diseases associated with latent viral infections who are amenable to treatment using lytic therapy, and the monitoring of therapeutic efficacy in such patients, and the discovery that tumors expressing viral polypeptides could be treated with radiopharmaceuticals.

Epstein-Barr virus (EBV) is associated with a variety of malignancies. The EBV thymidine kinase (TK) is either not expressed or is expressed at very low levels in EBV-associated tumors. As reported herein, EBV-TK expression can be induced *in vitro* with several agents that promote viral lytic induction. *In vitro* assays with ¹²⁻¹⁴C]2'-fluoro-2'-deoxy-β-D-5-iodouracil-arabinofuranoside ([^{14C}]FIAU) and *ex vivo* biodistribution studies with [¹²⁵I]FIAU showed that uptake and retention of radiolabeled FIAU was specific for cells that express EBV-TK. Planar gamma imaging of EBV(+) Burkitt's lymphoma xenografts in SCID mice demonstrated [¹²⁵I]FIAU localization within tumors following treatment with one such induction agent, bortezomib. These results indicate the feasibility of imaging chemotherapy-mediated viral lytic induction by radiopharmaceutical-based techniques such as single photon emission computed tomography (SPECT) and positron emission tomography (PET).

Furthermore, as reported herein, using a murine xenograft model, it was found that [¹²⁵I]2'-fluoro-2'-deoxy-beta-D-5-iodouracil-arabinofuranoside ([¹²⁵I]FIAU) could be targeted to tumors expressing an Epstein-Barr virus (EBV)-thymidine kinase (TK). Tumors expressing this viral polypeptide could be targeted with therapeutic radiopharmaceuticals (e.g., [¹³¹I]FIAU) to slow or stop tumor growth or to achieve tumor regression. These outcomes were achieved in xenografts with tumors that constitutively expressed the EBV-TK, as well as with naturally-infected EBV tumor cell lines. Burkitt's lymphoma and gastric carcinoma required activation of viral gene expression by pretreatment with bortezomib. Marked changes in tumor growth could also be achieved in naturally-infected Kaposi's sarcoma herpes virus (KSHV) tumors following bortezomib activation. Bortezomib-induced enzyme-targeted radiation (BETR) therapy indicates that it is feasible to effect targeted radiotherapy by pharmacologically modulating tumor gene expression. Although specific examples show that Tranilast, Leflunomide, Indoprofen, Cytarabine, Mebendazole and Bortezomib act as EBV lytic induction agents and are useful for the treatment of EBV associated tumors, the invention is not so limited. Methods of the invention may be practiced using any viral lytic induction agent. One skilled in the art appreciates that results obtained with EBV associated tumors are generally applicable to virtually any neoplasia that is associated with a viral infection. Moreover, while the Examples describe results achieved with FIAU and viral (EBV/KSHV) thymidine kinase, such results may be extrapolated to other viruses and viral kinases.

### Virus-associated Neoplasias

Epstein-Barr virus (EBV) is associated with a variety of lymphomas and carcinomas. Kaposi's sarcoma herpes virus (KSHV, HHV-8) is associated with sarcoma and lymphoma. In patients with these tumors, the viral genome serves as a nearly tumor-specific target insofar as these viruses generally infect a tiny percentage of lymphocytes and, in patients, nearly all of the infected cells are tumor cells. Thus, virus-associated metabolic pathways approximate tumor-specific metabolic pathways. The ability to target radioisotopes to herpes virus metabolic pathways has been demonstrated by investigators using vectors engineered with the herpes simplex 1 (HSV1) thymidine kinase (TK) gene to monitor gene expression in the gene therapy setting using radiolabeled nucleoside analogues. The ability to develop new therapies targeting cellular and viral metabolic pathways has been limited by the virtual absence of expression of TK and most of the other enzymes encoded by the EBV genome in tumor cells.

Targeting of radiopharmaceuticals to specific tissues provides an important tool for the therapy of malignancy. Targeting tissue-specific surface antigens, such as CD20 on B cells with monoclonal antibody radioconjugates, has expanded the application of therapeutic radiation beyond what might be achieved with external beam or other spatially-directed approaches. These approaches may be limited by the level of expression or affinity of the antibody for target molecules or the pharmacokinetics of the antibody. Even among lymphomas of B cell lineage, CD20 expression is often inadequate for antibody-targeted therapy. In other instances, the physical characteristics of the antibody conjugate impede delivery to cells in large tumors or in protected compartments such as the central nervous system. Targeting metabolic pathways such as those involved in concentrating iodine in thyroid tissue with isotopic iodine is a well established alternative approach, the more general application of which has been limited by the ability to identify appropriate tumor-specific pathways.

As reported herein, naturally occurring tumor cells harboring EBV could be imaged with a radiolabeled nucleoside analogue, if a pharmacologic inducer of the viral TK was utilized (Fu, D.X., et al. Virus-associated tumor imaging by induction of viral gene expression. Clin Cancer Res 13, 1453-1458 (2007)). This finding has been extended to provide novel compositions and methods for treating a neoplasia harboring a virus as demonstrated *in vivo* using virus harboring tumor cells in EBV and KSHV xenograft models. The method involves targeting neoplastic cells harboring a virus with an inducing agent having a radiotherapeutic nucleoside analogue.

The physical and chemical properties of a radionuclide for use in compositions and methods of the invention are important in its selection for radiotherapy, e.g., the type of particulate emission must be considered. Alpha particles have a high linear energy transfer (LET) effective in cell killing and a range of several cell diameters, 40-80 µm. Beta particles are less densely ionizing and have a range longer than alpha particle emitters so that the tumor distribution requirements are less restrictive. The gamma-ray energies and abundances are also important physical properties, because the presence of gamma rays offers the possibility of external imaging, but also adds to the whole-body radiation dose.

The application of methods of the invention to the treatment of neoplasia is not limited to the treatment of EBV and HSV associated neoplasia, but can be adapted for use with virtually any viral infection associated with a neoplasia. For example, human papilloma virus infections have been associated with the development of cervical cancer; the JC virus has been associated with the development of colon cancer; hepatitis virus B and C have been associated with liver cancer; and human T-lymphotrophic virus has been associated with the development of T-cell leukemia.

### Selection of a treatment method

After a subject is diagnosed as having a neoplasia associated with a viral infection, a method of treatment is selected. Subjects having a neoplasia associated with a viral infection that can be induced to enter the lytic phase are identified as amenable to treatment with a method of the invention. Such subjects can be identified by scanning their bodies to identify the presence or absence of viral lysis in a neoplasia (e.g., a tumor) within the subject. Subjects having tumors that can be visualized, i.e., tumors in which viral lysis has been induced, are identified as amenable to treatment with a method of the invention. Subjects having tumors that cannot be visualized, i.e., tumors in which viral lysis has not been induced, are identified as resistant to treatment with a method of the invention.

### Patient monitoring

The diagnostic methods of the invention are also useful for monitoring the course of a neoplasia in a patient or for assessing the efficacy of a therapeutic regimen. In one embodiment, the diagnostic methods of the invention are used periodically to monitor the size of a virus-associated tumor. In one example, the neoplasia is characterized using a diagnostic assay of the invention prior to administering therapy. This assay provides a baseline that describes the size of the tumor or that describes the susceptibility of the tumor to treatment with a viral lytic induction agent. Additional diagnostic assays are administered during the course of therapy to monitor the efficacy of a selected therapeutic regimen. A therapy is identified as efficacious when a diagnostic assay of the invention detects an increase in viral lytic induction in a cell of the tumor or that detects a decrease in tumor size.

### Lytic Induction Compounds of the Invention

Viral genes have been engineered to serve as reporters in a variety of gene therapy models. The herpes simplex virus (HSV) thymidine kinase (TK) in particular has been widely used. A homologue of the HSV-TK is encoded by Epstein-Barr virus (EBV), a herpes virus associated with a variety of tumors including endemic Burkitt's lymphoma, post-transplant lymphoma, AIDS lymphoma, Hodgkin's lymphoma, nasopharyngeal carcinoma and gastric carcinoma. The EBV-TK will selectively phosphorylate the nucleoside analog, 2'-fluoro-2'-deoxy-b-D-5-iodouracil-arabinofuranoside (FIAU). However, direct planar gamma or PET imaging of EBV-associated tumors with radiolabeled analogs of FIAU is not possible because the enzyme is either not expressed or is expressed at very low levels due to latent viral infection.

Lytic induction results in cell apoptosis and virus production. Intentional lytic induction has been proposed as a potential therapy for EBV associated tumor. Theoretically, this strategy could work on several levels: the lytic infection of EBV can directly kill the host cell, the lytic gene expressed can turn Ganciclovir into its cytotoxic form and kill the host cell, and the lytic antigen can be recognized by CTLs which will help to clean the host cell from body.

The idea of EBV lytic induction as a specific therapy for EBV associated tumors has not been practiced more, because those drugs reported to induce EBV lytic cycle are cytotoxic and cannot be administered at dosages sufficient to induce the EBV lytic cycle in a sufficient number of cells to have a therapeutic effect. Using conventional cytotoxic therapies to reactivate the endogenous EBV genome forfeits the original purpose of specific therapy and the advantage of killing the virus/host tumor cell without hurting normal cells.

The invention provides compositions and methods that induce viral lytic induction. Infection of epithelial cells with EBV results in productive infection, with replication of virus and lysis of infected cells. Immediate-early genes encode regulators of virus gene expression, including the BZLF1 and BRLF1 proteins, which act as switches to initiate lytic infection. The BZLF1 protein inhibits the expression of the interferon-γ receptor and the activity of interferon-γ. Early genes encode proteins that are involved in viral DNA synthesis, such as the viral DNA polymerase and thymidine kinase. Late genes encode structural proteins of the virus, including the viral capsid antigen and the major envelope glycoprotein gp350. After the initial infective stage, the EBV virus and herpes virus, as well as other viruses, may enter a latent stage where active viral production ceases, but the virus remains present. Under certain conditions, the virus exits the latent phase and re-enters the lytic phase.

The present invention provides agents that induce the virus to enter the lytic stage. Such agents are referred to as "viral lytic induction agents." Preferably, such agents are effective at inducing viral lysis without having adverse cytotoxic effects on the host or on normal tissues. Viral lytic induction agents include proteasome inhibitors, anti-tubulin drugs, glucocorticoid and steroid hormones, nucleoside analogs, and anti-inflammatory drugs. Such agents may be administered to subjects having a neoplasia that is associated with a latent viral infection to induce the virus to enter the lytic phase.

### Viral Imaging Agents

Induction of the viral lytic stage results in viral polypeptide production. Such polypeptides can serve as specific markers for the diagnosis and/or monitoring of virus-associated neoplasias. In particular embodiments, the invention provides a substrate for a viral enzyme. Because the substrate is susceptible to imaging, or includes a moiety that allows it to be imaged, it may be visualized using conventional imaging methods, such as PET or SPECT-CD. The viral enzyme concentrates the substrate in the tumor cell, thereby allowing the tumor cells to be visualized.

The ability to image tumor metabolism *in vivo* has broad application as exemplified by the increasing clinical use of positron emission tomography with [¹⁸F]fluorodeoxyglucose (FDG-PET). Of course, the specificity of such scans for tumor tissue is limited insofar as many tissues as well as malignant ones rapidly metabolize glucose. Thus brain, cardiac muscle, and foci of inflammation all yield signal with FDG-PET imaging. The language "effective amount for imaging" of a compound is the amount necessary or sufficient to provide a signal sufficient to visualize the presence or absence of a neoplasm. Neoplasms may be imaged using any method know in the art or described herein, e.g., planar gamma imaging, single photon emission computed tomography (SPECT) and positron emission tomography (PET). The effective amount can vary depending on such factors as the size and weight of the subject, the type of illness, or the particular compound. For example, the choice of the compound can affect what constitutes an "effective amount for imaging". One of ordinary skill in the art would be able to study the factors contained herein and make the determination regarding the effective amount of the compound without undue experimentation. Imaging can allow for the detection of the presence and/or location of the imaging agent bound, for example, to a thymidine kinase. Presence can include below the level of detection or not present, and the location can include none.

In particular, the invention provides agents, including agents that specifically bind to viral polypeptides (e.g., thymidine kinase binding compounds that bind to viral thymidine kinase polypeptide), in an organism and produce a detectable signal that can used to obtain an image of a subject and determine the presence and location of the thymidine kinase, preferably a viral thymidine kinase in subject. Thymidine kinases are particularly well suited for the methods of the invention. The viral thymidine kinases have a consensus sequence in the kinase catalytic domain that is not present in the kinase catalytic domain of mammalian thymidine kinases. Accordingly, compounds with high affinity for viral thymidine kinases exhibit greatly reduced affinity for mammalian thymidine kinases.

The invention utilizes thymidine kinase binding compounds that are easily synthesized and are detectable to an imaging apparatus, e.g., a PET or SPECT instrument. In one embodiment, the compounds are nucleoside analogs that bind to a kinase. In a specific embodiment, the kinase is a thymidine kinase. Thymidine kinase binding compounds for use in the methods of the invention are provided for example in WO2006/002142, incorporated herein by reference.

### Imaging

Generally, imaging techniques involve administering a compound to a subject that can be detected externally to the subject. Images are generated by virtue of differences in the spatial distribution of the imaging agents which accumulate in various locations in a subject. The methods of the present invention, the imaging techniques rely on the compounds being preferentially bound in a subject, e.g., viral thymidine kinase. The spatial distribution of the imaging agent accumulated in a subject, e.g., tumor volume, may be measured using any suitable means, for example, planar gamma imaging, single photon emission computed tomography (SPECT) and positron emission tomography (PET). Alternatively, imaging techniques that detect fluorescence may be used in the methods of the invention.

Exemplary compounds useful in the methods of the invention include 2'-fluoro-2'deoxy-1-beta-D-arabinofuranosyl-5-iodo-uracil ([¹²⁵I]-FIAU), 2'-fluoro-2' deoxy-1-beta-D-arabinofuranosyl-5-iodo-uracil ([¹²⁴I]-FIAU), 9-(4-¹⁸F-fluoro-3-[hydroxymethyl]butyl)guanine ([¹⁸F]-FHBG), (18)F-1-(2'-deoxy-2'-fluoro-beta-d-arabinofuranosyl)thymine([¹⁸F]-FMAU), ¹⁸F-2'- fluoro-2'deoxy-1beta-D-arabinofuranosyl-5-ethyl-uracil ([¹⁸F]-FEAU) and 1-(2'-deoxy-2'-fluoro-beta-D-arabinofuranosyl)-5-[¹⁸F] iodouracil ([¹⁸F]-FIAU).

Exemplary fluorescent compounds that may be used in the methods of the invention have recently been described by Golankiewicz et al. ((2001) J. Med. Chem. 44:4284-7) and Goslinski et al. ((2002) J. Med. Chem. 45:5052-7). The fluorescent tricyclic acyclovir and ganciclovir analogs described by Goslinski *et al*., particularly GCV3, are contemplated for use in the claimed methods as thymidine kinase binding compounds. The phrase "thymidine kinase binding compound" is understood as a compound that has a sufficient affinity for thymidine kinase such that they are able to be used as imaging agents and/or therapeutic agents. In an embodiment, a thymidine kinase binding compound can be a viral thymidine kinase binding compound. Viral thymidine kinase binding compounds have at least a 10-fold, preferably 100-fold, preferably 1000-fold higher affinity for viral thymidine kinase as compared to mammalian thymidine kinase. Viral thymidine kinase binding compounds include, for example, FIAU, FHBG, FMAU, FEAU, tricyclic acyclovir and ganciclovir analogs such as GCV3. Thymidine kinase binding compounds can be modified to include functional groups to facilitate their use as imaging and/or as therapeutic agents.

In specific embodiments, the invention provides nucleoside analogs, such as 1-(2'-deoxy-2 '-fluoro- β-D-arabinofuranosyl) -5-iodouracil (FIAU), which are described, for example, in U. S. Patent No. 4,211,773, as an antiviral and an antitumor agent. Whether the substrate is for imaging or for therapy merely depends on the radionuclide used, e.g., iodine-¹²³, ¹²⁴ or ¹²⁵ for imaging vs. iodine-¹³¹ or astatine-²¹¹ for therapy. The nucleoside analogs are labeled with a radioisotope, e.g., a radioisotope of iodine, fluorine, yttrium, bismuth, or astatine. In another embodiment, the nucleoside analogs may be fluorescent. Preferred radiolabeled compounds of the invention are nucleoside analogs that are easily synthesized and limited *in vivo* catabolism. Compounds such as those described in USPNs:5,879,661 and 6,331,287 can be used with the methods of the invention.

Among the most commonly used positron-emitting nuclides in PET are ¹¹C, ¹³N, ¹⁵O, and ¹⁸F. Isotopes that decay by electron capture and/or γ emission are used in SPECT, and include, for example, ¹²³I and ¹²⁴I_{.}

The methods of the invention include PET. Specifically, imaging is carried out by scanning the entire patient, or a particular region of the patient using the detection system, and detecting the signal, *e.g*., the radioisotope signal. The detected signal is then converted into an image. The resultant images should be read by an experienced observer, such as, for example, a physician. The foregoing process is referred to herein as "imaging" the patient. Generally, imaging is carried out about 1 minute to about 48 hours following administration of the compound used in the methods of the invention. The precise timing of the imaging will be dependant upon such factors as the clearance rate of the compound administered, as will be readily apparent to those skilled in the art.

Once an image has been obtained, one of skill in the art will be able to determine the location of the compound. Using this information, the artisan can determine, for example, if a tumor is present, if the virus has entered the lytic phase, the extent of the tumor, or the efficacy of treatment which the subject is undergoing. Images obtained at different time points, e.g., 12, 24, 36, 48 or more, hours apart are particularly useful in determining the efficacy of treatment, e.g., lytic therapy and/or chemotherapeutic treatment.

Unlike methods currently used, the imaging methods described herein allow the clinician to distinguish between tumor with latent and lytic viral infection.

### Screening Assays

The invention provides methods for identifying agents useful for viral lytic induction, for neoplastic imaging, or useful as agents for the treatment of a neoplasia (e.g., as radiopharmaceuticals). While the Examples described herein specifically discuss the use of FIAU as a substrate for viral thymidine kinase, one skilled in the art understands that the methods of the invention are not so limited. Virtually any agent that acts as a substrate for a viral polypeptide and that can be modified to include a moiety that provides for its visualization in an imaging technique, or that provides for the induction of cell death, for example, by release of a lethal dose of radiation to a neoplastic cell (e.g., useful as a radiopharmaceutical), may be employed in the methods of the invention.

Methods of the invention are useful for the high-throughput low-cost screening of candidate agents useful as imaging agents, useful as viral induction agents, or as radiopharmaceuticals. Agents isolated and tested for activity in an *in vitro* assay or *in vivo* assay are useful in the methods of the invention. One skilled in the art appreciates that the effects of a candidate agent on a cell is typically compared to a corresponding control cell not contacted with the candidate agent. Thus, in one embodiment, the screening methods include comparing lytic induction in a cell having a latent viral infection contacted by a candidate agent to the induction observed in an untreated control cell.

In other embodiments, the expression or activity of a viral polypeptide in a cell treated with a candidate agent is compared to untreated control samples to identify a candidate compound that increases the expression or activity of a viral polypeptide or that increases viral replication in the contacted cell. Polypeptide expression or activity can be compared by procedures well known in the art, such as Western blotting, flow cytometry, immunocytochemistry, binding to magnetic and/or CD137-specific antibody-coated beads, *in situ* hybridization, fluorescence *in situ* hybridization (FISH), ELISA, microarray analysis, RT-PCR, Northern blotting, or colorimetric assays, such as the Bradford Assay and Lowry Assay.

In one working example, one or more candidate agents are added at varying concentrations to the culture medium containing cell having a latent viral induction. An agent that promotes the expression of a viral polypeptide or a reporter gene under the control of a viral promoter expressed in the cell is considered useful in the invention; such an agent may be used, for example, as a therapeutic to prevent, delay, ameliorate, stabilize, or treat neoplastic disease associated with a viral infection. Once identified, agents of the invention (e.g., agents that specifically bind to a viral polypeptide and/or stimulate viral lytic induction) may be used to image or treat a tumor in a subject.

Alternatively, or in addition, candidate compounds may be identified by first assaying those that specifically bind to and activate a viral polypeptide and subsequently testing their effect on viral lytic induction as described herein. In one embodiment, the efficacy of a candidate agent is dependent upon its ability to interact with or act as a substrate for the thymidine kinase polypeptide. Such an interaction can be readily assayed using any number of standard binding techniques and functional assays (e.g., those described in Ausubel et al., supra). For example, a candidate compound may be tested *in vitro* for interaction and binding with a polypeptide of the invention and its ability to modulate a latent viral infection.

In one particular example, a candidate compound that binds to a viral polypeptide may be identified using a chromatography-based technique. For example, a recombinant viral polypeptide may be purified by standard techniques from cells engineered to express the polypeptide, or may be chemically synthesized, once purified the peptide is immobilized on a column. A solution of candidate agents is then passed through the column, and an agent that specifically binds the viral polypeptide or a fragment thereof is identified on the basis of its ability to bind to viral polypeptide and to be immobilized on the column. To isolate the agent, the column is washed to remove non-specifically bound molecules, and the agent of interest is then released from the column and collected. Agents isolated by this method (or any other appropriate method) may, if desired, be further purified (e.g., by high performance liquid chromatography). In addition, these candidate agents may be tested for their ability to act as viral induction agents or as substrates for a viral polypeptide (e.g., as described herein). Agents isolated by this approach may also be used, for example, as therapeutics to treat or prevent the onset of a viral associated neoplasia. Compounds that are identified as binding to a viral polypeptide with an affinity constant less than or equal to 1 nM, 5 nM, 10 nM, 100 nM, 1 mM or 10 mM are considered particularly useful in the invention.

Such agents may be used, for example, as a therapeutics to combat the neoplasia. Optionally, agents identified in any of the above-described assays may be confirmed as effective in any standard animal model (e.g., rodent xenograft) and, if successful, may be used as anti-neoplastic therapeutics.

### Test Compounds and Extracts

In general, agents that act as viral lytic induction agents, that act as substrates for a viral polypeptide, or that specifically bind to a viral polypeptide are identified from large libraries of natural product or synthetic (or semi-synthetic) extracts or chemical libraries or from polypeptide or nucleic acid libraries, according to methods known in the art. Those skilled in the field of drug discovery and development will understand that the precise source of test extracts or compounds is not critical to the screening procedure(s) of the invention. Agents used in screens may include known those known as therapeutics for the treatment of pathogen infections. Alternatively, virtually any number of unknown chemical extracts or compounds can be screened using the methods described herein. Examples of such extracts or compounds include, but are not limited to, plant-, fungal-, prokaryotic- or animal-based extracts, fermentation broths, and synthetic compounds, as well as the modification of existing polypeptides.

Libraries of natural polypeptides in the form of bacterial, fungal, plant, and animal extracts are commercially available from a number of sources, including Biotics (Sussex, UK), Xenova (Slough, UK), Harbor Branch Oceangraphics Institute (Ft. Pierce, Fla.), and PharmaMar, U.S.A. (Cambridge, Mass.). Such polypeptides can be modified to include a protein transduction domain using methods known in the art and described herein. In addition, natural and synthetically produced libraries are produced, if desired, according to methods known in the art, e.g., by standard extraction and fractionation methods. Examples of methods for the synthesis of molecular libraries can be found in the art, for example in: DeWitt et al., Proc. Natl. Acad. Sci. U.S.A. 90:6909, 1993; Erb et al., Proc. Natl. Acad. Sci. USA 91:11422, 1994; Zuckermann et al., J. Med. Chem. 37:2678, 1994; Cho et al., Science 261:1303, 1993; Carrell et al., Angew. Chem. Int. Ed. Engl. 33:2059, 1994; Carell et al., Angew. Chem. Int. Ed. Engl. 33:2061, 1994; and Gallop et al., J. Med. Chem. 37:1233, 1994. Furthermore, if desired, any library or compound is readily modified using standard chemical, physical, or biochemical methods.

Numerous methods are also available for generating random or directed synthesis (e.g., semi-synthesis or total synthesis) of any number of polypeptides, chemical compounds, including, but not limited to, saccharide-, lipid-, peptide-, and nucleic acid-based compounds. Synthetic compound libraries are commercially available from Brandon Associates (Merrimack, N.H.) and Aldrich Chemical (Milwaukee, Wis.). Alternatively, chemical compounds to be used as candidate compounds can be synthesized from readily available starting materials using standard synthetic techniques and methodologies known to those of ordinary skill in the art. Synthetic chemistry transformations and protecting group methodologies (protection and deprotection) useful in synthesizing the compounds identified by the methods described herein are known in the art and include, for example, those such as described in R Larock, Comprehensive Organic Transformations, VCH Publishers (1989); T. W. Greene and P. G. M. Wuts, Protective Groups in Organic Synthesis, 2nd ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

Libraries of compounds may be presented in solution (e.g., Houghten, Biotechniques 13:412-421, 1992), or on beads (Lam, Nature 354:82-84, 1991), chips (Fodor, Nature 364:555-556, 1993), bacteria (Ladner, U.S. Patent No. 5,223,409), spores (Ladner U.S. Patent No. 5,223,409), plasmids (Cull et al., Proc Natl Acad Sci USA 89:1865-1869, 1992) or on phage (Scott and Smith, Science 249:386-390, 1990; Devlin, Science 249:404-406, 1990; Cwirla et al. Proc. Natl. Acad. Sci. 87:6378-6382, 1990; Felici, J. Mol. Biol. 222:301-310, 1991; Ladner *supra*.)*.*

In addition, those skilled in the art of drug discovery and development readily understand that methods for dereplication (e.g., taxonomic dereplication, biological dereplication, and chemical dereplication, or any combination thereof) or the elimination of replicates or repeats of materials already known for their activity should be employed whenever possible.

When a crude extract is found to have viral polypeptide binding and/or viral lytic induction activity further fractionation of the positive lead extract is necessary to isolate molecular constituents responsible for the observed effect. Thus, the goal of the extraction, fractionation, and purification process is the careful characterization and identification of a chemical entity within the crude extract that is useful as an imaging agent, as a viral lytic induction agent, or as a neoplasia therapeutic. Methods of fractionation and purification of such heterogenous extracts are known in the art. If desired, compounds shown to be useful as therapeutics are chemically modified according to methods known in the art.

### Therapeutic Methods

Agents identified as acting as viral lytic induction agents, acting as substrates for a viral polypeptide, as binding a viral polypeptide, and/or as acting as radiopharmaceuticals are useful for preventing or ameliorating a neoplastic disease associated with a viral infection (e.g., a latent viral infection) Cancer associated with viral infection include, but are not limited endemic Burkitt's lymphoma, post-transplant lymphoma, AIDS lymphoma, Hodgkin's lymphoma, nasopharyngeal carcinoma and gastric carcinoma associated with Epstein Bar virus, and Kaposi's sarcoma tumors associated with herpes virus. Neoplasia associated with a virus may be treated, diagnosed, imaged, or monitored using the methods and compositions of the invention.

In one therapeutic approach, an agent identified as described herein is administered to the site of a potential or actual disease-affected tissue or is administered systemically. The dosage of the administered agent depends on a number of factors, including the size and health of the individual patient. For any particular subject, the specific dosage regimes should be adjusted over time according to the individual need and the professional judgment of the person administering or supervising the administration of the compositions.

### Pharmaceutical Therapeutics

The invention provides a simple means for identifying compositions (including nucleic acids, peptides, small molecule inhibitors, and antibodies) capable of binding to or acting as a substrate for a viral polypeptide, of inducing viral lytic phase, or acting as therapeutics for the treatment or prevention of a neoplasia. Accordingly, a chemical entity discovered to have medicinal value using the methods described herein is useful as a drug or as information for structural modification of existing compounds, e.g., by rational drug design. Such methods are useful for screening agents having an effect on a variety of neoplasias associated with viral infections.

For therapeutic uses, the compositions or agents identified using the methods disclosed herein may be administered systemically, for example, formulated in a pharmaceutically-acceptable buffer such as physiological saline. Preferable routes of administration include, for example, subcutaneous, intravenous, interperitoneally, intramuscular, or intradermal injections that provide continuous, sustained levels of the drug in the patient. Treatment of human patients or other animals will be carried out using a therapeutically effective amount of a therapeutic identified herein in a physiologically-acceptable carrier. Suitable carriers and their formulation are described, for example, in Remington's Pharmaceutical Sciences by E. W. Martin. The amount of the therapeutic agent to be administered varies depending upon the manner of administration, the age and body weight of the patient, and with the clinical symptoms of the pathogen infection or neoplasia. Generally, amounts will be in the range of those used for other agents used in the treatment of other diseases associated with pathogen infection or neoplasia, although in certain instances lower amounts will be needed because of the increased specificity of the compound. A compound is administered at a dosage that effectively induces viral lysis (e.g., induces lysis in at least about 3-5, 5-10, 10-15, 15-20, 20-30, 30-50, 50-75, or 75-100% of infected tumor cells) as determined by a method known to one skilled in the art, or using any that assay that measures the expression or the biological activity of a viral promoter, viral polypeptide or viral replication.

The administration of a compound for the treatment of a neoplasia may be by any suitable means that results in a concentration of the therapeutic that, combined with other components, is effective in ameliorating, reducing, or stabilizing a neoplasia. The compound may be contained in any appropriate amount in any suitable carrier substance, and is generally present in an amount of 1-95% by weight of the total weight of the composition. The composition may be provided in a dosage form that is suitable for parenteral (e.g., subcutaneously, intravenously, intramuscularly, or intraperitoneally) administration route. The pharmaceutical compositions may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

### Bacterial infections

Viruses are not the only pathogens that express thymidine kinases. Many bacterial pathogens also express such kinases. Accordingly, bacterial infections are also susceptible to treatment with FIAU, including radiolabeled FIAU analogs. Gram positive bacteria include, but are not limited to, *Pasteurella* species, *Staphylococci* species, and *Streptococcus* species. Gram negative bacteria include, but are not limited to, *Escherichia coli*, *Pseudomonas* species, and *Salmonella* species. Specific examples of infectious bacteria include but are not limited to, *Helicobacter* pyloris, *Borelia burgdorferi*, *Legionella pneumophilia*, *Mycobacteria* sps (e.g. *M*. *tuberculosis*, *M avium*, *M. intracellulare*, *M. kansaii*, *M. gordonae*), *Staphylococcus aureus, Neisseria gonorrhoeae*, *Neisseria meningitidis, Listeria monocylogenes*, *Streptococcus pyogenes* (Group A Streptococcus), *Streptococcus agalactiae* (Group B Streptococcus), *Streptococcus* (viridans group*), Streptococcus faecalis*, *Streptococcus bovis, Streptococcus* (anaerobic sps.), *Streptococcus pneumoniae*, pathogenic *Campylobacter sp*., *Enterococcus sp*., *Haemophilus influenzae, Bacillus antracis, corynebacterium diphtheriae*, *corynebacterium sp*., *Erysipelothrix rhusiopathiaei*, *Clostridium perfringers*, *Clostridium tetani*, *Enterobacter aerogenes*, *Klebsiella pneumoniae*, *Pasturella multocida*, *Bacteroides sp*., *Fusobacterium nucleatum*, *Streptobacillus moniliformis*, *Treponema pallidium*, *Treponema pertenue*, *Leplospira*, *Rickettsia*, and *Actinomyces israelli.* **Other bacterial pathogens include** *Aerobacter*, *Aeromonas*, *Acinetobacter*, *Actinomyces israelli*, *Agrobacterium*, *Bacillus*, *Bacillus antracis*, *Bacteroides*, *Bartonella*, *Bordetella, Bortella*, *Borrelia*, *Brucella*, *Burkholderia*, *Calymmatobacterium*, *Campylobacter*, *Citrobacter*, *Clostridium*, *Clostridium perfringers*, *Clostridium tetani*, *Cornyebacterium*, *corynebacterium diphtheriae*, *corynebacterium sp*., *Enterobacter*, *Enterobacter aerogenes*, *Enterococcus*, *Erysipelothrix rhusiopathiae*, *Escherichia*, *Francisella*, *Fusobacterium nucleatum*, *Gardnerella*, *Haemophilus*, *Hafnia*, *Helicobacter*, *Klebsiella*, *Klebsiella pneumoniae*, *Lactobacillus*, *Legionella*, *Leptospira*, *Listeriai, Morganella*, *Moraxella, Mycobacterium*, *Neisseria*, *Pasteurella*, *Pasturella multocida*, *Proteus*, *Providencia, Pseudomonas*, *Rickettsia*, *Salmonella*, *Serratia*, *Shigella*, *Staphylococcus*, *Stentorophomonas*, *Streptococcus*, *Streptobacillus moniliformis*, *Treponema*, *Treponema pallidium*, *Treponema pertenue*, *Xanthomonas*, *Vibrio*, and *Yersinia.*

### Parenteral Compositions

The pharmaceutical composition may be administered parenterally by injection, infusion or implantation (subcutaneous, intravenous, intramuscular, intraperitoneal, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions for parenteral use may be provided in unit dosage forms (e.g., in single-dose ampoules), or in vials containing several doses and in which a suitable preservative may be added (see below). The composition may be in the form of a solution, a suspension, an emulsion, an infusion device, or a delivery device for implantation, or it may be presented as a dry powder to be reconstituted with water or another suitable vehicle before use. Apart from the active agent that reduces or ameliorates a pathogen infection or neoplasia, the composition may include suitable parenterally acceptable carriers and/or excipients. The active therapeutic agent(s) may be incorporated into microspheres, microcapsules, nanoparticles, liposomes, or the like for controlled release. Furthermore, the composition may include suspending, solubilizing, stabilizing, pH-adjusting agents, tonicity adjusting agents, and/or dispersing, agents.

As indicated above, the pharmaceutical compositions according to the invention may be in the form suitable for sterile injection. To prepare such a composition, the suitable active active inflammatory bowel disorder disorder therapeutic(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution and dextrose solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol or the like.

### Controlled Release Parenteral Compositions

Controlled release parenteral compositions may be in form of aqueous suspensions, microspheres, microcapsules, magnetic microspheres, oil solutions, oil suspensions, or emulsions. Alternatively, the active drug may be incorporated in biocompatible carriers, liposomes, nanoparticles, implants, or infusion devices.

Materials for use in the preparation of microspheres and/or microcapsules are, e.g., biodegradable/bioerodible polymers such as polygalactin, poly-(isobutyl cyanoacrylate), poly(2-hydroxyethyl-L-glutam- nine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters) or combinations thereof).

### Solid Dosage Forms For Oral Use

Formulations for oral use include tablets containing the active ingredient(s) in a mixture with non-toxic pharmaceutically acceptable excipients. Such formulations are known to the skilled artisan. Excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug in a predetermined pattern (e.g., in order to achieve a controlled release formulation) or it may be adapted not to release the active drug until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material, such as, e.g., glyceryl monostearate or glyceryl distearate may be employed.

The solid tablet compositions may include a coating adapted to protect the composition from unwanted chemical changes, (e.g., chemical degradation prior to the release of the active a anti-pathogen or anti-neoplasia therapeutic substance). The coating may be applied on the solid dosage form in a similar manner as that described in Encyclopedia of Pharmaceutical Technology, supra.

At least two anti-pathogen or anti-neoplasia therapeutics may be mixed together in the tablet, or may be partitioned. In one example, the first active anti-pathogen or anti-neoplasia therapeutic is contained on the inside of the tablet, and the second active anti-pathogen or anti-neoplasia therapeutic is on the outside, such that a substantial portion of the second active anti-pathogen or anti-neoplasia therapeutic is released prior to the release of the first active anti-pathogen or anti-neoplasia therapeutic.

Formulations for oral use may also be presented as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders and granulates may be prepared using the ingredients mentioned above under tablets and capsules in a conventional manner using, e.g., a mixer, a fluid bed apparatus or a spray drying equipment.

### Controlled Release Oral Dosage Forms

Controlled release compositions for oral use may, e.g., be constructed to release the anti-neoplasia therapeutic by controlling the dissolution and/or the diffusion of the active substance. Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more therapeutic compounds may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the compound(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Combination Therapies

Optionally, anti-neoplasia therapeutic may be administered in combination with any other standard anti-neoplasia therapy; such methods are known to the skilled artisan and described in Remington's Pharmaceutical Sciences by E. W. Martin.

### Kits

The invention provides kits for the diagnostic imaging, treatment, prevention, or monitoring of virus associated neoplasias. In one embodiment, the kit includes a composition containing an effective amount of an agent (e.g., in unit dosage form) for the treatment of a neoplasia. In another embodiment, the kit includes a composition comprising a viral lytic induction agent. In still another embodiment, the kit includes an agent for imaging a neoplasia. In some embodiments, the kit comprises a sterile container which contains a therapeutic or prophylactic cellular composition; such containers can be boxes, ampoules, bottles, vials, tubes, bags, pouches, blister-packs, or other suitable container forms known in the art. Such containers can be made of plastic, glass, laminated paper, metal foil, or other materials suitable for holding medicaments.

If desired an agent of the invention is provided together with instructions for administering the agent to a subject having or at risk of developing a neoplasia. The instructions will generally include information about the use of the composition for imaging a neoplasia, or for the treatment or prevention of neoplasia. In other embodiments, the instructions include at least one of the following: description of the therapeutic or imaging agent; dosage schedule and administration for treatment or prevention of a neoplasia or symptoms thereof; precautions; warnings; indications; counter-indications; overdosage information; adverse reactions; animal pharmacology; clinical studies; and/or references. The instructions may be printed directly on the container (when present), or as a label applied to the container, or as a separate sheet, pamphlet, card, or folder supplied in or with the container.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the invention, and, as such, may be considered in making and practicing the invention. Particularly useful techniques for particular embodiments will be discussed in the sections that follow.

The following examples are put forth so as to provide those of ordinary skill in the art with a complete disclosure and description of how to make and use the assay, screening, and therapeutic methods of the invention, and are not intended to limit the scope of what the inventors regard as their invention.

This invention is further illustrated by the following examples, which should not be construed as limiting.

### EXAMPLES

### Example 1: Luciferase assay identified reagents that activated the Zta promoter

Zta is the key transactivator in the induction of EBV lytic infection. All reported lytic induction agents activate transcription of Zta. To identify drugs that induce immediate early gene expression in EBV, a Zta promoter luciferase reporter in a gastric carcinoma cell line background was assessed for its ability to detect known inducers of EBV lytic gene expression. In this promoter-epithelial cell assay, treatment with phorbol 12-tetradecanoate 13-acetate (TPA) and butyrate (TPA/butyrate) and valproic acid led to increased expression of luciferase in a dose-dependent manner as shown in Figure 1, while 5'-azadeoxycytidine did not activate this promoter (Figure 1).

The promoter-reporter system may not reflect regulation of Zta promoter in the context of the viral episome. The ZTA promoter- epithelial assay focuses upon a single well defined aspect of EBV replication with virus-free systems. EBV lytic infection is regulated on many levels including epigenetic modification and latent protein suppression of lytic promoter. The ZTA luciferase assay did not identify drugs such as 5'-azadeoxycytidine. So a complementary virus-cell based assay for high-throughput screening was developed.

### Example 2: Development of a GFP-virus-based assay to identify EBV lytic induction reagents

BX-1 is a recombinant EBV encoding GFP with the BXLF1 ORF disrupted, and AKATA-BX1 cells are Akata negative cells with this recombinant EBV. Microscopy showed that when EBV lytic infection was induced with Anti-IgG in AKATA-BX1 cells, the GFP signal would increase with lytic infection (Figure 2A). Since GFP was encoded by an EBV recombinant virus, viral replication would produce more GFP and yield stronger fluorescence signal. This GFP signal was detected by a microplate reader. An increase in relative fluorescence units (RFU) was detected after lytic induction (Figure 2A). The GFP signal was specifically inhibited by the S phase CDK inhibitor Purvalanol A, which is reported to inhibit EBV lytic induction [7] (Figure 2B). To determine whether the GFP signal induction was due to CMV promoter activation or reflected lytic replication, a stable Hela cell line with CMV promoter driven GFP plasmid was used as control. None of the lytic induction reagents studied showed increased GFP signal in this control cell line (Figure 2C).

In contrast with the luciferase assay, the GFP signal in Akata-BX1 cells was induced by 5'-azadeoxycytidine in a dose dependent manner (Figure 3). In a 96-well format, the GFP assay was easily adapted to high throughput assay.

### Example 3. Drug screen of a clinical compound based library

The Johns Hopkins Clinical Compound Library (JHCCL) consists of 2720 compounds, which were screened at a final concentration of 10uM. The compounds were dissolved in DMSO or PBS, and formatted into 96-well plates. A typical readout of the GFP-whole virus assay is shown in Figure 4. More than 200 compounds were identified as potential lytic induction drugs in one or both assays. Most agents identified as having activity could be grouped into 5 families: Proteasome inhibitors, anti-tubulin drugs, glucocorticoid and steroid hormones, nucleoside analogs, and anti-inflammatory drugs. Although many hits were in common, glucocorticoid activity was most frequently identified in the lymphoma cell line/whole virus assay, and antitubulin drugs were mostly often found in the epithelial-promoter reporter assay. Interestingly, many suppression hits are not drugs traditionally known as cytotoxic drugs. Among those hits, there are simvastatin and lovastatin, the two drugs that recently were reported to induce apoptosis of EBV transformed lymphoblastoid cell lines (Katano et al., Proc Natl Acad Sci U S A, 2004. 101(14):4960-5. Further study of these reagents may reveal some tumor specific therapies for EBV associated tumors.

The reidentification of drugs that had been previously shown to induce EBV lytic cycle represented a critical validation of the approach used in the two assays. Drugs verified by the epithelial promoter reporter assay only were Methotrexate, cis-platinum, 5-FU, and Taxol. Drugs verified by lymphoma cell line/whole virus assay only were most glucocorticoid drugs and anti-IgG. Drugs verified by both were Doxorubicin, PMA, and some glucocorticoid drugs. Drugs missed by both assays were Sodium butyrate, Arginine butyrate, and Cobalt chloride (Figure 5).

The hits were then further studied with the same assay carried out at 5 µM, 1 µM , 100 nM and 10 nM to determine EC₅₀ for inducing lytic infection. Promising hits were then tested in EBV positive cell lines. For these further investigations, compounds were obtained independently from the library. These were selected in part based on the availability of the agents for further analysis and in part to represent the diversity of compounds identified in the initial screen. Compounds active at low micromolar concentrations and compounds that are not traditional cytotoxic drugs were of lesser interest.

### Example 4: Bortezomib, mebendazole, cytarabine were identified as lytic induction reagents

Promoter-epithelial assay revealed many drugs that can induce the BZLF promoter. Tranilast is an anti-allergic drug that suppresses mast cell degranulation, histamine release and fibrosis process (Figure 6). The anti-inflammatory effect is through inhibition ofNF-KB function. Leflunomide inhibits dihydroorotate dehydrogenase, the fourth enzyme in the pyrimidine biosynthetic pathway, and antagonizes growth-factor mediated smooth muscle cell proliferation *in vitro.* It's used to treat rheumatoid arthritis.

Tranilast and Leflunomide induced the BZLF promoter up to 15 fold at therapeutic serum concentration (Figure 6). It's reported that immediate early protein BZLF alone is sufficient to trigger the entire lytic cascade. However, drugs capable of inducing BZLF promoter in luciferase assay do not necessarily induce BZLF protein expression. The above reagents did not induce BZLF protein expression in EBV positive cell lines such as SNU719, AKATA, Raji, LCL and Rael cells.

Other hits in the luciferase assay induced BZLF1 protein, but not viral replication. Drugs in this category included Cytarabine (Ara-C) (Figure 7A) and Indoprofen (Figure 9A). Ara-c induced the BZLFI promoter in a luciferase assay.(Figure 7B), as well as inducing ZTA expression in LCL cells (Figure 7C).

Cytarabine and Indoprofen triggered the EBV immediate early gene expression, but stopped the lytic cascade at later stage. Cytarabine induced immediate early protein ZTA in SNU719, LCL, AKATA-BX1 cells (Figure 8A and 8B), and also induced lytic gene RPA and TK expression(Figure 8C), but did not induce viral replication (Figure 8D). Indoprofen induced ZTA in SNU719, but not viral replication(Figure 9A-9D).

Bortezomib was the most prominent hit in both assays (Figure 10A). Bortezomib EC₅₀ was at nanomolar scale in the assays, which is consistant with the therapeutic serum concentration. When tested in EBV(+) B cells, Bortezomib did not only induce lytic genes such as ZTA (Figure 10B), RTA, and EBV-TK, but also EBV replication(Figure 10C). Mebendazole can also induce BZLF1 protein expression and viral replication in EBV+ cell lines (Figure 11).

Collectively, by applying two assays in a clinical compound library, the screening identified many FDA approved agents with potent EBV lytic induction activity.

It has been reported that the regulation of Zta promoter in a stably transfected plasmid with oriP is similar to endogenous Zta promoter regulation in EBV (Jenkins et al., J Virol, 2000. 74(2): p. 710-20). In the oriP plasmid while chromatin structure is maintained in terms of acetylation, the Zp promoter region is not methylated. Although the present report employed a "regular" pGL2 plasmid for drug screening, a similar chromatin structure was observed. The Zp promoter was induced by butyrate, an HDAC inhibitor, but was not induced by the methylation inhibitor 5-azadeoxycytidine.

The GFP signal observed corresponds with viral replication and is a reliable reporter for EBV lytic induction. Its continuous production during replication and easy quantification are important strengths. GFP has been used as a reporter in drug screening assays for many pathogens, such as *Mycobacterium tuberculosis* (Collins et al., Antimicrob Agents Chemother, 1998. 42(2): p. 3447), *HIV* (Daelemans et al., Mol Pharmacol, 2005. 67(5): p. 1574-80), *CMV* and *HCV* Lee et al., J Virol Methods, 2004. 116(1): p. 27-33). The present invention established a novel assay for real-time monitoring of intracellular EBV replication, and adapted this assay for high-throughput screening.

The different hits found in the GFP and luciferase assays reflect different features of the two assays. Also, as with any other high throughput screen, the sensitivity and specificity of the assay is limited by the drug concentration used in the screen. Arginine butyrate and sodium butyrate were missed as hits in both screens because the concentration tested (10 µM) was much lower than the effective concentration (0.5-2 mM) reported.

As demonstrated herein, the present invention provides agents that induce lytic infection of EBV. EBV lytic infection is also considered to play a role in Posttransplantation lymphoproliferative disorders (PTLD). Understanding various reagents capable of reactivating EBV replication may be useful in treating these disorders.

Tranilast and leflunomide activated the lytic promoter at therapeutic concentrations. Although they don't induce lytic protein expression or viral replication in B cells or gastric epithelial cells (SNU 719), Indoprofen shows promise in inducing Zta protein in SNU719 cells at a high dose. Most of our hits are cytotoxic drugs, which may be consistent with the notion that the virus reactivates in response to stress. One distinct group of drugs are microtubule disturbance drugs.

Cytarabine represents a special class of drugs that can induce immediate early and early gene expression, but not full lytic replication. Its action may be due to inhibition of the viral DNA polymerase. This class of drugs is of interest in that it can accomplish major steps required for host cell apoptosis without causing viremia. Not only were immediate early gene BZLF I and BRLF 1 induced, the lytic cascade can be carried to TK, which allows ganciclovir and viral imaging to work. Finding new uses for existing drugs is a promising approach. The screening of existing compounds is likely to identify novel activities/indications of some known drugs. One of the obvious advantages of this library is that the follow-up clinical trials of the screen by-pass the time- and cost-consuming processes required to establish toxicity and pharmacokinetics.

### Example 5: Bortezomib induced EBV-TK.

Bortezomib treatment induced EBV-TK and EBV-ZTA expression as assessed by immunoblot (Figures 12A and 12B), ZTA/luciferase activity (Figure 12C) and TK functional activity as reflected in the accumulation of [¹⁴C]FIAU (Figure 12D). No antigen or functional activity was detected in the absence of bortezomib or in EBV(-) cell lines. The effects of bortezomib were dose dependent as shown in the BX-1 cell line expressing green fluorescent protein (GFP) (Figures 13A and 13B). Viral copy number, as measured by real time DNA PCR, also increased with treatment (Figure 13C). One of the effects ofbortezomib that is well recognized is stabilization of IκB and resultant inhibition ofNF-KB. In an effort to determine whether this pathway might also be important in induction of lytic gene expression the effects of bortezomib were investigated in an IκB super repressor (IκB (sr)) in transfection experiments in a Burkitt's cell line. As shown in Figure 13D, EBV viral load increased by approximately 12-fold following transfection. Moreover, when EBV(+) Akata cells were incubated in the presence of 20 nM bortezomib for 48 hours, 67% of cells entered the lytic cycle, and the induction proved to be dose-dependent.

Mice bearing EBV(+) Burkitt's lymphoma xenografts were treated with either bortezomib or phosphate buffered saline (PBS) and imaged at various times after injection of [¹²⁵I]FIAU (Figure 14). Bortezomib was always administered 24 hours before C¹²⁵I]FIAU, also intravenously. Images obtained at early time points reveal only cardiac blood pool, liver, urinary bladder and thyroid, consistent with the established biodistribution of [¹²⁵I]FIAU. By 1 day postinjection of radiopharmaceutical, the tumor in the animal treated with bortezomib could be visualized and by 4 days postinjection, the tumor in the treated animal was clearly visible, despite abundant radioactivity within the bladder. 2 µg/g of bortezomib stimulated the maximum target to non-target signal ratio at 96 hours after treatment in this cell line. The radioactivity depicted by the arrow in Figure 14B at the 96 hour time point was within the tumor as shown by concurrent ex vivo gamma counting. Similar results were obtained in animals bearing another EBV(+) Burkitt cell line (Rael) xenograft. Following treatment with bortezomib, but not PBS, the tumor was clearly visualized by 30 hours after radiopharmaceutical injection in that case. Figure 15 shows SPECT-CT images of a xenograft derived from the EBV(+) Akata cell line at 72 and 96 hours after radiopharmaceutical administration. The percentage of injected dose per gram (%ID/g) derived from the images was 1.13 ± 0.01 and 0.70 ± 0.01 at 72 and 96 hours, respectively. Only one mouse was imaged with SPECT-CT, so the standard deviation (SD) values were derived from the multiple slices of each image.

### Example 6: Ex vivo biodistribution.

In order to confirm that the radioactivity ostensibly demonstrated within tumor on the images in Figures 14 and 15 was indeed within tumor, and to quantify the amount of uptake, *ex vivo* biodistribution assays were performed in animals bearing EBV(+) xenografts in additional experiments. Although increased [¹²⁵I)FIAU uptake was evident earlier, it peaked at 96 hours postinjection in tumors of animals pretreated with bortezomib. Figure 14A showed a lack of tumor uptake at 96 hours (0.039 %ID/g) in mice pretreated with PBS, while Figure 14B showed the time course of uptake in the tumors of bortezomib pretreated animals. Tumor radiopharmaceutical uptake increased from 0.039 to 0.85% ID/g, a nearly 22-fold increase over baseline values, after treatment with bortezomib (Table 1).

| **Table 1.** Tissue distribution of [¹²⁵I]FIAU in severe combined immunodeficient mice bearing human EBV(+) tumors (Akata) | | |
|---|---|---|
| **Tissue** | **PBS treatment** | **Bortezomib treatment** |
| | **%ID/g ± SD (n = 4; 96 h)** | |
| Tumor | 0.039 ± 0.010 | 0.850 ± 0.102 |
| Liver | 0.029 ± 0.011 | 0.016 ± 0.002 |
| Spleen | 0.014 ± 0.006 | 0.060 ± 0.011 |
| Kidney | 0.068 ± 0.014 | 0.070 ± 0.010 |
| Muscle | 0.030 ± 0.010 | 0.040 ± 0.005 |
| Blood | 0.033 ± 0.009 | 0.022 ± 0.010 |

The radioactivity detected within other tissues, i.e., liver, spleen, kidney and muscle, diminished between the 24 and 96 hour time points.

In order to confirm that the ability to image tumors following bortezomib treatment did not reflect induction of a human cellular kinase leading to FIAU accumulation, EBV(-) osteosarcoma xenografts were also imaged. No tumor uptake was evident, with or without bortezomib. However, the same cell line engineered to express constitutively the EBV-TK was readily imaged (Figure 16). These results indicated that the effects of pharmacologic induction that are relevant to imaging are mediated by the viral TK rather than activation of a cellular kinase.

The use of a reporter transgene to determine the expression of an endogenous gene of interest has become a mainstay of cell and molecular biology. Those techniques, previously reserved for work done *in vitro,* have been extended to imaging *in vivo.* The relatively new area of *in vivo* molecular-genetic imaging uses a variety of modalities, most of which parallel those used in the clinic, including magnetic resonance imaging and PET. By transfecting cells with suitable imaging reporters, primarily but not limited to HSV1-TK, and using a variety of radiolabeled substrates and ligands, investigators can measure diverse cellular processes including T-cell trafficking, immune activation, response to mechanism-specific anticancer agents, and assembly of protein interaction networks. Two reports use molecular-genetic imaging to identify cells productively transfected with HSV1-TK in preparation for antitumor gene therapy in patients. The present inventors have imaged endogenous TK expression to study combined bacteriolytic therapy in experimental models of colon cancer and bacterial infection in general (Bettegowda et al., Proc Natl Acad Sci U S A 2005;102:1145, which is hereby incorporated by reference). Herein molecular-genetic imaging was used to study expression of a gene, EBV-TK, as a surrogate marker for induction of the viral lyric cycle, which enabled avoidance of a gene transfection step.

EBV Burkitt's and Akata lymphoma xenografts were imaged with [¹²⁵I]FIAU using a dedicated gamma camera after pharmacologic induction of viral TK expression. Etiologic viral-associated tumors themselves provided the reporter gene to image the tumors directly. The method was highly sensitive, with as few as 5% of the cells within the tumor mass needing to be induced into the lytic cycle for detection by imaging in certain EBV(+) cell lines. Although the tumor models used were subcutaneous, the lack of significant attenuation of γ rays suggests that the method are applicable to tumors deep within the body.

As described above, bortezomib was identified in a screen of 2,700 Food and Drug Administration-approved drugs as the most active in induction of the EBV lytic cycle, providing the rationale for this study. Inhibition of the NF-κB pathway induced the EBV lytic cycle. Bortezomib is known to have anti-NFκB activity by inhibition of the degradation of IκB. The results reported herein uncover a link between anti-NFκB activity and lytic induction, providing a mechanism for the result observed in the EBV-associated lymphomas studied. Based on these findings it is likely that the viral association of tumors for imaging can be exploited for cancer therapy. Notably, several therapeutic strategies have been explored that involve modulation of viral gene expression in tumors (Jacobs et al., Lancet 2001;358:727 -9; Yaghoubi et al., J Nucl Med 2006; 47:706-15). Such therapies may be monitored noninvasively in preclinical models, as described in this report, or in patients by using radiolabeled nucleoside analogues already in clinical use (Jacobs et al., Lancet 2001;358:727 -9; Yaghoubi et al., J Nucl Med 2006; 47:706-15). Methods to optimize the treatment of certain virus-associated tumors are routine and are known to one of skill in the art. Bortezomib is one of an array of new and previously known agents that are effective for inducing lytic infection and a particular agent may be tailored for use, through imaging, in a specific patient. For example, if a tumor cannot be positively delineated on a [²⁴I]FIAU-PET scan soon after therapy is initiated, lytic induction therapy should be stopped in that patient in favor of another potentially more beneficial approach. Demonstration of a positive signal on an imaging study to indicate the salutary effect of therapy, linked mechanistically to the induction of specific genes of naturally associated viral genomes, without the need for viral or other transfection of the imaging reporter, may provide a readily translatable asset to anticancer therapy.

### Example 7: Engineered constitutive EBV TK expression

In order to evaluate the specificity that might be achieved with viral TK induction with regard to the concentration of 2'-fluoro-2'-deoxy-beta-D-5-iodouracil-arabinofuranoside (FIAU) in tumor tissue, a human osteosarcoma cell line previously engineered to express the EBV-TK was used (Moore, S.M., Cannon, J.S., Tanhehco, Y.C., Hamzeh, F.M. & Ambinder, R.F. Induction of Epstein-Barr virus kinases to sensitize tumor cells to nucleoside analogues. Antimicrob Agents Chemother 45, 2082-2091 (2001)). Tumor cells were engrafted subcutaneously on the flanks of SCID mice. After tumor was palpable, [¹²⁵I]FIAU was administered intravenously and mice were sacrificed at various time points for tissue distribution studies (Figure 17). Selective concentration of radioactivity in TK(+) tumor was apparent by 2 hours and radioactivity levels in the tumor remained constant until the last time point at 96 hours, whereas levels in nontarget tissues decreased. In a parallel experiment carried out to later time points, radioactivity in the tumor was stable from 2 hours to the last time point at 4 days. The tumor to muscle ratio climbed from 4.6 at 2 hours post- infusion to peak at 205 at 24 hours post-infusion and fall to 114 at 96 hours post- infusion.

To determine whether EBV-TK mediated concentration of radioisotope in tumor tissue was adequate to achieve a therapeutic effect, mice with tumors were treated with [¹³¹I]FIAU or buffered saline (Figure 18A-18C). Control and TK tumors in mice injected with buffered saline, and control tumors in mice injected with [¹³¹I]FIAU showed similar growth curves, and of note, the 95% confidence intervals of the slopes of those growth curves (as determined by linear regression) overlapped. The growth slope of TK tumors in mice injected with [¹³¹I]FIAU flattened (i.e. the confidence interval for the slope of TK with [¹³¹I]FIAU in particular includes a zero slope estimate and does not overlap with the confidence intervals of the other growth curves) indicating that the growth rate is dramatically slowed by this treatment. In separate experiments, increasing doses of [¹³¹I]FIAU were associated with increasing effects on tumor growth (Figure 18B). When admixtures of TK and control tumor cells were engrafted to generate chimeric tumors, increasing percentages of TK-expressing tumor cells in the admixture were also associated with increasing effects on tumor growth (Figure 18C).

### Example 8: Bortezomib-induced enzyme targeted radiotherapy for EBV-Burkitt's lymphoma

As described above, the proteasome inhibitor bortezomib was identified as a potent inducer of lytic EBV infection in Burkitt's lymphoma cell lines *in vitro* and in cell line murine xenograft models. In order to evaluate the specificity that might be achieved with pharmacologic induction in naturally infected tumor tissue, EBV (+) Burkitt's lymphoma cells were engrafted subcutaneously on the flanks of SCID mice. After the tumor was palpable, [¹²⁵I]FIAU was administered intravenously 24 hours after bortezomib administration and the mice were sacrificed for tissue distribution studies. Selective concentration of radioactivity as measured per gram of tissue was apparent in these tumors (Table 1).

**Table 2. Tissue distribution of [125I]FIAU (5µCi) in mice bearing human tumors post injection (p.i.).**

| Tumor | TK143b Osteosarcoma | Rael Burkitt's lymphoma | KT gastric cancer | BC3 lymphoma | BCBL1 lymphoma |
|---|---|---|---|---|---|
| Days p.i. | 4 | 4 | 4 | 5 | 4 |
| N | 3 | 4 | 3 | 3 | 5 |

| Virus | EBV TK | EBV | | KSHV | |
|---|---|---|---|---|---|
| TK expression | <-constitutive-> | <---------------------------------bortezomib induction -------------------------------> | | | |
| Tissue | | | %ID/g±SD | | |
| Tumor | 1.667±0.681 | 1.200±0.356 | 0.598±0.472 | 1.213±0.214 | 1.100±0.361 |
| Liver | 0.013±0.006 | 0.052±.027 | 0.050±0.017 | 0.009±0.002 | 0.0617±0.022 |
| Spleen | 0.027±0.015 | 0.064±.011 | 0.080±0.035 | 0.008±0.002 | 0.064±0.014 |
| Kidney | 0.033±0.058 | 0.146±.054 | 0.090±0.030 | 0.027±0.003 | 0.167±0.038 |
| Muscle | 0.015±0.014 | 0.084±.012 | 0.080±0.062 | 0.008±0.004 | 0.078±0.005 |

Therefore, an evaluation of a therapeutic isotope was carried out. As described above, SCID mice engrafted with EBV Burkitt's lymphoma xenografts received either buffer or bortezomib followed the next day by treatment with [¹³¹I]FIAU or no treatment. As can be seen in Figure 19A, tumor growth curves in mice injected with buffer followed by [¹³¹I]FIAU were similar to tumor growth curves in mice injected with buffer alone. Bortezomib without [¹³¹I]FIAU slowed tumor growth. Bortezomib followed by [¹³¹I]FIAU stopped tumor growth (the 95% confidence interval overlapped 0, but did not overlap the 95% confidence intervals of the slopes of the growth curves associated with buffer alone or buffer with [¹³¹I]FIAU). In experiments with a second EBV(+) Burkitt's lymphoma cell line (Akata), parallel results were achieved. Thus, bortezomib allowed targeting of [¹³¹I]FIAU with therapeutic effect. Other proteasome inhibitors are also likely to be active. Several are in clinical trial.

### Example 9: Bortezomib-induced enzyme targeted radiotherapy for EBV gastric carcinoma

Although EBV was discovered in African Burkitt's' lymphoma and is associated with many AIDS-related lymphomas, as well as Hodgkin's lymphoma, the numerically most important associations are with epithelial cancers. EBV is associated with virtually all nasopharyngeal cancers (∼80,000 new cases/year) and approximately 10% of gastric carcinoma (∼93,000 new cases/year). In order to determine whether the bortezomib-induction strategy might also be applicable to epithelial malignancies, bortezomib-induced FIAU targeting was studied in a transplantable human EBV-associated gastric cancer (KT) xenograft model (Chong, J.M., et al. Interleukin-Ibeta expression in human gastric carcinoma with Epstein-Barr virus infection. J Virol 76, 6825-6831 (2002)). Following engraftment with KT tumor, SCID mice were induced with bortezomib followed by [¹²⁵I]FIAU and tumors were imaged with single photon emission computed tomography (SPECT) (Figure 20A). Tissue distribution studies showed selective concentration of [¹²⁵I]FIAU in tumor tissue, although the ratios in tumor tissue to other tissues was not as dramatic as with the Burkitt's lines (Table 1). When induction was followed by [¹³¹I]FIAU, tumors growth rate was diminished (Figure 19B).

### Example 10: Bortezomib-induced enzyme targeted radiotherapy in KSHV malignancy

The approach observed in EBV-associated tumors is also applicable to other KSHV-associated tumors as shown in studies on two primary effusion lymphoma (PEL) cell lines that harbor KSHV. In a SCID xenograft model, bortezomib allowed imaging of both BCBL1 and BC3 with [¹²⁵I]FIAU (Figure 20B). Tissue distribution studies following bortezomib showed selective concentration in tumor tissue (Table 1). Finally, when induction with bortezomib was followed by treatment with [¹³¹I]FIAU tumors regressed (Figure 19C).

The HSV1-TK has been used as a tool in gene therapy approaches to cancer. Introduction into tumor cells by retroviral vectors, adenoviral vectors, and others have all been studied in clinical investigations as well as animal models. Similarly, induction of the EBV-TK with a histone deacetylase inhibitor followed by treatment with KSHV has been reported to induce remissions in some patients. The results reported herein provide a new approach: bortezomib-induced enzyme targeting of radionuclide (BETR) therapy. BETR therapy was shown to be more effective at inducing regression of lymphoid and epithelial malignancies in murine xenografts of human tumors.

Results with ¹³¹I-labeled antibodies against lymphoma in patients demonstrates that clinical responses are achievable with 0.5 to 2 Gy to the tumor. The biodistribution data presented in Figure 17 allows estimates of the absorbed dose per administered activity to tumor and vital organs, and suggests that the kidneys and red marrow are the dose limiting organs for [¹³¹I]FIAU. As detailed in the online supplement, delivery of 0.5 to 2 Gy to a 1 gram tumor in a 70 kg patient with critical organ doses well below toxicity.

With regard to the potential clinical use of BETR therapy, FIAU-associated toxicity warrants discussion. FIAU was previously investigated as an antiviral agent. Chronic dosing of FIAU was associated with hepatic damage and in some cases fatality, apparently the result of mitochondrial damage. In those trials, treatment for less than four weeks with a cumulative dose less than 200 mg was not associated with either clinical or biochemical evidence of toxicity. Thus, the "no-effect dose" of FIAU might be conservatively estimated to be on the order of 0.1 mg/kg per administration. [¹²⁴I]FIAU has been administered to patients for imaging without any adverse effect on hepatic function (Diaz et al., PLoS ONE 2, e1007 (2007)). Extrapolating from xenograft experiments, a radiotherapeutic effect is likely to be achieved at doses of FIAU that are orders of magnitude times less than the no-effect FIAU dose in humans.

The results reported above show that bortezomib treatment results in concentration of FIAU in lymphoma and carcinoma xenograft models and impacts on tumor growth curves for each of the tumors studied (Figure 19). In the lymphoma models, growth stopped and in the gastric carcinoma model growth was slowed. Optimization of bortezomib dosing and [¹³¹I]FIAU administration in gastric carcinoma using routine methods should achieve similar levels of efficacy as that observed in lymphoma.

FIAU is not prone to safety concerns that effect other radiopharmaceuticals because biotransformation of FIAU is limited because of the high chemical and metabolic stability of the *N*-glycosyl linkage in pyrimidine nucleosides that contain the 2'-fluoro substituent in the arabinosyl ("up") configuration (Jacobs et al., J Nucl Med 42, 467-475 (2001)). Studies in serum and whole blood over a 24 hour period indicated excellent stability and low susceptibility to deiodination with 97.8% ± 0.1% of labeled compound remaining unchanged. FIAU is also cleared more rapidly from plasma. Given the low level of deiodination and more rapid clearance, adverse effects on healthy tissues are expected to be minimal. Moreover potential adverse effects of radiation must be evaluated in the context of understanding that radiation is a mainstay for the treatment of many EBV-associated tumors, including gastric cancer, nasopharyngeal cancer, Hodgkin's and non-Hodgkin's lymphoma. Metabolic targeting of radiation specifically to tumor tissues should minimize adverse effects related to the exposure of normal tissues.

BETR therapy offers advantages over other lytic induction-suicide prodrug approaches. All lytic induction approaches are limited by the tendency of gamma herpes viruses to latency. When ganciclovir or a similar agent mediates cell killing, a therapeutic effect will likely require that a large fraction of tumor cells express viral enzymes. Although the phenomenon of bystander killing attributed to the exchange of phosphorylated nucleotides and nucleotide analogues between cells via gap junctions has been recognized, such killing is limited. In the gene therapy setting attempts have been made to engineer increased expression of the connexin protein in the gene therapy vector so as to increase such killing. However, this approach to increasing bystander killing is not readily applicable in approaches that do not involve gene therapy. Indeed, some of the therapeutic agents used to treat tumors may interfere with such bystander killing. [¹³¹I]FIAU bystander killing is not limited by connexin expression or gap junctions but is a function of beta emissions with a maximum energy of 0.61 MeV that deposit 90% of their energy in a sphere of radius 0.7 mm[²⁶. This "cross-fire" effect is likely an important contributor to the efficacy of radioimmunotherapy in lymphoma. "Cross-fire" with [¹³¹I]FIAU may be inferred from Figure 19C where the tumor growth rate is substantially slowed when even 10% of tumor cells harbor the EBV-TK. Cell level dosimetry modeling is presented in Figure 24.

A second limitation of the lytic induction-suicide prodrug approaches is that ganciclovir-mediated killing is cell cycle dependent. As reported by several groups of investigators, activation of lytic infection in EBV and KSHV infected cells leads to cell cycle arrest, thus likely compromising cell cycle-dependent tumor kill. In contrast, radiation effects are not similarly diminished by lytic induction.

BETR therapy may also offer advantages over radioimmunotherapeutic approaches. Internalization of radioimmunoconjugates may lead to degradation and release of free isotope with consequent loss of specificity. In contrast, following injection of FIAU, isotope was stably associated with EBV-TK expressing tumor (Figure 17a). Enzymatic phosphorylation and incorporation into DNA (as has been reported in HSV1- TK expressing cells) offers the possibility of achieving high concentrations in tumor tissue not limited by concentration gradients. Finally, both FIAU and bortezomib are small molecules and thus better tumor penetration would be anticipated in comparison with antibody conjugates. FIAU does not penetrate the blood-brain barrier but does reach brain tumors reflecting disruption of the blood brain barrier. More broadly, the use of small molecules to activate tissue or tumor-specific metabolic pathways suggests the possibility that metabolic targeting of radiation may have applications beyond thyroid cancer.

### Example 11: KSHV tumor treatment

Figures 22-24 depict treatment of KSHV tumors with bortezomib and [¹³¹I]FLAU.

### Example 12: Scale-up of mouse biodistribution data to the human and MIRD dosimetry calculations using the OLINDA software

Murine biodistribution data were converted to equivalent human data for dosimetry by assuming that the organ or tumor to whole-body concentration ratio in the mouse is equivalent to that in the human. Absorbed dose estimates for critical normal organs are listed in Table 3.

**Table 3. Critical Organ Absorbed Dose Estimates**

| | Absorbed dose | |
|---|---|---|
| | (mGy/MBq) | (rad/mCi) |
| Liver | 0.011 | 0.040 |
| Kidneys | 0.010 | 0.037 |
| Marrow | 0.001 | 0.003 |
| Total Body | 0.002 | 0.008 |

The initial (i.e., 2-h) activity concentration in the mouse tumor of 1.95%ID/g becomes 7x10⁴ %ID/g, assuming a 1 gm tumor in the human. At this concentration, and assuming negligible biological clearance of ¹³¹I, as shown in the biodistribution data, the absorbed dose per administered activity to a 1 gm tumor in a 70 kg human is 0.1 mGy/MBq (0.38 rad/mCi). Experience with ¹³¹I-labeled antibodies against lymphoma in patients suggests that responses are achievable with 0.5 to 2 Gy to the tumor. The 5 to 20 GBq (135 to 540 mCi) required to deliver this dose-range is well within normal organ tolerance given the normal organ absorbed dose estimates listed in the table. A total administered activity of 20 GBq would result in 0.2 and 0.02 Gy to the kidneys and red marrow, respectively. Based on external beam and radiopeptide experience, the kidneys can tolerate 25 to 27 Gy before the onset of toxicity; the red marrow maximally tolerated dose is 2 Gy. Administration of a 540 mCi dose would likely present logistical problems, but these could be accommodated by fractioning the administration over three to four injections.

### Example 13- Cell-level dosimetry analysis

Using the Monte Carlo simulation package, GEANT4, a spherical tumor model of spherical cells was constructed (15 µm diameter) in a hexagonal lattice (74% occupied volume vs. 52% for a cubic lattice) of varying sizes (∼10 cells, ∼20 cells, ∼50 cells, ∼150 cells per side). ¹³¹I is randomly allowed to decay according to three different patterns:
- all cells contain ¹³¹I
- 50% of cells contain ¹³¹I
- 10% of cells contain ¹³¹I
The energy deposited in each cell is collected and the dose to each cell calculated after (usually) 10 million Monte Carlo histories. Activity distributions in the cell cluster are illustrated in Figure 24.

Regardless of the percentage of cells expressing TK, the average absorbed dose to the tumor cell cluster is approximately, 4.3 to 4.4 Gy per 2.5 million decays. The dose per cell increases slightly at low percentage of TK-expressing cells because the total activity is allocated to smaller number of (randomly selected) cells. The mean dose per cell is independent of the fraction taking up activity because of the long range of ¹³¹I beta particle emissions relative to tumor cell dimensions.

The impact of different percentages of TK-expressing cells on the absorbed dose profile within a tumor is depicted in the figures below. Regardless of the percentage of cells expressing TK the average absorbed dose to the tumor cell cluster is approximately, 4.3 to 4.4 Gy per 2.5 million decays. The dose per cell increases slightly at low percentage of TK-expressing cells because the total activity is allocated to smaller number of (randomly selected) cells. The mean dose per cell is independent of the fraction taking up activity because of the long range of I-¹³¹ beta particle emissions relative to tumor cell dimensions.

Experiments described in Example 1-6 were carried out using the following methods and materials.

### Cell lines and plasmids.

Two EBV(+) Burkitt's lymphoma cell lines, and a subclone of one that lacks EBV (Rael, Akata EBV(+), Akata EBV(-)) were studied (Ambinder et al., Hematol Oncol Clin North Am 2003;17:697-702, v- vi; and Feng et al., J Virol 2004;78:1893-902). Tumor cell lines were maintained in 1640 RPMI (Life Technologies) with 10% fetal bovine serum, 100 U/ml penicillin, 100 pg/mL streptomycin, and 100 mM L-glutamine. A human osteosarcoma cell line (143B) was stably transfected with a plasmid expressing EBV-TK and a control vector (PCDNA3), as previously described (Moore et al., Antimicrob Agents Chemother 2001;45:2082-91). The 143B cell line was passaged in 15 ng/mL bromodeoxyuridine (BrdU, Sigma, St. Louis, MO) to maintain the cellular TK(-) phenotype. TK-143B and V143B cells were maintained in Dulbecco's modified essential medium (Gibco BRL, Gaithersburg, MD) supplemented with 10% fetal bovine serum (Gemini BioProducts, Calabasas, CA), 100 U/mL penicillin, 100 µg/mL, streptomycin 2 m*M*, L-glutamine 400 µg/mL, G418 for selection at 37° C with 5% CO₂. BX-1 is a recombinant EBV cell line encoding GFP with the BXLF1 ORF disrupted. GFP was transcribed from a CMV promoter. Akata-BX1 and AGS-BX1 cells are Akata cells and AGS cells containing this recombinant EBV, respectively (Wang et al. J Virol 77, 9590-9612 (2003)). An IκBα-based NF-κB super repressor (sr), IκBα(sr), was generated by either mutating serines 32 and 36, which are the targets of the IκB kinases, or by deleting the N-terminal portion of IκB that harbors these targets. IκBα (sr) suppressed stimuli-induced NF-κB activation by preventing its phosphorylation and subsequent degradation. The DNA fragments were cloned into the expression vector pEVRF (Wang et al., (1999) Nat Med 5, 412-7; Fu et al., (2004) J Biol Chem 279, 12819-26).

### Chemicals.

Bortezomib was provided by Millennium Predictive Medicine Inc. (Cambridge, MA). 5-Aza-2'-deoxycytidine, sodium butyrate and ganciclovir (GCV) were purchased from Sigma (St. Louis, MO). [2-¹⁴C]2'-Fluoro-2'-deoxy-β-D-5-iodouracil-arabinofuranoside ([¹⁴C]FIAU), FIAU and 2'-fluoro-2'-deoxy-uracil-β-D-arabinofuranoside (FAU) were obtained from Moravek Biochemicals (Brea, CA). [¹²⁵I]NaI was purchased from MP Biomedicals (Costa Mesa, CA). [¹²⁵I]FIAU was synthesized according to Jacobs et al., (2001) J Nucl Med 42, 467-75. Briefly, 30 µg FAU (1.22 mmol) was dissolved in 170 µL 2 *M* HNO₃. To this solution, 1-5 mCi of [¹²⁵I]NaI was added and the contents heated at 130°C for 45 minutes. The reaction was then quenched with 150 µL of high performance liquid chromatography (HPLC) mobile phase (20:79.9:0.1 MeCN:H₂O:triethylamine). The resulting [¹²⁵I]FIAU was then purified by reverse phase HPLC using a Phenomenex Luna C₁₈ semi-prep column (Phenomenex, Torrance, CA) (10 µm, 4.6 x 250 mm) using the above mentioned isocratic mobile phase at a flow rate of 2 mL/min. The product was concentrated under reduced pressure and formulated in 0.9% physiological saline before sterile filtration over a 0.22 µm syringe filter. Formulations were kept at ∼1 mCi/mL to minimize the volume of injection into mouse tail veins. The final radiochemical yield was ∼50% and the radiochemical purity was >99%. The specific radioactivity was always ≥ 2,000 Ci/mmol.

### In vitro cell uptake.

For *in vitro* [¹⁴C]FIAU uptake studies, cells were seeded at 5x10⁵cells/mL and incubated with 10 nM bortezomib at 37° C for 6-12 hrs. They were washed and then incubated with 0.04 µCi/mL [¹⁴C]FIAU at 37°C for 2, 4, 8, 24, 48, 72 hours. Cells were pelleted by centrifugation and washed with unlabeled FIAU. Radioactivity of the isolated cells and pooled medium with washes were then counted separately by scintillation counting with a Beckman Coulter LS5000TA scintillation counter (Fullerton, CA). Total cellular protein was extracted by incubating in lysis buffer (0.05 *M* Tris, pH 7.6, 0.15 *M* NaCl, 1% triton-X 100, 2 mM EDTA) and cell debris were removed by centrifugation. The supernatant was collected and the protein concentration was determined with the BCA protein assay kit (Pierce, Rockford, IL) according to the manufacturer's instructions. Cell uptake was expressed as the accumulation ratio, that is, the counts per minute per gram (cpm/g) of cells divided by the cpm/g (mL) of medium.

### Tumor generation.

5x10⁶cells were resuspended in 200 µL Matrigel Matrix (ED Biosciences, Bedford, MA) and injected subcutaneously near the shoulder of 6-7-week-old male severe combined immunodeficient (SCID) mice. Subsequent studies were performed when tumors reached a size of approximately 1 cm in diameter.

### Immunoblot analysis.

To detect expression of the EBV-TK or EBV-ZTA, total cell protein was first extracted by treating cells with lysis buffer (0.05 *M* Tris, pH 7.6, 0.15 *M* NaCl, 1% triton-X 100, 2 m*M* EDTA) containing protease inhibitors for 10 minutes at 4°C. The following reagents were added to 1 mL of lysis buffer: 2 µL leupeptin (5 mg/mL), 1 µL 0.2 M Na₃VO₄, 1 µL NaF, 1 µL aprotinin (10 mg/ml), 1 µL pepstatin (2 mM), and 10 µL PMSF (100mM) for 10 minutes at 4°C. Following centrifugation, the supernatant was collected and stored at-80°C. Protein concentration was determined with the BCA Protein Assay Kit (Pierce, Rockford, IL) according to the manufacturer's instructions. A 10 µg aliquot of total cellular protein was fractionated on a 7.5% SDS-polyacrylamide gel. Proteins were transferred to PROTRAN nitrocellulose membrane (Schleicher & Schuell, Keene, NH). The membranes were blocked with 5% blotting grade non-fat dry milk (Bio-rad, Hercules, CA) in PBS with 0.1 % TWEEN-20 (PBS-T) at 4° C overnight. The membranes were then washed with PBS-T twice followed by incubation with 1/1,000-diluted rabbit anti-TK sera from a rabbit immunized with a synthetic EBV-TK peptide (GRHESGLDAGYLKSVNDAC) or anti-ZTA monoclonal antibody (Argene, North Massapequa, NY) at room temperature for 1 hour. After washing, the membranes were incubated with HRP-conjugated goat anti-rabbit IgG antibodies or HRP-conjugated goat anti-mouse IgG antibodies (Amersham Pharmacia Biotech, Piscataway, NJ) at a 1/5,000 dilution in PBS-T at room temperature for 1 hour. The membranes were washed twice and proteins were detected with ECL Western Blotting Detection reagents (Amersham Pharmacia Biotech). The membranes were exposed to X-OMAT film (Kodak, Rochester, NY) at room temperature.

### Real-time PCR for EBV copy number.

Genomic DNA from treated cells were extracted with the QIAamp DNA mini kit (Qiagen, CA) according to the manufacturer's instructions. PCRs were set up in a volume of 50 µL with a TaqMan PCR core reagent kit (Perkin-Elmer Corp., Branchburg, NJ). Fluorescent probes were custom synthesized by Perkin-Elmer Applied Biosystems. PCR primers were synthesized by Gibco BRL (Frederick, Md.). Each reaction contained 5 µL of 10x buffer A (4 mM MgCl₂), amplification primers (300 nM), fluorescent probe (25 nM), dATP, dCTP, and dGTP (200 µM each), dUTP (400 uM), 1.25 U of AmpliTaq Gold, and 0.5 U of AmpErase uracil *N*-glycosylase. Extracted genomic DNA was used for amplification. DNA amplification was carried out in a 96-well reaction plate in a Perkin-Elmer Applied Biosystems 7700 sequence detector. Each sample was analyzed in duplicate, and multiple negative water blanks were included in each analysis. A calibration curve was run in parallel and in duplicate with each analysis, with DNA extracted from the EBV-positive cell line Namalwa as a standard.

### Animal preparation.

All work was undertaken in accordance with the regulations of Johns Hopkins Animal Care and Use Committee. Adult male SCID mice (5-6 weeks old) were purchased from the National Cancer Institute (Frederick, MD). Three days before imaging, all mice received 1 mL intraperitoneal (i.p.) injections of 0.9% NaI solution daily for 3 consecutive days to block the thyroid uptake of free radioiodide. Twenty four hours prior to [¹²⁵I]FIAU injection, lytic induction was initiated with bortezomib. Each mouse was injected i.p. with a dose of 2 µg/g of bortezomib. Bortezomib was made fresh in PBS and filter sterilized with a 0.2 µm syringe filter (Millipore, Billerica, MA) immediately before each injection. Control mice were injected i.p. with PBS alone. On the day of radiotracer injection, the animals were anesthetized with 2.3 mL/kg of a mixture of 25 mg/mL ketamine and 2 mg/mL acepromazine in 0.9% saline injected i.p. Isoflurane (0.5% - 1% at 0.5 - 1 L/minute) was administered to maintain anesthesia. Subsequently, 4.44 MBq (1.2 mCi) of [¹²⁵I]FIAU, which was measured using a dose calibrator (CRC-15R, Capintec, Ramsey, NJ), was then injected into each mouse by the tail vein.

### Ex vivo biodistribution.

After treatment with bortezomib, 0.074 MBq (2 µCi) of [¹²⁵I]FIAU in PBS was injected through the tail vein. Four mice were sacrificed at each indicated time by cervical dislocation. Tumor, liver, spleen, kidney, fat, blood, and muscle were removed and weighed after which the tissue radioactivity was measured using an automated gamma counter. A 0.1 mL sample of blood was also collected. The percent injected dose per gram (%ID/g) of tissue was calculated by comparison with samples of a standard dilution of the initial dose. The standard deviation (SD) was also calculated.

### Planar gamma imaging and SPECT-CT.

Imaging was performed as an adjunct to the *ex vivo* biodistribution studies. Quantitative data were not derived from the images except for once instance that employed SPECT-CT (see below). The X-SPECT (Gamma Medica Instruments, Northridge, CA) has a gamma ray detector head dimension of 20.5 cm x 15 cm x 9 cm and a 120 mm x 125 mm field of view (FOV). The high-resolution parallel hole collimator used in this study has the following specifications: 1.22 mm hole diameter, 0.20 mm septa thickness, 25.4 mm bore hole length. The detector material or scintillator crystal is composed of NaI[T1], which has a pixel size 2 mm x 2 mm x 6 mm. Mice were placed in a prone position on the parallel hole collimator, kept under anesthesia with isoflurane, which was delivered using a precision vaporizer (VetEquip, Pleasanton, CA). The static acquisition protocol of the LumaGEM™ software provided with the X-SPECT was used. Ten-minute high resolution scans of each mouse were obtained. SPECT-CT was performed using the abovementioned gamma ray detector and the CT detector, sequentially, without removing the mouse from the gantry.

### Analysis of imaging data.

Images were analyzed using ImageJ 1.30v (http://rsb.info.nih.gov/ij, National Institutes of Health, Bethesda, MD) or AMIDE (A Medical Image Data Examiner), for SPECT-CT, free software provided by Source Forge (http://amide.sourceforge.net).

### Cell lines

Akata-BX-1 cells are derived from the Akata EBV(-)cells with recombinant GFPEBV.AGS-BX-1 cells are AGS cells with recombinant GFP-EBV (Haubner, R., Avril, N., Hantzopoulos, P.A., Gansbacher, B. & Schwaiger, M. In vivo imaging of herpes simplex virus type 1 thymidine kinase gene expression: early kinetics of radiolabelled FIAU. Eur J Nucl Med 27, 283-291 (2000)). GFP is transcribed from a CMV immediate early promoter. To control for non-specific activation of the CMV promoter, a stable Hela cell line with a Pegfp Ni plasmid (CLONTECH) with CMV-promoter driven GFP was used. Cells were grown in RPMI 1640 medium (Akata), DMEM (Hela) or Ham's F-12 medium (AGS-BX1) supplemented with 10% fetal bovine serum (FBS) and 0.8 mg/ml G418. Plasmid HC131 contains BZLF IE promoter sequence (-578-+13 relative to the mRNA start site) linked to a lucifcrase reporter gene. A stable cell line containing HC 131 plasmid was established by cotransfection of HC 131 and pBabe-puro to AGS cells, and selected with 1 µg/ml puromycin. A clone of AGS cells that stably express a decent basal level of luciferase was used in the drug screen. AGSHC131 was grown in Hams F-12 with 10% FBS and I ug/ml puromycin. LCL, Raji, and SNU-719 were grown in RPMI 1640 medium with 10% FBS. All cells were grown at 37°C with 5% CO2 and 100% humidity.

### Chemicals and library

Johns Hopkins Clinical Compound Library (JHCCL) consists of 2720 compounds. All the clinical compounds were dissolved in DMSO or PBS and formatted into 96-well plates at 200 µM. Each plate included 80 drug wells and 16 blank control wells (1 column on each side). Anti-IgG was obtained from ABI. Tranilast, mebendazole, indoprofen, Ara-c, butyrate, valproate, leflunomide, and TPA were obtained from Sigma (St. Louis, MO). Bortezomib was obtained from Millennium Predictive Medicine Inc. (Cambridge, MA). Purvalanol A was obtained from Calbiochem.

For lytic induction studies, cells were seeded at 10⁶/ml, and treated with test compounds for 48 hours. Control untreated cells were incubated in parallel.

### GFP-Microplate reading:

For GFP fluorescent scanning, AKATA-BX-1 cells were aliquotted into regular 96 well plates (Costar3595) at 2 x 10⁵ cells/200µl per well. Then for each well, drugs to be tested (10µl 200µM) were transferred from drug plates to cell plates and mixed yielding a concentration of 10 µM. In addition to test drugs, the plates included wells with medium without cells or drugs and wells with cells but no drugs. Anti-IgG was added to four wells as positive control. Plates were maintained for up to 30 days. Readings were taken every other day. Plates were scanned with Fluorocount Microplate fluorometer (Packard Bioscience Company, Wellesley, MA). Excitation for GFP was at 485nm, while emission was measured at 530 nm. The readings were exported to Excel for spreadsheet analysis. The first readings were performed 30 minutes after adding the library drugs so as to eliminate drugs with autofluorescence. On day 3, positive controls showed increased GFP signal, and any readings that are more than 2/3 reading of the positive control were considered as hits.

### Luciferase reporter assay

For the luciferase assay, AGS-HC 131 cells were seeded into 96-well plates at a concentration of 4x 10⁴/200 µl the night before. Then for each well, 10 µl 200 µM drugs were transferred from drug plates to cell plates and mixed. Butyrate was added to one blank column of each plate as positive control. The plates were then incubated for 48 hours before a luciferase assay. The luciferase reporter gene assay reagents were obtained from Promega, and the assay was performed per the manufacturer's instructions.

### Western blot analysis

Cells were washed with PBS twice. Total cellular protein was extracted with lysis buffer. Proteins were electrophoresed in polyacrylamide and then transferred onto a PROTRAN nitrocellulose membrane (Schleicher&Schuell, Keene, NH). Blots were probed with primary antibody (Ab) overnight at 4°C. HRP-conjugated secondary Ab was added after washing and detected by an enhanced chemiluminescence system (ECL) (Amersham). The following antibodies were used: anti-ZTA (Argene), anti-f3-actin (Sigma), anti-mouse (Sigma).

### IFA

LCL cells were treated with 1 µM Ara-c for 48 hours. The cells were then washed with PBS, resuspended, and plated on polylysine glass slides. Indirect immunofluorescence assays and fluorescence microscopy were performed. The slides were fixed in methanol at - 20 °C, washed with PBS, then incubated in primary antibody anti-ZTA (1:200)(Argene), washed with PBS, followed by secondary antibody incubation of donkey rhodamine-conjugated IgG (Jackson Pharmaceuticals, west Grove, Pa). Mounting solution with 4',6'-diamidino-2-phenylindole (DAPI) (Vector Shield) was used to mark the nucleus of the cell. Slides were then observed and photographed with 40x objectives on a E800 microscope (NIKON) with CCD camera (Princeton Instruments) using Metamorph software (Universial Imaging-Molecular Devices).

### Quantitative PCR

The EBV genome was quantified by real-time PCR with the ICycler system (Biorad, Hercules, CA). Template DNA was extracted with a mini blood DNA Kit(Qiagen,Valencia, CA). The BamHI-W primers were BamH5' (5'- CCCAACACTCCACCACACC-3 ') and BamH3' (5 '-TCTTAGGAGCTGTCCGAGGG3') and the fluorescent probe (5'-(FAM)CACACA CTACACACACCCACCCGTCTC3'). A calibration curve was run in parallel and in duplicate with each analysis, using DNA extracted from the diploid EBV-positive cell line Namalwa containing two integrated viral genomes per cell as a standard. Amplification data were collected with an MyiQ single color real-time PCR Detector (Biorad) and analyzed with the MyiQ software developed by Biorad.

Results reported in Examples 7-10 were carried out using the following methods and materials.

### Cell lines and plasmids.

Lymphoma cell lines were maintained in suspension culture in RPMI 1640 (Life Technologies, Gaithersburg, MD) with 10% fetal bovine serum, 100 units/mL penicillin, 100 µg/mL streptomycin, and 100 mmol/L L-glutamine (as described above). TK-143B and V143B cells are stably transfected lines derived from the human osteosarcoma (143B) cell line carrying either a plasmid expressing EBV-TK or a control vector (PCDNA3), as previously described (Moore et al., Antimicrob Agents Chemother 45, 2082-2091 (2001)). These were maintained in DMEM (Life Technologies) supplemented with 10% fetal bovine serum (Gemini BioProducts, Calabasas, CA), 100 units/mL penicillin, 100 Ag/mL streptomycin, 2 mmol/L L-glutamine, and 400 Ag/mL G418 for selection. The KT transplantable EBV(+) gastric adenocarcinoma was passaged in SCID mice (Chong et al., J Viro/ 76,6825-6831 (2002)).

### Chemicals.

Bortezomib was administered intravenously at 1.67 µg/gram. FIAU, and 2'-fluoro-2'-deoxy-uracil-β-D-arabinofuranoside were obtained from Moravek Biochemicals (Brea, CA). [¹²⁵I] and [¹³¹I]NaI were purchased from MP Biomedicals (Costa Mesa, CA). [¹²⁵I]FIAU and [¹³¹I]FIAU was synthesized as previously described above. For imaging or treatment, FIAU was administered 24 hours after bortezomib. In each of the experiments described bortezomib was administered as a single dose.

### Tumor generation.

Cultured cell lines were tested for mycoplasma and found to be negative. Cells (5 x 10⁶) were resuspended in 200 µL Matrigel matrix (BD Biosciences, Bedford, MA) and injected SC in 6- to 7-week-old male severe combined immunodeficient mice. Caliper measurements of the longest perpendicular tumor diameters were performed every other day. Tumor volume was estimated according to the formula for a three dimensional ellipse: 4π/3 x (width/2)² x (length/2) (LeBlanc et al., Cancer Res 62, 4996-5000 (2002)). Treatment and imaging studies were performed when tumors reached a size of∼1 cm in diameter.

### Ex vivo biodistribution.

As previously described above, [¹²⁵I]FIAU in PBS was injected into the tail vein, 3 to 4 mice were sacrificed at each indicated time point, organs were removed and weighed, and tissue radioactivity measured using an automated gamma counter. The percent injected dose per gram (%ID/g) of tissue was calculated by comparison with samples of a standard dilution of the initial dose.

### Planar gamma imaging, SPECT-computed tomography and image analysis.

As previously described herein, imaging was done as an adjunct to the *ex vivo* biodistribution studies. The X-SPECT (Gamma Medica Instruments, Northridge, CA) has a γ-ray detector head dimension of 20.5 x 15 x 9 cm and a field of view of 120 x 125 mm. The high-resolution parallel hole collimator used has the following specifications: 1.22mm hole diameter, 0.20mm septa thickness, 25.4mm bore hole length. The detector material composed of NaI[TI] has a pixel size of 2 x 2 x 6 mm. Mice were placed in a prone position on the parallel hole collimator and kept under anesthesia with isoflurane. Ten-minute high-resolution scans of each mouse were obtained. SPECT-CT was done using the abovementioned γ-ray detector and the CT detector sequentially without removing the mouse from the gantry. Analysis of imaging data was with ImageJ v1.30 (NIH, Bethesda, MD) or AMIDE (A Medical Image Data Examiner) for SPECT-CT (free software provided by SourceForge).

Biostatistics. Linear regression and curve fitting were undertaken carried using GraphPad Prism software version 5.00 for Windows, GraphPad Software, San Diego California USA, www.graphpad.com.

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

## Claims

1. A pharmaceutical composition for use in the treatment of a neoplasia naturally infected with a virus or associated with a virus, the composition comprising an effective amount of a proteasome inhibitor and a radiolabeled analog of 2'-fluoro-2'-deoxy-β-D-5-iodouracil-arabinofuranoside (FIAU).

2. The pharmaceutical composition for use according to claim 1, wherein the proteasome inhibitor is Bortezomib.

## Patentansprüche

1. Pharmazeutische Zusammensetzung zur Verwendung bei der Behandlung einer Neoplasie, die natürlicherweise mit einem Virus infiziert oder mit einem Virus assoziiert ist, wobei die Zusammensetzung eine wirksame Menge eines Proteasom-Inhibitors und ein radioaktiv markiertes Analogon von 2'-Fluor-2'-desoxy-[beta]-D-5-iodouracil-arabinofuranosid (FIAU) umfasst.

2. Pharmazeutische Zusammensetzung zur Verwendung gemäß Anspruch 1, wobei der Proteasom-Inhibitor Bortezomib ist.

## Revendications

1. Composition pharmaceutique pour l'utilisation dans le traitement d'une néoplasie naturellement infectée par un virus ou associée à un virus, ladite composition comprenant une quantité efficace d'un inhibiteur de protéasome et un analogue radiomarqué de la 2'-fluoro-2'-désoxy-[beta]-D-5-iodouracile-arabinofuranoside (FIAU).

2. Composition pharmaceutique pour l'utilisation selon la revendication 1, dans laquelle l'inhibiteur de protéasome est le bortézomib.


### SEQUENCE LISTING

<110> THE JOHNS HOPKINS UNIVERSITY
<120> IMAGING AND THERAPY OF VIRUS-ASSOCIATED TUMORS
<130> 20736EP
<140> 08 742 868.6
   <141> 2007-04-10
<150> US 60/931,921 <151> 2007-05-25
<150> US 60/922,755 <151> 2007-04-10
<160> 57
<170> PatentIn version 3.5
<210> 1
   <211> 19
   <212> PRT
   <213> Artificial Sequence
<220>
   <223> Synthetic peptide
<400> 1
   Gly Arg His Glu Ser Gly Leu Asp Ala Gly Tyr Leu Lys Ser Val Asn
   1 5 10 15
   Asp Ala Cys
<210> 2
   <211> 19
   <212> DNA
   <213> Artificial Sequence
<220>
   <223> Synthetic primer
<400> 2
   cccaacactc caccacacc 19
<210> 3
   <211> 20
   <212> DNA
   <213> Artificial Sequence
<220>
   <223> Synthetic primer
<400> 3
   tcttaggagc tgtccgaggg 20
<210> 4
   <211> 27
   <212> DNA
   <213> Artificial Sequence
<220>
   <223> Synthetic primer
<400> 4
   cacacactac acacacccac ccgtctc 27
<210> 5
   <211> 97
   <212> PRT
   <213> Escherichia coli
<400> 5
   Ile His Cys Val Leu Val Asp Glu Cys Gln Phe Leu Thr Arg Gln Gln
   1               5                   10                  15
   Val Tyr Glu Leu Ser Glu Val Val Asp Gln Leu Asp Ile Pro Val Leu
               20                  25                  30
   Cys Tyr Gly Leu Arg Thr Asp Phe Arg Gly Glu Leu Phe Ile Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Ala
   65                  70                  75                  80
   Gly Arg Pro Tyr Asn Glu Gly Glu Gln Val Val Ile Gly Gly Asn Glu
                   85                  90                  95
   Arg
<210> 6
   <211> 97
   <212> PRT
   <213> Escherichia coli
<400> 6
   Ile His Cys Val Leu Val Asp Glu Cys Gln Phe Leu Thr Arg Gln Gln
   1               5                   10                  15
   Val Tyr Glu Leu Ser Glu Val Val Asp Gln Leu Asp Ile Pro Val Leu
               20                  25                  30
   Cys Tyr Gly Leu Arg Thr Asp Phe Arg Gly Glu Leu Phe Ile Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Ala
   65                  70                  75                  80
   Gly Arg Pro Tyr Asn Glu Gly Glu Gln Val Val Ile Gly Gly Asn Glu
                   85                  90                  95
   Arg
<210> 7
   <211> 97
   <212> PRT
   <213> Escherichia coli
<400> 7
   Ile His Cys Val Leu Val Asp Glu Cys Gln Phe Leu Thr Arg Gln Gln
   1               5                   10                  15
   Val Tyr Glu Leu Ser Glu Val Val Asp Gln Leu Asp Ile Pro Val Leu
               20                  25                  30
   Cys Tyr Gly Leu Arg Thr Asp Phe Arg Gly Glu Leu Phe Ile Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Ala
   65                  70                  75                  80
   Gly Arg Pro Tyr Asn Glu Gly Glu Gln Val Val Ile Gly Gly Asn Glu
                   85                  90                  95
   Arg
<210> 8
   <211> 97
   <212> PRT
   <213> Escherichia coli
<400> 8
   His Cys Val Leu Val Asp Glu Cys Gln Phe Leu Thr Arg Gln Gln Val
   1 5 10 15
   Tyr Glu Leu Ser Glu Val Val Asp Gln Leu Asp Ile Pro Val Leu Cys
   20 25 30
   Tyr Gly Leu Arg Thr Asp Phe Arg Gly Glu Leu Phe Ile Gly Ser Gln
   35 40 45
   Tyr Leu Leu Ala Trp Ser Asp Lys Leu Val Glu Leu Lys Thr Ile Cys
   50 55 60
   Phe Cys Gly Arg Lys Ala Ser Met Val Leu Arg Leu Asp Gln Ala Gly
   65 70 75 80
   Arg Pro Tyr Asn Glu Gly Glu Gln Val Val Ile Gly Gly Asn Glu Arg
   85 90 95
   Tyr
<210> 9
   <211> 97
   <212> PRT
   <213> Escherichia coli
<400> 9
   Met Asp Val Ile Ala Ile Asp Glu Val Gln Phe Phe Asp Gly Asp Ile
   1 5 10 15
   Val Glu Val Val Gln Val Leu Ala Asn Arg Gly Tyr Arg Val Ile Val
   20 25 30
   Ala Gly Leu Asp Gln Asp Phe Arg Gly Leu Pro Phe Gly Gln Val Pro
   35 40 45
   Gln Leu Met Ala Ile Ala Glu His Val Thr Lys Leu Gln Ala Val Cys
   50 55 60
   Ser Ala Cys Gly Ser Pro Ala Ser Arg Thr Gln Arg Leu Ile Asp Gly
   65 70 75 80
   Glu Pro Ala Ala Phe Asp Asp Pro Ile Ile Leu Val Gly Ala Ser Glu
   85 90 95
   Ser
<210> 10
   <211> 97
   <212> PRT
   <213> Bacillus cereus
<400> 10
   Met Asp Val Ile Ala Ile Asp Glu Val Gln Phe Phe Asp Gly Asp Ile
   1               5                   10                  15
   Val Glu Val Val Gln Val Leu Ala Asn Arg Gly Tyr Arg Val Ile Val
               20                  25                  30
   Ala Gly Leu Asp Gln Asp Phe Arg Gly Leu Pro Phe Gly Gln Val Pro
           35                  40                  45
   Gln Leu Met Ala Ile Ala Glu His Val Thr Lys Leu Gln Ala Val Cys
       50                  55                  60
   Ser Ala Cys Gly Ser Pro Ala Ser Arg Thr Gln Arg Leu Ile Asp Gly
   65                  70                  75                  80
   Glu Pro Ala Ala Phe Asp Asp Pro Ile Ile Leu Val Gly Ala Ser Glu
                   85                  90                  95
   Ser
<210> 11
   <211> 97
   <212> PRT
   <213> Bacillus cereus
<400> 11
   Leu Asp Val Ile Ala Ile Asp Glu Val Gln Phe Phe Asp Gly Asp Ile
   1               5                   10                  15
   Val Glu Val Val Gln Val Leu Ala Asn Arg Gly Tyr Arg Val Ile Val
               20                  25                  30
   Ala Gly Leu Asp Gln Asp Phe Arg Gly Leu Pro Phe Gly Gln Val Pro
           35                  40                  45
   Gln Leu Met Ala Ile Ala Glu His Val Thr Lys Leu Gln Ala Val Cys
   50 55 60
   Ser Val Cys Gly Ser Pro Ala Ser Arg Thr Gln Arg Leu Ile Asp Gly
   65 70 75 80
   Glu Pro Ala Ala Phe Asp Asp Pro Ile Ile Leu Val Gly Ala Ser Glu
   85 90 95
   Ser
<210> 12
   <211> 97
   <212> PRT
   <213> Bacillus subtilis
<400> 12
   Thr Asp Val Val Ala Val Asp Glu Val Gln Phe Phe Asp Gln Glu Ile
   1 5 10 15
   Val Glu Val Leu Ser Ser Leu Ala Asp Lys Gly Tyr Arg Val Ile Ala
   20 25 30
   Ala Gly Leu Asp Met Asp Phe Arg Gly Glu Pro Phe Gly Val Val Pro
   35 40 45
   Asn Ile Met Ala Ile Ala Glu Ser Val Thr Lys Leu Gln Ala Val Cys
   50 55 60
   Ser Val Cys Gly Ser Pro Ala Ser Arg Thr Gln Arg Leu Ile Asp Gly
   65 70 75 80
   Lys Pro Ala Ser Tyr Asp Asp Pro Val Ile Leu Val Gly Ala Ala Glu
   85 90 95
   Ser
<210> 13
   <211> 95
   <212> PRT
   <213> Chromobacterium violaceum
<400> 13
   Cys Val Leu Val Asp Glu Ala Gln Phe Met Thr Pro Glu Gln Ala Gln
   1 5 10 15
   Gln Leu His Arg Leu Ala His Lys Arg Asn Ile Pro Val Ile Cys Phe
   20 25 30
   Gly Leu Arg Thr Asp Phe Gln Gly His Pro Phe Pro Gly Ser Ala Trp
   35 40 45
   Leu Leu Ser Leu Ala Asp Asp Val Glu Glu Ile Lys Thr Ile Cys His
   50 55 60
   Cys Gly Arg Lys Ala Thr Met His Ile Arg Ile Asp Gly Asp Gly Arg
   65 70 75 80
   Arg Val Lys Glu Gly Pro Gln Val Glu Ile Gly Gly Glu Ala Arg
   85 90 95
<210> 14
   <211> 96
   <212> PRT
   <213> Clostridium perfringens
<400> 14
   Ile Asp Cys Ile Ile Val Asp Glu Val Gln Phe Leu Lys Ala His His
   1 5 10 15
   Ile Asp Glu Leu Phe Glu Ile Ala Val Gly Lys Gly Ile Pro Val Ile
   20 25 30
   Cys Tyr Gly Leu Arg Thr Asp Phe Gln Met Asn Gly Phe Glu Gly Ser
   35 40 45
   Glu Arg Leu Leu Leu Leu Ala His Ser Ile Glu Glu Met Lys Thr Ile
   50 55 60
   Cys Arg Cys Gly Arg Lys Ala Ile Leu Asn Gly Arg Met Ile Asn Gly
   65 70 75 80
   Lys Phe Thr Phe Glu Gly Glu Gln Val Ala Ile Asp Leu Val Asp Asn
   85 90 95
<210> 15
   <211> 97
   <212> PRT
   <213> Clostridium tetani
<400> 15
   Thr Glu Val Val Ala Ile Asp Glu Ala Gln Phe Phe Asp Lys Gly Ile
   1 5 10 15
   Leu Glu Val Val Asn Lys Ile Ala Asn Glu Gly Lys Arg Val Ile Cys
   20 25 30
   Ala Gly Leu Asp Gln Asp Phe Lys Gly Glu Pro Phe Gly Tyr Met Pro
   35 40 45
   Asp Ile Ile Ala Val Ala Glu Phe Val His Lys Val Gln Ala Val Cys
   50 55 60
   Met Ile Cys Gly Asn Pro Ala Thr Arg Thr Gln Arg Leu Ile Asn Gly
   65 70 75 80
   Lys Pro Ala Lys Tyr Asp Asp Pro Val Val Leu Val Gly Ala Lys Glu
   85 90 95
   Ser
<210> 16
   <211> 97
   <212> PRT
   <213> Coxiella burnetii
<400> 16
   Ile Arg Cys Val Leu Val Asp Glu Ala Gln Phe Leu Thr Lys Ser Gln
   1 5 10 15
   Val Glu Ala Leu Ala Leu Val Thr Asp Glu Leu Asn Leu Pro Val Leu
   20 25 30
   Ala Tyr Gly Ile Arg Thr Asp Phe Gln Gly Glu Pro Phe Glu Gly Ser
   35 40 45
   Val Tyr Leu Leu Ala Trp Ala Asp Leu Leu Ile Glu Ile Lys Thr Ile
   50 55 60
   Cys His Cys Gly Arg Lys Ala Thr Met Asn Leu Arg Ile Asp Asp Glu
   65 70 75 80
   Gly Asn Pro Ile Arg Glu Gly Glu Gln Ile Arg Leu Gly Gly Asn Asp
   85 90 95
   Arg
<210> 17
   <211> 95
   <212> PRT
   <213> Enterococcus faecalis
<400> 17
   Cys Val Leu Val Asp Glu Cys Gln Phe Leu Asn Lys His His Val Ile
   1 5 10 15
   Glu Phe Ala Arg Ile Val Asp Glu Leu Asp Ile Pro Val Met Ala Phe
   20 25 30
   Gly Leu Lys Asn Asp Phe Arg Asn Glu Leu Phe Glu Gly Ser Lys Tyr
   35 40 45
   Leu Leu Leu Tyr Ala Asp Lys Leu Glu Glu Leu Lys Thr Ile Cys Trp
   50 55 60
   Phe Cys His Lys Lys Ala Thr Met Asn Leu His Tyr Ile Asp Gly Asn
   65 70 75 80
   Pro Val Tyr Glu Gly Asp Gln Val Gln Ile Gly Gly Asn Glu Ala
   85 90 95
<210> 18
   <211> 97
   <212> PRT
   <213> Haemophilus ducreyi
<400> 18
   Leu His Cys Ile Met Ile Asp Glu Ala Gln Phe Leu Thr Lys Lys Gln
   1 5 10 15
   Val His Gln Leu Thr Asp Val Val Asp Glu Leu Lys Ile Pro Val Leu
   20 25 30
   Cys Tyr Gly Leu Arg Thr Asp Phe Gln Lys Glu Leu Phe Glu Gly Ser
   35 40 45
   Gln Tyr Leu Leu Ala Trp Ala Asp Glu Leu Gln Glu Leu Lys Thr Ile
   50 55 60
   Cys Glu Cys Gly Lys Lys Ala His Phe Val Ile Arg Leu Asn Glu Asn
   65 70 75 80
   Gly Glu Thr Val Thr Thr Gly Glu Gln Ile Gln Ile Gly Gly Asn Asp
   85 90 95
   Lys
<210> 19
   <211> 97
   <212> PRT
   <213> Haemophilus influenzae
<400> 19
   Val His Cys Val Leu Val Asp Glu Ala Gln Phe Leu Ser Lys Gln Gln
   1               5                   10                  15
   Val Tyr Gln Leu Ser Asp Val Val Asp Lys Leu Lys Ile Pro Val Leu
               20                  25                  30
   Cys Tyr Gly Leu Arg Thr Asp Phe Gln Ala Glu Leu Phe Glu Gly Ser
           35                  40                  45
   Lys Tyr Leu Leu Ala Trp Ala Asp Gln Leu Glu Glu Leu Lys Thr Ile
       50                  55                  60
   Cys Tyr Cys Gly Arg Lys Ala Asn Phe Val Leu Arg Leu Asn Asp Gln
   65                  70                  75                  80
   Gly Glu Val Ile Lys Glu Gly Ala Gln Ile Gln Ile Gly Gly Asn Asp
                   85                  90                  95
   Ser
<210> 20
   <211> 96
   <212> PRT
   <213> Listeria innocua
<400> 20
   Cys Val Leu Leu Asp Glu Ser Gln Phe Leu Glu Lys Glu His Val Phe
   1               5                   10                  15
   Gln Leu Ala Lys Ile Val Asp Asp Leu Asn Ile Pro Val Ile Ala Tyr
               20                  25                  30
   Gly Leu Lys Asn Asp Phe Arg Asn Glu Leu Phe Glu Gly Ser Lys Tyr
           35                  40                  45
   Leu Leu Leu Tyr Ala Asp Lys Leu Glu Glu Met Lys Thr Ile Cys Trp
       50                  55                  60
   Phe Cys Ala Lys Lys Ala Thr Met Val Leu Arg Val Asp Asp Lys Gly
   65                  70                  75                  80
   Lys Pro Val Tyr Thr Gly Glu Gln Ile Met Ile Gly Gly Asn Asp His
                   85                  90                  95
<210> 21
   <211> 96
   <212> PRT
   <213> Listeria monocytogenes
<400> 21
   Cys Val Leu Leu Asp Glu Ser Gln Phe Leu Glu Lys Glu His Val Phe
   1               5                   10                  15
   Gln Leu Ala Lys Ile Val Asp Glu Leu Asn Ile Pro Val Ile Ala Tyr
               20                  25                  30
   Gly Leu Lys Asn Asp Phe Arg Asn Glu Leu Phe Glu Gly Ser Lys Tyr
           35                  40                  45
   Leu Leu Leu Tyr Ala Asp Lys Leu Glu Glu Met Lys Thr Ile Cys Trp
       50                  55                  60
   Phe Cys Ala Lys Lys Ala Thr Met Val Leu Arg Val Asp Asp Lys Gly
   65                  70                  75                  80
   Lys Pro Val Tyr Thr Gly Glu Gln Ile Met Ile Gly Gly Asn Asp His
                   85                  90                  95
<210> 22
   <211> 97
   <212> PRT
   <213> Mycoplasma gallisepticum
<400> 22
   Pro Gln Leu Val Gly Ile Asp Glu Val Gln Phe Phe Asp Asp Ser Ile
   1               5                   10                  15
   Val Glu Val Ile Gln Thr Leu Ala Asp Asn Gln Ile Asn Val Ile Val
               20                  25                  30
   Ala Gly Leu Asp Arg Asp Phe Arg Gly Glu Pro Phe Gly Pro Ile Pro
           35                  40                  45
   Lys Ile Leu Gly Ile Ala Glu Ser Val Ile Arg Leu Thr Ala Ile Cys
   50 55 60
   Ser Glu Cys Gly Ala Glu Ala Ser Arg Ser Gln Arg Leu Ile Asp Asn
   65 70 75 80
   Gln Pro Ala Asp Tyr Asn Cys Glu Thr Ile Leu Ile Gly Asp Thr Glu
   85 90 95
   Ser
<210> 23
   <211> 94
   <212> PRT
   <213> Mycoplasma genitalium
<400> 23
   Tyr Gln Ile Val Ala Ile Asp Glu Ala Gln Phe Phe Ser Asn Glu Ile
   1 5 10 15
   Ile Glu Val Val Thr Thr Leu Asn Glu Ile Gly Thr Asn Val Ile Ile
   20 25 30
   Ser Gly Leu Asp Thr Asp Phe Arg Ala Glu Pro Phe Gly Cys Ile Pro
   35 40 45
   Gln Leu Leu Ala Ile Ala Asp Val Val Asn Lys Leu Asp Ala Ile Cys
   50 55 60
   Asn Val Cys Gly Ser Leu Ala Gln Arg Thr Gln Arg Leu Val Asn Lys
   65 70 75 80
   Asn Thr Asn Asp Asn Leu Val Leu Ile Gly Asp Ala Glu Ala
   85 90
<210> 24
   <211> 97
   <212> PRT
   <213> Mycoplasma mycoides
<400> 24
   Val Asp Val Ile Gly Ile Asp Glu Val Gln Phe Phe Asp Glu Gln Val
   1 5 10 15
   Val Glu Leu Ile Glu Gln Leu Ala Asn Gln Gly Ile Ile Val Ile Val
   20 25 30
   Asn Gly Leu Asp Lys Asp Phe Arg Cys Leu Pro Phe Lys Asn Val Asp
   35 40 45
   Lys Leu Leu Val Thr Ala Glu Phe Val Thr Lys Leu Arg Ala Arg Cys
   50 55 60
   His Leu Cys Gly Asn Phe Ala Asn Arg Ser Gln Lys Ile Val Asn Gly
   65 70 75 80
   Gln Pro Ala Leu Trp Asp Ser Pro Leu Ile Leu Val Asp Gly Lys Glu
   85 90 95
   Ser
<210> 25
   <211> 97
   <212> PRT
   <213> Mycoplasma penetrans
<400> 25
   Pro His Val Val Ala Ile Asp Glu Ala Gln Phe Ala Asp Glu Ser Ile
   1 5 10 15
   Val Asp Val Cys Gln Ala Leu Ala Asp Ser Gly Tyr Ile Val Tyr Val
   20 25 30
   Ser Ala Leu Asp Lys Asn Phe Lys Asn Glu Pro Phe Met Val Thr Ala
   35 40 45
   Lys Ile Ala Cys Ile Ala Glu Tyr Val Glu Lys Leu Ser Ala Ile Cys
   50 55 60
   Thr Asp Cys Gly Ala Pro Gly Thr Ala Thr Gln Arg Ile Ile Asn Asp
   65 70 75 80
   Lys Pro Ser Asn Tyr Asp Glu Pro Val Val Gln Ile Gly Asn Tyr Glu
   85 90 95
   Thr
<210> 26
   <211> 92
   <212> PRT
   <213> Mycoplasma pneumoniae
<400> 26
   Phe Asp Val Val Ala Ile Asp Glu Ala Gln Phe Phe Ser Ser Glu Ile
   1               5                   10                  15
   Val Glu Val Val Lys Ser Leu Asn Asp Leu Gly Ile Asn Val Ile Val
               20                  25                  30
   Ser Gly Leu Asp Thr Asp Phe Arg Ala Glu Pro Phe Gly Ser Ile Pro
           35                  40                  45
   Gln Leu Leu Ala Ile Ala Asp Lys Ile Cys Lys Leu Asp Ala Val Cys
       50                  55                  60
   Asn Val Cys Gly Gln Leu Ala Gln Arg Thr Gln Arg Ile Val Ser Lys
   65                  70                  75                  80
   Ser Asn Glu Thr Val Leu Ile Gly Asp Ile Glu Ala
                   85                  90
<210> 27
   <211> 91
   <212> PRT
   <213> Mycoplasma pulmonis
<400> 27
   Phe Asp Ala Leu Val Ile Asp Glu Ile His Phe Phe Asp Phe Asp Ile
   1               5                   10                  15
   Val Tyr Leu Ile Glu Glu Leu Ala Asn Ser Gly Tyr His Ile Ile Val
               20                  25                  30
   Ser Gly Leu Asp Gln Asn Phe Lys Arg Glu Pro Phe Glu Val Val Ser
           35                  40                  45
   Tyr Leu Leu Ser Ile Ala Glu Lys Val Thr Lys Leu Gln Ala Ile Cys
       50                  55                  60
   Val Lys Cys Gln Arg Ala Ala Thr Thr Thr Phe Arg Lys Val Glu Ser
   65                  70                  75                  80
   Lys Glu Ile Lys Leu Leu Gly Asp Val Asp Glu
                   85                  90
<210> 28
   <211> 97
   <212> PRT
   <213> Pasteurella multocida
<400> 28
   Leu His Cys Ile Leu Val Asp Glu Ala Gln Phe Leu Thr Lys Thr Gln
   1               5                   10                  15
   Val Tyr Gln Leu Ser Glu Val Val Asp Lys Leu Lys Ile Pro Val Leu
               20                  25                  30
   Cys Tyr Gly Leu Arg Thr Asp Phe Gln Ala Glu Leu Phe Glu Gly Ser
           35                  40                  45
   Lys Tyr Leu Leu Ala Trp Ala Asp Gln Leu Glu Glu Leu Lys Thr Ile
       50                  55                  60
   Cys Tyr Cys Gly Arg Lys Ala Asn Phe Val Leu Arg Leu Asn Asp Lys
   65                  70                  75                  80
   Gly Glu Val Val Arg Asp Gly Ala Gln Ile Gln Ile Gly Gly Asn Asp
                   85                  90                  95
   Ser
<210> 29
   <211> 91
   <212> PRT
   <213> Porphyromonas gingivalis
<400> 29
   Val Asp Val Val Gly Ile Asp Glu Ala Gln Phe Phe Asp Glu Gly Leu
   1               5                   10                  15
   Val Glu Val Ala Gln Gln Leu Ala Asp Gln Gly Val Arg Val Val Ile
               20                  25                  30
   Ala Gly Leu Asp Met Asp Phe Arg Arg Gln Pro Phe Gly Pro Met Pro
           35                  40                  45
   Gly Leu Cys Ala Ile Ala Asp Ser Val Thr Lys Val His Ala Val Cys
       50                  55                  60
   Val Glu Cys Gly Arg Leu Ala Ser Tyr Ser Phe Arg Arg Val Gln Gly
   65                  70                  75                  80
   Asp Gln Gln Val Met Leu Gly Glu Leu Asn Glu
                   85                  90
<210> 30
   <211> 91
   <212> PRT
   <213> Prevotella intermedia
<400> 30
   Ile Glu Val Val Gly Ile Asp Glu Ala Gln Phe Leu Asp Glu Gly Leu
   1               5                   10                  15
   Val Asp Ile Cys Asn Gln Leu Ala Asn Asn Gly Val Arg Val Ile Val
               20                  25                  30
   Ala Gly Leu Asp Met Asp Phe Lys Gly Val Pro Phe Gly Pro Ile Pro
           35                  40                  45
   Ser Leu Cys Ala Val Ala Asp Gln Val Thr Lys Val His Ala Ile Cys
       50                  55                  60
   Val Lys Cys Gly Ala Leu Ala Tyr Ala Ser His Arg Leu Val Asp Asn
   65                  70                  75                  80
   Asp His Arg Val Met Leu Gly Glu Gln Asn Glu
                   85                  90
<210> 31
   <211> 97
   <212> PRT
   <213> Salmonella enterica
<400> 31
   Ile His Cys Val Leu Val Asp Glu Ser Gln Phe Leu Thr Arg Gln Gln
   1               5                   10                  15
   Val Tyr Gln Leu Ser Glu Val Val Asp Lys Leu Asp Ile Pro Val Leu
               20                  25                  30
   Cys Tyr Gly Leu Arg Thr Asp Phe Arg Gly Glu Leu Phe Val Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Asp
   65                  70                  75                  80
   Gly Arg Pro Tyr Asn Glu Gly Glu Gln Val Val Ile Gly Gly Asn Glu
                   85                  90                  95
   Arg
<210> 32
   <211> 97
   <212> PRT
   <213> Salmonella enterica
<400> 32
   Ile His Cys Val Leu Val Asp Glu Ser Gln Phe Leu Thr Arg Gln Gln
   1               5                   10                  15
   Val Tyr Gln Leu Ser Glu Val Val Asp Lys Leu Asp Ile Pro Val Leu
               20                  25                  30
   Cys Tyr Gly Leu Arg Thr Asp Phe Arg Gly Glu Leu Phe Val Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Asp
   65                  70                  75                  80
   Gly Arg Pro Tyr Asn Glu Gly Glu Gln Val Val Ile Gly Gly Asn Glu
                   85                  90                  95
   Arg
<210> 33
   <211> 97
   <212> PRT
   <213> Salmonella typhimurium
<400> 33
   Ile His Cys Val Leu Val Asp Glu Ser Gln Phe Leu Thr Arg Gln Gln
   1               5                   10                  15
   Val Tyr Gln Leu Ser Glu Val Val Asp Lys Leu Asp Ile Pro Val Leu
               20                  25                  30
   Cys Tyr Gly Leu Arg Thr Asp Phe Arg Gly Glu Leu Phe Val Gly Ser
           35                  40                  45
   Gln Tyr Leu Leu Ala Trp Ser Asp Lys Leu Val Glu Leu Lys Thr Ile
       50                  55                  60
   Cys Phe Cys Gly Arg Lys Ala Ser Met Val Leu Arg Leu Asp Gln Asp
   65 70 75 80
   Gly Arg Pro Tyr Asn Glu Gly Glu Gln Val Val Ile Gly Gly Asn Glu
   85 90 95
   Arg
<210> 34
   <211> 97
   <212> PRT
   <213> Shigella flexneri
<400> 34
   Ile His Cys Val Leu Val Asp Glu Cys Gln Phe Leu Thr Arg Gln Gln
   1 5 10 15
   Val Tyr Glu Leu Ser Glu Val Val Asp Gln Leu Asp Ile Pro Val Leu
   20 25 30
   Cys Tyr Gly Leu Arg Thr Asp Phe Arg Gly Glu Leu Phe Ile Gly Ser
   35 40 45
   Gln Tyr Leu Leu Ala Trp Ser Asp Lys Leu Val Glu Leu Lys Thr Ile
   50 55 60
   Cys Phe Cys Gly Arg Lys Ala Ser Met Val Leu Arg Leu Asp Gln Ala
   65 70 75 80
   Gly Arg Pro Tyr Asn Glu Gly Glu Gln Val Val Ile Gly Gly Asn Glu
   85 90 95
   Arg
<210> 35
   <211> 97
   <212> PRT
   <213> Shigella flexneri
<400> 35
   Ile His Cys Val Leu Val Asp Glu Cys Gln Phe Leu Thr Arg Gln Gln
   1 5 10 15
   Val Tyr Glu Leu Ser Glu Val Val Asp Gln Leu Asp Ile Pro Val Leu
   20 25 30
   Cys Tyr Gly Leu Arg Thr Asp Phe Arg Gly Glu Leu Phe Ile Gly Ser
   35 40 45
   Gln Tyr Leu Leu Ala Trp Ser Asp Lys Leu Val Glu Leu Lys Thr Ile
   50 55 60
   Cys Phe Cys Gly Arg Lys Ala Ser Met Val Leu Arg Leu Asp Gln Ala
   65 70 75 80
   Gly Arg Pro Tyr Asn Glu Gly Glu Gln Val Val Ile Gly Gly Asn Glu
   85 90 95
   Arg
<210> 36
   <211> 97
   <212> PRT
   <213> Staphylococcus aureus
<400> 36
   Val Asp Val Ile Gly Ile Asp Glu Val Gln Phe Phe Asp Asp Glu Ile
   1 5 10 15
   Val Ser Ile Val Glu Lys Leu Ser Ala Asp Gly His Arg Val Ile Val
   20 25 30
   Ala Gly Leu Asp Met Asp Phe Arg Gly Glu Pro Phe Glu Pro Met Pro
   35 40 45
   Lys Leu Met Ala Val Ser Glu Gln Val Thr Lys Leu Gln Ala Val Cys
   50 55 60
   Ala Val Cys Gly Ser Ser Ser Ser Arg Thr Gln Arg Leu Ile Asn Gly
   65 70 75 80
   Lys Pro Ala Lys Ile Asp Asp Pro Ile Ile Leu Val Gly Ala Asn Glu
   85 90 95
   Ser
<210> 37
   <211> 97
   <212> PRT
   <213> Staphylococcus aureus
<400> 37
   Val Asp Val Ile Gly Ile Asp Glu Val Gln Phe Phe Asp Asp Glu Ile
   1 5 10 15
   Val Ser Ile Val Glu Lys Leu Ser Ala Asp Gly His Arg Val Ile Val
   20 25 30
   Ala Gly Leu Asp Met Asp Phe Arg Gly Glu Pro Phe Glu Pro Met Pro
   35 40 45
   Lys Leu Met Ala Val Ser Glu Gln Val Thr Lys Leu Gln Ala Val Cys
   50 55 60
   Ala Val Cys Gly Ser Ser Ser Ser Arg Thr Gln Arg Leu Ile Asn Gly
   65 70 75 80
   Lys Pro Ala Lys Ile Asp Asp Pro Ile Ile Leu Val Gly Ala Asn Glu
   85 90 95
   Ser
<210> 38
   <211> 97
   <212> PRT
   <213> Staphylococcus aureus
<400> 38
   Val Asp Val Ile Gly Ile Asp Glu Val Gln Phe Phe Asp Asp Glu Ile
   1 5 10 15
   Val Ser Ile Val Glu Lys Leu Ser Ala Asp Gly His Arg Val Ile Val
   20 25 30
   Ala Gly Leu Asp Met Asp Phe Arg Gly Glu Pro Phe Glu Pro Met Pro
   35 40 45
   Lys Leu Met Ala Val Ser Glu Gln Val Thr Lys Leu Gln Ala Val Cys
   50 55 60
   Ala Val Cys Gly Ser Ser Ser Ser Arg Thr Gln Arg Leu Ile Asn Gly
   65 70 75 80
   Lys Pro Ala Lys Ile Asp Asp Pro Ile Ile Leu Val Gly Ala Asn Glu
   85 90 95
   Ser
<210> 39
   <211> 97
   <212> PRT
   <213> Staphylococcus aureus
<400> 39
   Val Asp Val Ile Gly Ile Asp Glu Val Gln Phe Phe Asp Asp Glu Ile
   1               5                   10                  15
   Val Ser Ile Val Glu Lys Leu Ser Ala Asp Gly His Arg Val Ile Val
               20                  25                  30
   Ala Gly Leu Asp Met Asp Phe Arg Gly Glu Pro Phe Glu Pro Met Pro
           35                  40                  45
   Lys Leu Met Ala Val Ser Glu Gln Val Thr Lys Leu Gln Ala Val Cys
       50                  55                  60
   Ala Val Cys Gly Ser Ser Ser Ser Arg Thr Gln Arg Leu Ile Asn Gly
   65                  70                  75                  80
   Lys Pro Ala Lys Ile Asp Asp Pro Ile Ile Leu Val Gly Ala Asn Glu
                   85                  90                  95
   Ser
<210> 40
   <211> 97
   <212> PRT
   <213> Staphylococcus epidermidis
<400> 40
   Val Asn Val Ile Gly Ile Asp Glu Val Gln Phe Phe Glu Asp Asp Ile
   1               5                   10                  15
   Val Asn Ile Val Glu Lys Leu Ala Glu Asn Gly His Arg Val Ile Val
               20                  25                  30
   Ala Gly Leu Asp Met Asp Phe Arg Gly Glu Pro Phe Lys Pro Met Pro
           35                  40                  45
   Lys Leu Leu Ala Val Ser Glu His Ile Thr Lys Leu Gln Ala Val Cys
       50                  55                  60
   Ser Val Cys Gly Ser Pro Ser Ser Arg Thr Gln Arg Leu Ile Asn Gly
   65                  70                  75                  80
   Glu Pro Ala Lys Val Asp Asp Pro Ile Ile Leu Val Gly Ala Asn Glu
                   85                  90                  95
   Ser
<210> 41
   <211> 95
   <212> PRT
   <213> Streptococcus agalactiae
<400> 41
   Cys Val Leu Ile Asp Glu Cys Gln Phe Leu Ser Lys Lys Asn Val Tyr
   1               5                   10                  15
   Asp Leu Ala Arg Val Val Asp Asp Leu Asp Val Pro Val Met Ala Phe
               20                  25                  30
   Gly Leu Lys Asn Asp Phe Gln Asn Asn Leu Phe Glu Gly Ser Lys His
           35                  40                  45
   Leu Leu Leu Leu Ala Asp Lys Ile Asp Glu Ile Lys Thr Ile Cys Gln
       50                  55                  60
   Tyr Cys Ser Lys Lys Ala Thr Met Val Leu Arg Thr Glu Asn Gly Lys
   65                  70                  75                  80
   Pro Val Tyr Glu Gly Asp Gln Ile Gln Ile Gly Gly Asn Glu Thr
                   85                  90                  95
<210> 42
   <211> 95
   <212> PRT
   <213> Streptococcus mitis
<400> 42
   Cys Val Leu Val Asp Glu Ala Gln Phe Leu Lys Arg His His Val Tyr
   1               5                   10                  15
   Asp Leu Ala Arg Val Val Asp Glu Leu Asp Ile Pro Val Met Ala Phe
               20                  25                  30
   Gly Leu Lys Asn Asp Phe Arg Asn Glu Leu Phe Glu Gly Ser Lys Tyr
           35                  40                  45
   Leu Leu Leu Leu Ala Asp Lys Ile Glu Glu Ile Lys Thr Ile Cys Gln
   50 55 60
   Tyr Cys Lys Lys Lys Ala Thr Met Val Leu Arg Thr Gln Asp Gly Val
   65 70 75 80
   Pro Val Tyr Asp Gly Glu Gln Ile Gln Ile Gly Gly Asn Glu Thr
   85 90 95
<210> 43
   <211> 95
   <212> PRT
   <213> Streptococcus mutans
<400> 43
   Cys Ile Leu Ile Asp Glu Ser Gln Phe Leu Ser Gln Lys Asn Val Tyr
   1 5 10 15
   Asp Leu Ala Arg Ile Val Asp Glu Leu Asp Val Pro Val Met Ala Phe
   20 25 30
   Gly Leu Lys Asn Asp Phe Gln Asn His Leu Phe Glu Gly Ser Arg Glu
   35 40 45
   Leu Leu Leu Leu Ala Asp Lys Ile Glu Glu Ile Lys Thr Ile Cys Gln
   50 55 60
   Phe Cys Ser Lys Lys Ala Thr Met Val Leu Arg Thr Glu Asn Gly Arg
   65 70 75 80
   Pro Val Tyr Lys Gly Asn Gln Ile Gln Ile Gly Gly Asn Glu Thr
   85 90 95
<210> 44
   <211> 95
   <212> PRT
   <213> Streptococcus pneumoniae
<400> 44
   Cys Val Leu Val Asp Glu Ala Gln Phe Leu Lys Arg His His Val Tyr
   1 5 10 15
   Asp Leu Ala Arg Val Val Asp Glu Leu Asp Ile Pro Val Met Ala Phe
   20 25 30
   Gly Leu Lys Asn Asp Phe Arg Asn Glu Leu Phe Glu Gly Ser Lys Tyr
   35 40 45
   Leu Leu Leu Leu Ala Asp Lys Ile Asp Glu Ile Lys Thr Ile Cys Gln
   50 55 60
   Tyr Cys Lys Lys Lys Ala Thr Met Val Leu Arg Thr Gln Asp Gly Leu
   65 70 75 80
   Pro Val Tyr Asp Gly Glu Gln Ile Gln Ile Gly Gly Asn Glu Thr
   85 90 95
<210> 45
   <211> 95
   <212> PRT
   <213> Streptococcus pneumoniae
<400> 45
   Cys Val Leu Val Asp Glu Ala Gln Phe Leu Lys Arg His His Val Tyr
   1 5 10 15
   Asp Leu Ala Arg Val Val Asp Glu Leu Asp Ile Pro Val Met Ala Phe
   20 25 30
   Gly Leu Lys Asn Asp Phe Arg Asn Glu Leu Phe Glu Gly Ser Lys Tyr
   35 40 45
   Leu Leu Leu Leu Ala Asp Lys Ile Asp Glu Ile Lys Thr Ile Cys Gln
   50 55 60
   Tyr Cys Lys Lys Lys Ala Thr Met Val Leu Arg Thr Gln Asp Gly Leu
   65 70 75 80
   Pro Val Tyr Asp Gly Glu Gln Ile Gln Ile Gly Gly Asn Glu Thr
   85 90 95
<210> 46
   <211> 95
   <212> PRT
   <213> Streptococcus pyogenes
<400> 46
   Cys Val Leu Ile Asp Glu Ser Gln Phe Leu Ser Lys Gln Asn Val Tyr
   1 5 10 15
   Asp Leu Ala Arg Val Val Asp Glu Leu Asn Val Pro Val Met Ala Phe
   20 25 30
   Gly Leu Lys Asn Asp Phe Gln Asn Asn Leu Phe Glu Gly Ser Lys His
   35 40 45
   Leu Leu Leu Leu Ala Asp Lys Ile Asp Glu Ile Lys Thr Ile Cys Gln
   50 55 60
   Tyr Cys Ser Lys Lys Ala Thr Met Val Leu Arg Ile Glu Asn Gly Lys
   65 70 75 80
   Pro Val Tyr Glu Gly Asp Gln Ile Gln Ile Gly Gly Asn Glu Thr
   85 90 95
<210> 47
   <211> 95
   <212> PRT
   <213> Streptococcus pyogenes
<400> 47
   Cys Val Leu Ile Asp Glu Ser Gln Phe Leu Ser Lys Gln Asn Val Tyr
   1 5 10 15
   Asp Leu Ala Arg Val Val Asp Glu Leu Asn Val Pro Val Met Ala Phe
   20 25 30
   Gly Leu Lys Asn Asp Phe Gln Asn Asn Leu Phe Glu Gly Ser Lys His
   35 40 45
   Leu Leu Leu Leu Ala Asp Lys Ile Asp Glu Ile Lys Thr Ile Cys Gln
   50 55 60
   Tyr Cys Ser Lys Lys Ala Thr Met Val Leu Arg Thr Glu Asn Gly Lys
   65 70 75 80
   Pro Val Tyr Glu Gly Asp Gln Ile Gln Ile Gly Gly Asn Glu Thr
   85 90 95
<210> 48
   <211> 95
   <212> PRT
   <213> Streptococcus pyogenes
<400> 48
   Cys Val Leu Ile Asp Glu Ser Gln Phe Leu Ser Lys Gln Asn Val Tyr
   1 5 10 15
   Asp Leu Ala Arg Val Val Asp Glu Leu Asn Val Pro Val Met Ala Phe
   20 25 30
   Gly Leu Lys Asn Asp Phe Gln Asn Asn Leu Phe Glu Gly Ser Lys His
   35 40 45
   Leu Leu Leu Leu Ala Asp Lys Ile Asp Glu Ile Lys Thr Ile Cys Gln
   50 55 60
   Tyr Cys Ser Lys Lys Ala Thr Met Val Leu Arg Ile Glu Asn Gly Lys
   65 70 75 80
   Pro Val Tyr Glu Gly Asp Gln Ile Gln Ile Gly Gly Asn Glu Thr
   85 90 95
<210> 49
   <211> 98
   <212> PRT
   <213> Streptomyces avermitilis
<400> 49
   Val Asp Tyr Leu Ile Val Asp Glu Ala Gln Phe Leu Ala Pro Glu Gln
   1 5 10 15
   Ile Asp Gln Leu Ala Arg Val Val Asp Asp Leu Asp Leu Asp Val Phe
   20 25 30
   Ala Phe Gly Ile Thr Thr Asp Phe Arg Thr Lys Leu Phe Pro Gly Ser
   35 40 45
   Gln Arg Leu Ile Glu Leu Ala Asp Arg Ile Glu Thr Leu Gln Val Glu
   50 55 60
   Ala Met Cys Trp Cys Gly Ala Arg Ala Thr His Asn Ala Arg Thr Val
   65 70 75 80
   Gly Gly Glu Met Val Val Glu Gly Glu Gln Val Val Val Gly Asp Val
   85 90 95
   Asn Arg
<210> 50
   <211> 98
   <212> PRT
   <213> Streptomyces coelicolor
<400> 50
   Val Asp Tyr Val Ile Ala Asp Glu Ala Gln Phe Leu Ala Pro Asp Gln
   1 5 10 15
   Ile Asp Gln Leu Ala Arg Val Val Asp Asp Leu Asp Val Asp Val Tyr
   20 25 30
   Ala Phe Gly Ile Thr Thr Asp Phe Arg Ser Lys Leu Phe Pro Gly Ser
   35 40 45
   Gln Arg Leu Val Glu Leu Ala Asp Arg Val Glu Val Leu Gln Val Glu
   50 55 60
   Ala Leu Cys Trp Cys Gly Ala Arg Ala Thr His Asn Ala Arg Thr Val
   65 70 75 80
   Gly Gly Val Met Val Val Glu Gly Ala Gln Val Val Val Gly Asp Val
   85 90 95
   Ala Gln
<210> 51
   <211> 97
   <212> PRT
   <213> Ureaplasma urealyticum
<400> 51
   Thr Lys Val Ile Gly Ile Asp Glu Val Gln Phe Phe Asp Asp Arg Ile
   1 5 10 15
   Cys Glu Val Ala Asn Ile Leu Ala Glu Asn Gly Phe Val Val Ile Ile
   20 25 30
   Ser Gly Leu Asp Lys Asn Phe Lys Gly Glu Pro Phe Gly Pro Ile Ala
   35 40 45
   Lys Leu Phe Thr Tyr Ala Asp Lys Ile Thr Lys Leu Thr Ala Ile Cys
   50 55 60
   Asn Glu Cys Gly Ala Glu Ala Thr His Ser Leu Arg Lys Ile Asp Gly
   65 70 75 80
   Lys His Ala Asp Tyr Asn Asp Gln Ile Val Lys Ile Gly Cys Asp Glu
   85 90 95
   Phe
<210> 52
   <211> 97
   <212> PRT
   <213> Vibrio cholerae
<400> 52
   Arg His Cys Ile Leu Met Asp Glu Cys Gln Phe Leu Ser Lys Glu Gln
   1 5 10 15
   Val Tyr Gln Leu Thr Glu Val Val Asp Lys Leu Asp Ile Pro Val Leu
   20 25 30
   Cys Tyr Gly Leu Arg Thr Asp Phe Leu Gly Glu Leu Phe Glu Gly Ser
   35 40 45
   Lys Tyr Leu Leu Ser Trp Ala Asp Lys Leu Ile Glu Leu Lys Thr Ile
   50 55 60
   Cys His Cys Gly Arg Lys Ala Asn Met Val Ile Arg Thr Asp Glu His
   65 70 75 80
   Gly Asn Ala Ile Ser Glu Gly Asp Gln Val Ala Ile Gly Gly Asn Asp
   85 90 95
   Lys
<210> 53
   <211> 97
   <212> PRT
   <213> Vibrio parahaemolyticus
<400> 53
   Arg His Cys Ile Leu Ile Asp Glu Cys Gln Phe Leu Ser Lys Glu Gln
   1 5 10 15
   Val Tyr Gln Leu Thr Glu Val Val Asp Lys Leu His Ile Pro Val Leu
   20 25 30
   Cys Tyr Gly Leu Arg Thr Asp Phe Leu Gly Glu Leu Phe Glu Gly Ser
   35 40 45
   Lys Tyr Leu Leu Ser Trp Ala Asp Lys Leu Val Glu Leu Lys Thr Ile
   50 55 60
   Cys His Cys Gly Arg Lys Ala Asn Met Val Ile Arg Thr Asp Glu His
   65 70 75 80
   Gly Val Ala Ile Lys Glu Gly Asp Gln Val Ala Ile Gly Gly Asn Asp
   85 90 95
   Arg
<210> 54
   <211> 97
   <212> PRT
   <213> Vibrio vulnificus
<400> 54
   Arg His Cys Ile Leu Val Asp Glu Cys Gln Phe Leu Ser Lys Glu Gln
   1               5                   10                  15
   Val Tyr Gln Leu Thr Glu Val Val Asp Lys Leu His Ile Pro Val Leu
               20                  25                  30
   Cys Tyr Gly Leu Arg Thr Asp Phe Leu Gly Glu Leu Phe Glu Gly Ser
           35                  40                  45
   Lys Tyr Leu Leu Ser Trp Ala Asp Lys Leu Val Glu Leu Lys Thr Ile
       50                  55                  60
   Cys His Cys Gly Arg Lys Ala Asn Met Val Ile Arg Thr Asp Glu His
   65                  70                  75                  80
   Gly Lys Ala Ile Lys Glu Gly Asp Gln Val Ala Ile Gly Gly Asn Asp
                   85                  90                  95
   Arg
<210> 55
   <211> 97
   <212> PRT
   <213> Vibrio vulnificus
<400> 55
   Arg His Cys Ile Leu Val Asp Glu Cys Gln Phe Leu Ser Lys Glu Gln
   1               5                   10                  15
   Val Tyr Gln Leu Thr Glu Val Val Asp Lys Leu His Ile Pro Val Leu
               20                  25                  30
   Cys Tyr Gly Leu Arg Thr Asp Phe Leu Gly Glu Leu Phe Glu Gly Ser
           35                  40                  45
   Lys Tyr Leu Leu Ser Trp Ala Asp Lys Leu Val Glu Leu Lys Thr Ile
       50                  55                  60
   Cys His Cys Gly Arg Lys Ala Asn Met Val Ile Arg Thr Asp Glu His
   65 70 75 80
   Gly Asn Ala Ile Lys Glu Gly Asp Gln Val Ala Ile Gly Gly Asn Asp
   85 90 95
   Arg
<210> 56
   <211> 97
   <212> PRT
   <213> Yersinia pestis
<400> 56
   Ile His Cys Ile Leu Leu Asp Glu Cys Gln Phe Leu Thr Lys Glu Gln
   1 5 10 15
   Val Gln Glu Leu Cys Gln Val Val Asp Glu Leu His Leu Pro Val Leu
   20 25 30
   Cys Tyr Gly Leu Arg Thr Asp Phe Leu Gly Glu Leu Phe Pro Gly Ser
   35 40 45
   Lys Tyr Leu Leu Ala Trp Ala Asp Lys Leu Val Glu Leu Lys Thr Ile
   50 55 60
   Cys His Cys Gly Arg Lys Ala Asn Met Val Leu Arg Leu Asp Glu Gln
   65 70 75 80
   Gly Arg Ala Val His Asn Gly Glu Gln Val Val Ile Gly Gly Asn Glu
   85 90 95
   Ser
<210> 57
   <211> 97
   <212> PRT
   <213> Yersinia pestis
<400> 57
   Ile His Cys Ile Leu Leu Asp Glu Cys Gln Phe Leu Thr Lys Glu Gln
   1 5 10 15
   Val Gln Glu Leu Cys Gln Val Val Asp Glu Leu His Leu Pro Val Leu
   20 25 30
   Cys Tyr Gly Leu Arg Thr Asp Phe Leu Gly Glu Leu Phe Pro Gly Ser
           35                  40                  45
   Lys Tyr Leu Leu Ala Trp Ala Asp Lys Leu Val Glu Leu Lys Thr Ile
       50                  55                  60
   Cys His Cys Gly Arg Lys Ala Asn Met Val Leu Arg Leu Asp Glu Gln
   65                  70                  75                  80
   Gly Arg Ala Val His Asn Gly Glu Gln Val Val Ile Gly Gly Asn Glu
                   85                  90                  95
   Ser